# EUROPEAN PATENT APPLICATION

(11) **EP 4 837 915 A1**
(43) Date of publication of application: **11.11.2026**
(21) Application number: 25174892.7
(22) Date of filing: 07.05.2025
(51) Int. Cl.: C07F 7/08

(54) **SILICON CONTAINING IONIZABLE LIPIDS**

(71) Applicant: Wacker Chemie AG, 81671 München (DE)
(72) Inventor: Koch, Vanessa, 81671 Munich (DE); Dubiella, Christian, 81671 Munich (DE); Lindenthal, Laura, 81671 Munich (DE)
(74) Representative: Wimmer, Andreas

(57) **Abstract**

The present invention is directed to silicon containing ionizable lipids, lipid nanoparticles (LNPs) comprising at least one ionizable lipid according to the invention, pharmaceutical compositions comprising a lipid nanoparticle according to the inventio and a pharmaceutically acceptable carrier as well as their pharmaceutical use.

## Description

The present invention is directed to the field of biomedicine, specifically focusing on lipid nanoparticles (LNPs) and ionizable lipids.

Lipid nanoparticles (LNPs) are increasingly utilized as delivery vehicles for biologically active agents, such as polynucleotides, including RNAs and mRNAs, into cells. These compositions are particularly valuable for applications in delivering biologically active substances to cells, where efficient and targeted delivery of nucleic acid components is essential for therapeutic efficacy.

Delivering biologically active substances to cells can be utilized technologically in the field of transfection. Transfection is the process of introducing biologically active substances, such as nucleic acids, into eukaryotic cells to modify gene expression or study gene function. Transfection reagents are substances that facilitate this delivery, ensuring efficient uptake by cells. Lipid nanoparticles (LNPs) serve as effective transfection reagents due to their ability to encapsulate and protect nucleic acids, enhancing cellular uptake and transfection efficiency.

Currently, the market offers a limited selection of ionizable lipids for LNP formulation, primarily based on glycerin or glycerin derivatives. These existing ionizable lipids serve as the mass component of LNPs and significantly influence their physicochemical properties, such as particle size and encapsulation efficiency, which in turn affect transfection efficiency and targeting specificity. Despite their utility, the available ionizable lipids do not fully meet the demands for enhanced delivery efficiency and specificity required for advanced therapeutic applications, such as vaccinations and cancer therapies.

Thus, there is a need to overcome the above limitations at least in part by providing an improved ionizable lipid for the formulation of lipid nanoparticles.

The present invention introduces silicon-containing ionizable lipids, which, when incorporated into LNPs, exhibit remarkable encapsulation efficiencies and luciferase activities, a measure for transfection efficiency. These novel lipids are designed to facilitate the delivery of biologically active substances into cells, thereby expanding the potential for technological applications and therapeutic uses, including vaccinations and cancer treatments.

A **first aspect of the present invention** is directed to a compound according to chemical formula (I) or its isomeric form 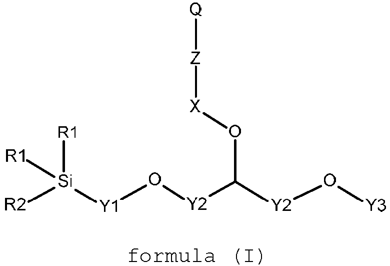 wherein
- **R1**: represents independently from each other H, OH, or a C1-C16 hydrocarbon residue, which can be linked to the Si atom by an O atom,
preferably H, OH, or a C1-C6 hydrocarbon residue, which can be linked to the Si atom by an O atom,
more preferably H, OH, or a C1-C6 alkyl group, which can be linked to the Si atom by an O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl linking group, in particular -CH₂-CH₂-CH₂-;
- **Y2**: represents independently from each other a C1-C10 hydrocarbon linking group, preferably a C1-C6 hydrocarbon linking group, more preferably a C1-C4 alkyl linking group, more preferably -CH₂-or -CH₂-(CH₂)₂-CH₂-, in particular -CH₂-;
- **Y3**: represents
-**Y1**-Si(**R1**)₂**R2** with the definitions given above, or
-(C=O)-**R5**, wherein **R5** represents an organic residue comprising at least 5 C atoms, preferably at least 8 C atoms, in particular 8 to 21 C atoms,
   wherein the organic residue **R5** can be branched or linear and/or
   wherein the organic residue **R5** can be saturated or unsaturated, preferably saturated and/or
   wherein the organic residue **R5** can contain further heteroatoms selected from O, N or S, preferably O and S;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **Q**: represent an organic residue comprising 2 to 20 C-atoms, preferably 2 to 12 C atoms, and at least one N atom, wherein the organic residue can be saturated or unsaturated, preferably saturated.

"Hydrocarbon residue" in terms of the present application refers to a structural unit within a molecule that consists of a chain or ring of carbon and hydrogen atoms. The hydrocarbon residue may be saturated or unsaturated and/or linear or branched. In particular, the hydrocarbon residue may contain at least one double bond, preferably 1 to 3 double bonds.

"Organic residue" in terms of the present application refers to a structural unit within a molecule that consists of a chain, ring, or network of carbon atoms bonded to hydrogen and potentially other elements such as oxygen, nitrogen, sulfur, or halogens.

If not otherwise stated, the term "heteroatom" represents Si, O, P or N.

Preferably, the organic residue **Q** is attached to **Z** or - if **Z** represents a chemical bond - to **X** via the at least one N atom or one C atom, preferably via the at least one N atom.

The organic residue **Q** can be linear, branched or cyclic, preferably branched or cyclic.

The organic residue **Q** can comprise further heteroatoms, such as O.

Examples for cyclic organic residues **Q** are pyrrolidine, piperidine, alkylpiperidine, such as methylpiperidine, azepane, alkylpiperazine, such as N-methylpiperazine, and morpholine.

The organic residue **Q** can be represented by -N**R3R4**, wherein **Q** is attached to **Z** or - if **Z** represents a chemical bond - to **X** via the "-N" unit, wherein
- **R3** and **R4**: represent independently from each other a C1-C6 hydrocarbon residue, preferably a C1-C4 alkyl residue, in particular a methyl, ethyl or butyl residue, wherein **R3** and/or **R4** may contain heteroatoms selected from N and O,
or **R3** and **R4** can form a ring together, including the N atom they are bound to, wherein this ring comprises 4-8 carbon atoms, preferably 4-6 carbon atoms, and optionally further heteroatoms selected from O and N, in particular selected from the group comprising pyrrolidine, piperidine, alkylpiperidine, such as methylpiperidine, azepane, alkylpiperazine, such as *N-*methylpiperazine, and morpholine.

**R5** is preferably a linear or branched alkyl or alkenyl residue, which may contain heteroatoms, preferably O, N and S, in particular O and S.

Heteroatoms selected from O, N and S in **R5** are in particular realized by interruption of the chain in **R5** by an ester, ether, amide, thioether, thioester and/or disulfide unit, preferably a thioether- and/or ester unit.

In particular, **R5** represents a saturated hydrocarbon residue comprising at least 8 carbon atoms, preferably at least 17 carbon atoms, wherein the hydrocarbon residue may be linear or branched and wherein the chain in **R5** may be interrupted by an ester, ether, amide, thioether, thioester and/or disulfide unit, preferably a thioether- and/or ester unit.

Alternatively, **R5** represents a saturated hydrocarbon residue comprising 8-21 carbon atoms, preferably 17-21 carbon atoms, wherein the hydrocarbon residue may be linear or branched and wherein the chain in **R5** may be interrupted by an ester, ether, amide, thioether, thioester and/or disulfide unit, preferably a thioether- and/or ester unit..

Preferably, **X** is selected from

**b¹**-(C=O)-**b**², **b¹**-(C=O)-O-**b²**, **b¹**-(C=O)-S-**b²** or **b¹**-(C=O)(N-**R'**)-**b²**,

wherein **b¹** represents the bond to the O atom and **b²** represents the bond to **Z** or the unit **Q,** in case **Z** represents a chemical bond, and **R'** is selected from the group consisting of H and C1-C4 hydrocarbon, preferably H and C1-C4 alkyl, more preferably H or -CH₃, in particular H.

In one preferred embodiment of formula (I), **Y3** represents **-Y1**-Si(**R1**)₂**R2** with the definitions given above.

In an alternative preferred embodiment of formula (I), **Y3** represents -(C=O)-**R5**, wherein **R5** represents an organic residue comprising at least 5 C atoms, preferably at least 8 C atoms, in particular 8 to 21 C atoms, wherein the organic residue **R5** can be branched or linear and/or wherein the organic residue **R5** can be saturated or unsaturated, preferably saturated and/or wherein the organic residue **R5** can contain further heteroatoms selected from O, N or S, preferably O and S.

In a preferred embodiment, the compound according to the invention is a compound according to chemical formula (Ia) or its isomeric form 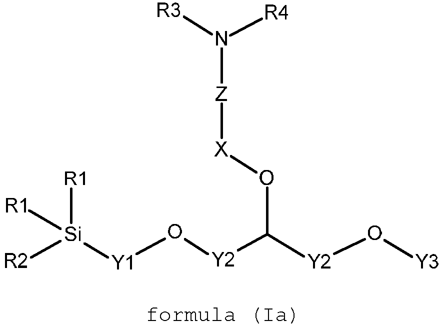 wherein
- **R1**: represents independently from each other H, OH, or a C1-C16 hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl group, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl linking group, in particular -CH₂-CH₂-CH₂-;
- **Y2**: represents independently from each other a C1-C10 hydrocarbon linking group, preferably a C1-C6 hydrocarbon linking group, more preferably a C1-C4 alkyl linking group, more preferably -CH₂- or -CH₂-(CH₂)₂-CH₂-, in particular -CH₂-;
- **Y3**: represents
**-Y1**-Si(**R1**)₂**R2** with the definitions given above, or
-(C=O)-**R5**, wherein **R5** represents an organic residue comprising at least 5 C atoms, preferably at least 8 C atoms, in particular 8 to 21 C atoms
   wherein the organic residue **R5** can be branched or linear and/or
   wherein the organic residue **R5** can be saturated or unsaturated, preferably saturated and/or
   wherein the organic residue **R5** can contain further heteroatoms selected from O, N or S, preferably O and S;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **R3** and **R4**: represent independently from each other a C1-C6 hydrocarbon residue, preferably a C1-C4 alkyl residue, in particular a methyl, ethyl or butyl residue, wherein **R3** and/or **R4** may contain heteroatoms selected from N and O,
or **R3** and **R4** can form a ring together, including the N atom they are bound to, wherein this ring comprises 4-8 carbon atoms, preferably 4-6 carbon atoms, and optionally further heteroatoms selected from O and N.

Preferably, **X** is selected from

**b¹**-(C=O)-**b**², **b¹**-(C=O)-O-**b²**, **b¹**-(C=O)-S-**b²** or **b¹**-(C=O)(N-**R'**)-**b²**,

wherein **b¹** represents the bond to the O atom and **b²** represents the bond to **Z** or the N atom, in case **Z** represents a chemical bond, and **R'** is selected from the group consisting of H and C1-C4 hydrocarbon, preferably H and C1-C4 alkyl, more preferably H or -CH₃, in particular H.

In one preferred embodiment of formula (Ia), **Y3** represents **-Y1**-Si**(R1)**₂**R2** with the definitions given above.

In an alternative preferred embodiment of formula (Ia), **Y3** represents **-**(C=O)-**R5,** wherein **R5** represents an organic residue comprising at least 5 C atoms, preferably at least 8 C atoms, in particular 8 to 21 C atoms, wherein the organic residue **R5** can be branched or linear and/or wherein the organic residue **R5** can be saturated or unsaturated, preferably saturated and/or wherein the organic residue **R5** can contain further heteroatoms selected from O, N or S, preferably O and S.

In an alternative embodiment, the compound according to the invention is a compound according to chemical formula (Ib) or its isomeric form 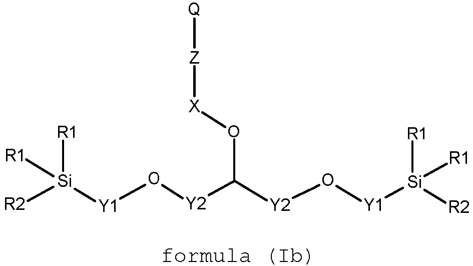 wherein
- **R1**: represents independently from each other H, OH, or a C1-C16 hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl residue, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl linking group, in particular -CH₂-CH₂-CH₂-;
- **Y2**: represents independently from each other a C1-C10 hydrocarbon linking group, preferably a C1-C6 hydrocarbon linking group, more preferably a C1-C4 alkyl linking group, more preferably -CH₂- or -CH₂-(CH₂)₂-CH₂-, in particular -CH₂-;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **Q**: represent an organic residue comprising 2 to 20 C atoms, preferably 2 to 12 C atoms, and at least one N atom, wherein the organic residue can be saturated or unsaturated, preferably saturated.

Preferably, **X** is selected from

**b¹**-(C=O)-**b**², **b¹**-(C=O)-O-**b²**, **b¹-**(C=O)-S-**b²** or **b¹**-(C=O)(N-**R'**)-**b²**,

wherein **b¹** represents the bond to the O atom and **b²** represents the bond to **Z** or the unit **Q,** in case **Z** represents a chemical bond, and **R'** is selected from the group consisting of H and C1-C4 hydrocarbon, preferably H and C1-C4 alkyl, more preferably H or -CH₃, in particular H.

In a preferred embodiment, the compound according to the invention is a compound according to chemical formula (Ic) or its isomeric form 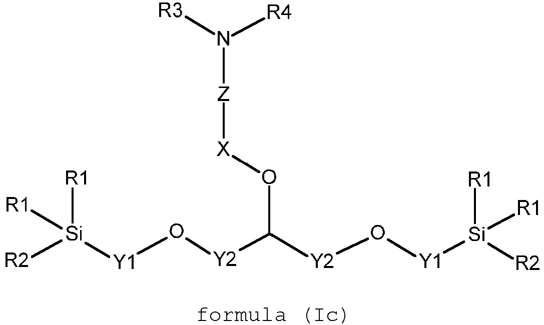 wherein
- **R1**: represents independently from each other H, OH, or a C1-C16 hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl goup, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl linking group, in particular -CH₂-CH₂-CH₂-;
- **Y2**: represents independently from each other a C1-C10 hydrocarbon linking group, preferably a C1-C6 hydrocarbon linking group, more preferably a C1-C4 alkyl linking group, more preferably -CH₂- or -CH₂-(CH₂)₂-CH₂-, in particular -CH₂-;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **R3** and **R4**: represent independently from each other a C1-C6 hydrocarbon residue, preferably a C1-C4 alkyl residue, in particular a methyl, ethyl or butyl residue, wherein **R3** and/or **R4** may contain heteroatoms selected from N and O,
or **R3** and **R4** can form a ring together, including the N atom they are bound to, wherein this ring comprises 4-8 carbon atoms, preferably 4-6 carbon atoms, and optionally further heteroatoms selected from O and N.

In an embodiment, **R1** and **R2** are identical.

In an embodiment, **R1** and **R2** are identical and represent a hexyl radical C₆H₁₃.

In case the linker **Z** is an organic linker comprising 1 to 5 C atoms and comprising at least one heteroatom selected from O and N, the hydrocarbon chain within the linker may be interrupted by amine units, ether units or ester units.

All **R1** can represent the same residue, selected from OH or CH₃, preferably CH₃.

If not otherwise stated, the heteroatom can be selected from Si, O, P and N, preferably O and N.

**R2** can be independently from each other selected from a linear or branched C6-C20 alkyl or alkenyl residue, in particular alkyl residue, preferably from a linear or branched C6-C12 alkyl or alkenyl residue, in particular alkyl residue, most preferably selected from the group consisting of -C₆H₁₃, -C₈H₁₇, -C₁₂H₂₅ and -CH₂-CH(C₆H₁₃)(C₄H₉).

All **R2** can represent the same residue, selected from a linear or branched C6-C20 alkyl residue, preferably a linear or branched C6-C12 alkyl residue, in particular selected from the group consisting of - C₆H₁₃, -C₈H₁₇, -C₁₂H₂₅ and -CH₂-CH(C₆H₁₃)(C₄H₉).

All **Y1** can represent the same linking group, selected from a C1-C10 hydrocarbon linking group, preferably a C1-C6 hydrocarbon linking group, more preferably a C1-C4 alkyl group, in particular -CH₂-CH₂-CH₂-.

All **Y2** can represent the same linking group, selected from a C1-C10 hydrocarbon linking group, preferably a C1-C6 hydrocarbon linking group, more preferably a C1-C4 alkyl linking group, more preferably - CH₂- or -CH₂-(CH₂)₂-CH₂-, in particular -CH₂-.

**X** can be selected from

**b¹**-(C=O)-**b**², **b¹**-(C=O)-O-**b²**, **b¹-**(C=O)-S-**b²** or **b¹-**(C=O)(N-**R'**)-**b²**,

wherein **b¹** represents the bond to the O atom and **b²** represents the bond to **Z** or the N atom, in case **Z** represents a chemical bond, and **R'** is selected from the group consisting of H and C1-C4 hydrocarbon, preferably H and C1-C4 alkyl, more preferably H or -CH₃, in particular H.

**Z** can represents a chemical bond or a C1-C5 alkyl linker, more preferably a chemical bond, -CH₂-CH₂- or -CH₂-CH₂-CH₂-, in particular -CH₂-CH₂- or -CH₂-CH₂-CH₂-.

Preferably, the ring, which **R3** and **R4** form together with the N atom they are bound to represents a pyrrolidine, piperidine, alkylpiperidine, such as methylpiperidine, azepane, alkylpiperazine, such as *N*-methylpiperazine, and morpholine.

Thus, Examples for cyclic organic residues **Q** are pyrrolidine, piperidine, alkylpiperidine, such as methylpiperidine, azepane, alkylpiperazine, such as *N*-methylpiperazine, and morpholine.

A preferred compound according to the invention is a compound according to chemical formula (II) 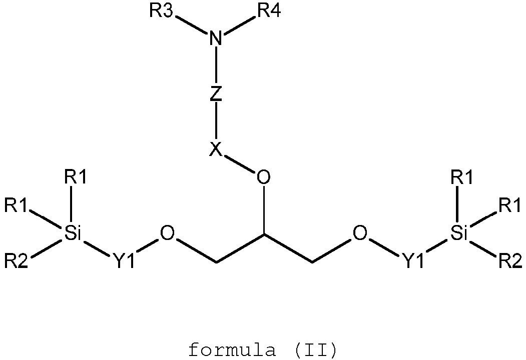 wherein
- **R1**: represents independently from each other H, OH, or a C1-C16 hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl group, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl group, in particular -CH₂-CH₂-CH₂-;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **R3** and **R4**: represent independently from each other a C1-C6 hydrocarbon residue, preferably a C1-C4 alkyl residues, in particular a methyl, ethyl or butyl residue,
or **R3** and **R4** can form a ring together, including the N atom they are bound to, wherein this ring comprises 4-8 carbon atoms, preferably 4-6 carbon atoms, and optionally heteroatoms selected from O and N.

All **R1** can represent the same residue, selected from OH or CH₃, preferably CH₃.

If not otherwise stated, said heteroatom can be selected from Si, O, P and N, preferably O and N.

**R2** can be independently from each other selected from a linear or branched C6-C20 alkyl residue, preferably from a linear or branched C6-C12 alkyl residue, in particular selected from the group consisting of -C₆H₁₃, -C₈H₁₇, -C₁₂H₂₅ and -CH₂-CH(C₆H₁₃)(C₄H₉).

All **R2** can represent the same residue, selected from a linear or branched C6-C20 alkyl residue, preferably a linear or branched C6-C12 alkyl residue, in particular selected from the group consisting of - C₆H₁₃, -C₈H₁₇, -C₁₂H₂₅ and -CH₂-CH(C₆H₁₃)(C₄H₉).

All **Y1** can represent the same residue, selected from a C1-C10 hydrocarbon linking group, preferably a C1-C6 hydrocarbon linking group, more preferably a C1-C4 alkyl group, in particular -CH₂-CH₂-CH₂-.

**X** can be selected from

**b¹-**(C=O)-**b**², **b¹-**(C=O)-O-**b²**, **b¹**-(C=O)-S-**b²** or **b¹**-(C=O)(N-**R'**)-**b**²,

wherein **b¹** represents the bond to the O atom and **b²** represents the bond to **Z** or the N atom, in case **Z** represents a chemical bond, and **R'** is selected from the group consisting of H and C1-C4 hydrocarbon, preferably H and C1-C4 alkyl, more preferably H or -CH₃, in particular H.

**Z** can represent a chemical bond or a C1-C5 alkyl linker, more preferably a chemical bond, -CH₂-CH₂- or -CH₂-CH₂-CH₂-, in particular -CH₂-CH₂- or -CH₂-CH₂-CH₂-.

Preferably, the ring, which **R3** and **R4** form together with the N atom they are bound to represents a pyrrolidine, piperidine, alkylpiperidine, such as methylpiperidine, azepane, alkylpiperazine, such as *N*-methylpiperazine, and morpholine.

A preferred compound according to the invention is a compound according to chemical formula (III) 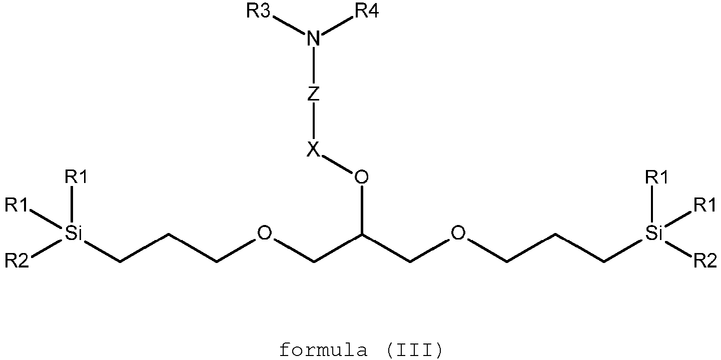 wherein
- **R1**: represents independently from each other H, OH, or a C1-C16 hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl group, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **R3** and **R4**: represent independently from each other a C1-C6 hydrocarbon residue, preferably a C1-C4 alkyl residues, in particular a methyl, ethyl or butyl residue,
or **R3** and **R4** can form a ring together, including the N atom they are bound to, wherein this ring comprises 4-8 carbon atoms, preferably 4-6 carbon atoms, and optionally heteroatoms selected from O and N.

All **R1** can represent the same residue, selected from OH or CH₃, preferably CH₃.

If not otherwise stated, said heteroatom can be selected from O, P and N, preferably O and N.

**R2** can be independently from each other selected from a linear or branched C6-C20 alkyl residue, preferably from a linear or branched C6-C12 alkyl residue, in particular selected from the group consisting of -C₆H₁₃, -C₈H₁₇, -C₁₂H₂₅ and -CH₂-CH(C₆H₁₃)(C₄H₉).

All **R2** can represent the same residue, selected from a linear or branched C6-C20 alkyl residue, preferably a linear or branched C6-C12 alkyl residue, in particular selected from the group consisting of - C₆H₁₃, -C₈H₁₇, -C₁₂H₂₅ and -CH₂-CH(C₆H₁₃)(C₄H₉).

**X** can be selected from

**b¹**-(C=O)-**b**², **b¹-**(C=O)-O-**b²**, **b¹**-(C=O)-S-**b²** or **b¹**-(C=O)(N-**R'**)-**b²**,

wherein **b¹** represents the bond to the O atom and **b²** represents the bond to **Z** or the N atom, in case **Z** represents a chemical bond, and **R'** is selected from the group consisting of H and C1-C4 hydrocarbon, preferably H and C1-C4 alkyl, more preferably H or -CH₃, in particular H.

**Z** can represent a chemical bond or a C1-C5 alkyl linker, more preferably a chemical bond, -CH₂-CH₂- or -CH₂-CH₂-CH₂-, in particular -CH₂-CH₂- or -CH₂-CH₂-CH₂-.

Preferably, the ring, which **R3** and **R4** form together with the N atom they are bound to represents a pyrrolidine, piperidine, alkylpiperidine, such as methylpiperidine, azepane, alkylpiperazine, such as *N*-methylpiperazine, and morpholine.

Preferably, the compound according to the invention is selected from the group consisting of the compounds according to chemical formula (IV) to (LV) as depicted in Table 1 below.

More preferably, the compound according to the invention is selected from the group consisting of the compound according to formulas (VI), (X) (XI), (XIII), (XVII), (XXV), (XXIX), (XXXI), (XXXVI), (XXXVII), (XXXVIII), (XLVI), (LII), (LIV), and (LV).

More preferably, the compound according to the invention is selected from the group consisting of the compound according to formulas (X), (XI), (XIII), (XVII), (XXV), (XXXI), (XXXVI), (XXXVII), (LII), and (LIV) .

In particular, the compound according to the invention is selected from the group consisting of the compounds according to formulas (X), (XI), (XIII), (XXXVII), (LII), and (LIV).

In an alternative preferred embodiment, the compound according to the invention is selected from the group constating of the compounds according to formulas (XLIX) to (LV).

Compounds according to the invention based on glycerol can be synthesized in general by the following method, the method comprising the following steps in the given order:
(A) Reacting allylglycidyl ether with allyl alcohol to prepare 1,3-bis(allyloxy)propan-2-ol (intermediate A). This step can be carried out by adding NaHSO₄ or MgHSO₄, preferably NaHSO₄. 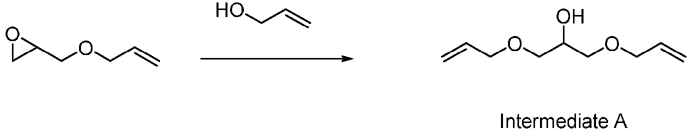
(B) Reacting 1,3-bis(allyloxy)propan-2-ol with a silane HSi(R1)₂R2 to produce intermediate B according to the chemical formula below. This step is preferably carried out via Pt catalysis, such as by using chloroplatinic acid. 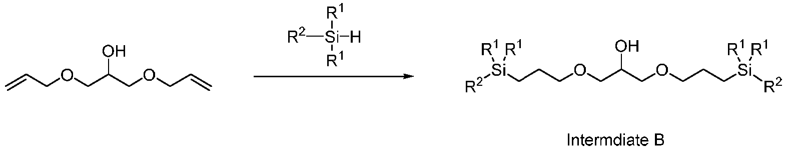
(C) Functionalizing the intermediate B according to one of the following steps (C1) to (C4):
   (C1) in case **X** represents together with the O-atom, to which **X** is bound, a carbonate based linker:
      Intermediate B is reacted with 4-nitrophenyl chloroformate followed by the reaction of an alcohol to produce the compound according to formula (III). This reaction can be carried out under addition of a base, such as 4-Dimethylaminophenol (4-DMAP), pyridine or *N,N-*diisopropylethylamine (DIPEA) 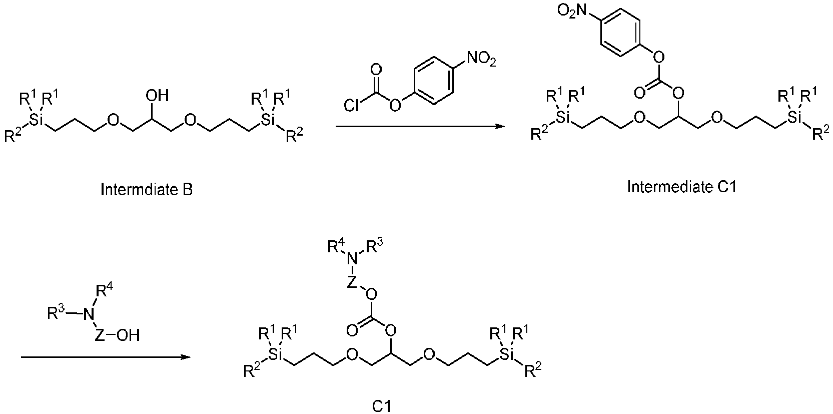
   (C2) in case **X** represents together with the O-atom, to which **X** is bound, a thio-carbonate based linker:
      Intermediate B is reacted with 4-nitrophenyl chloroformate followed by the reaction of an thiol to produce the compound according to formula (III) 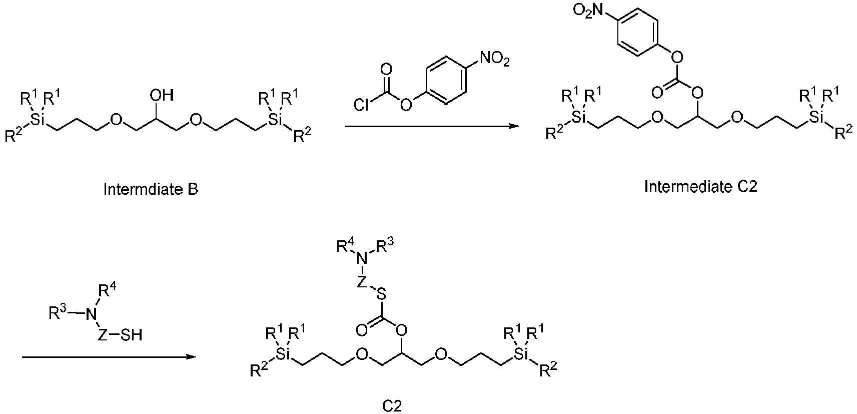
   (C3) in case **X** represents together with the O-atom, to which **X** is bound, a carbamate based linker:
      Intermediate B is reacted with 4-nitrophenyl chloroformate followed by the reaction of an amine to produce the compound according to formula (III) 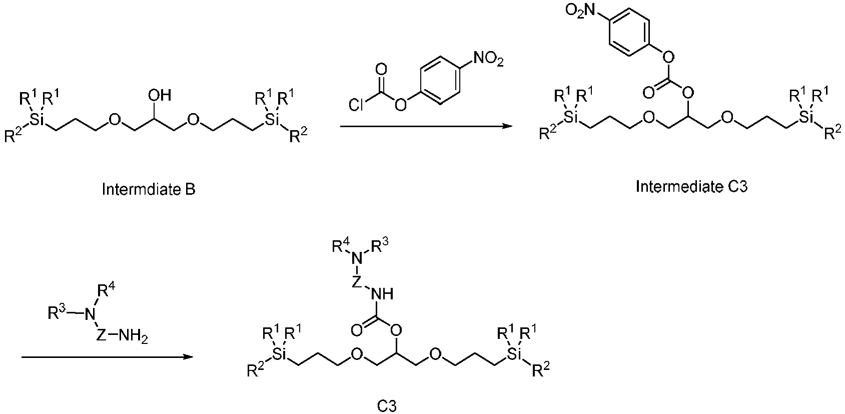
   (C4) in case **X** represents together with the O-atom, to which **X** is bound, an ester based linker:
      Intermediate B is reacted with a carboxylic acid to produce the compound according to formula (III). This reaction can be carried out according to a Steglich esterification using a coupling reagent, such as DIC or EDC, 4-DMAP and DIPEA, NEt3 or pyridine in dichlormethane (DCM).

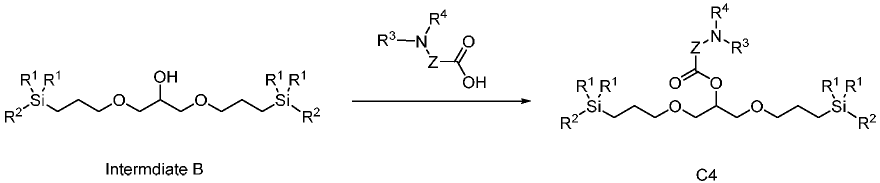

### Precursors synthesis

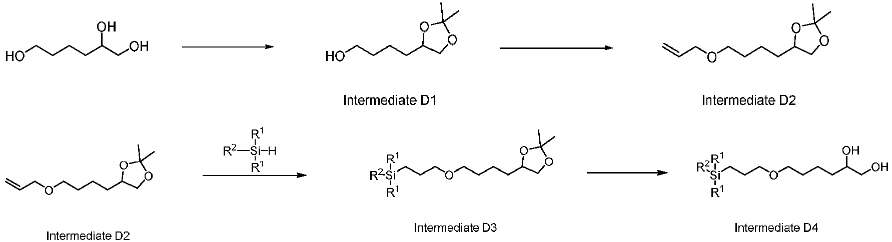

### Esterification

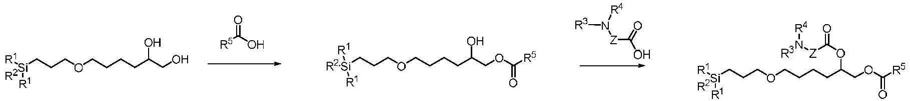

The present invention further covers the use of the compounds according to the present invention as an ionizable lipid.

A **further aspect of the present invention is directed to** Lipid nanoparticle (LNP) comprising at least one compound according to the present invention.

The at least one compound according to the present invention can be contained in a molar range of 10 mol% to 90 mol%, preferably 30 to 70 mol% based on the total amount of substance of the lipid nanoparticle.

An "ionizable lipid" is a type of lipid molecule that can acquire or lose a charge depending on the pH of its environment. These lipids typically contain functional groups that can be protonated or deprotonated, allowing them to transition between neutral and charged states. Ionizable lipids are crucial components in the formulation of lipid nanoparticles (LNPs) used, e.g., for drug delivery.

Preferably, the ionizable lipids according to the invention can become cationic by protonating the amine functionality within the molecule.

The term "Lipid Nanoparticles (LNPs)" represents particles at nanoscale, preferably spherical particles, made from ionizable lipids, such as the compounds of the present invention.

Typically, LNPs further comprise at least one non-cationic lipid and/or at least one conjugated lipid that prevents aggregation of the lipid and/or at least one active agent. Preferably, the at least one active agent is encapsulated by within the lipid. That way, the LNPs can represent a lipid-active agent particle or an active agent-lipid particle.

Preferably, the lipid nanoparticle further comprises at least one colloidal stabilizing lipid.

The colloidal stabilizing lipid is preferably a lipid that reduces, preferably inhibits, aggregation of the lipids.

The colloidal stabilizing lipid can be a conjugated lipid.

Preferably, the colloidal stabilizing lipid is a polyethylenglycol(PEG)-lipid conjugate.

The PEG-lipid conjugate can be contained in a molar range of 0.1 mol% to 5 mol% based on the total amount of substance of the lipid nanoparticle.

Preferably, the lipid nanoparticle further comprises a non-cationic lipid, more preferably selected from a sterol, a phospholipid or mixtures thereof.

The sterol can be cholesterol.

Cholesterol can be contained in a molar range of 10 mol% to 50 mol% based on the total amount of substance of the lipid nanoparticle.

The phospholipid can be DSPC(1,2-Distearoyl-sn-glycero-3-phosphocholin).

DSPC can be contained in a molar range of 1 mol% to 30 mol% based on the total amount of substance of the lipid nanoparticle.

Preferably, the lipid nanoparticle comprises at least one active agent.

The active ingredient can be a therapeutically active biomolecule, preferably selected from nucleic acid molecules, in particular from mRNA.

LNPs can contain further additives, such as solubility enhancers, cryoprotectants, more preferably in a molar range of 1 mol% to 20 mol% based on the total amount of substance of the lipid nanoparticle.

A **further aspect of the present invention** is directed to a pharmaceutical composition comprising a lipid nanoparticle according to the present invention, wherein the lipid nanoparticle comprises at least one active agent, and a pharmaceutically acceptable carrier.

A **further aspect of the present invention** is directed to a method for the in vivo delivery of an active agent, the method comprising administering to a mammalian subject a lipid nanoparticle, wherein the lipid nanoparticle comprises at least one active agent, or a pharmaceutical composition according to the invention.

A **further aspect of the present invention** is directed to a method for treating a disease or disorder in a mammalian subject in need thereof, the method comprising administering to the mammalian subject a therapeutically effective amount of a lipid nanoparticle, wherein the lipid nanoparticle comprises at least one active agent, or a pharmaceutical composition according to the invention.

A **further aspect of the present invention** is directed to a lipid nanoparticle, wherein the lipid nanoparticle comprises at least one active agent, or pharmaceutical composition according to the invention for use in the in vivo delivery of an active agent to a mammal.

A **further aspect of the present invention** is directed to a lipid nanoparticle, wherein the lipid nanoparticle comprises at least one active agent, or pharmaceutical composition according to the invention for use in the treatment of a disease or a disorder in a mammalian subject.

A **further aspect of the present invention** is directed to the use of a lipid nanoparticle, wherein the lipid nanoparticle comprises at least one active agent, or a pharmaceutical composition according to the present invention to prepare a medicament for the in vivo delivery of an active agent to a mammal.

A **further aspect of the present invention** is directed to the use of a lipid nanoparticle, wherein the lipid nanoparticle comprises at least one active agent, or a pharmaceutical composition according to the present invention to prepare a medicament for the treatment of a disease or a disorder in a mammalian subject.

### Examples

The present invention and its accompanying surprising technical advantages are further illustrated by the following examples, without being limited thereto.

The following abbreviations are used.
- CH: Cyclohexane
- 4-DMAP: 4-*N*,*N*-dimethylaminopyridine
- DCM: Dichloromethane
- EDC-HCl: 1-Ethyl-3-(3-dimethylaminopropyl)carbodiimid
- DIPEA: *N*,*N*-Diisopropylethylamine
- HRMS: High resolution mass spectrometry
- Karstedt's: Platinum-divinyltetramethyldisiloxane complex in
- catalyst: xylene (2.1-2.4%Pt)
- NMR: Nuclear magnetic resonance
- d, t, m, s: dublet, tritplet, multiplet, singlet
- br: broad
- T3P: Propanephosphonic acid anhydride
- TLC: Thin layer chromatography
- THF: Tetrahydrofuran

### 1 Sila-Glycerin Framework

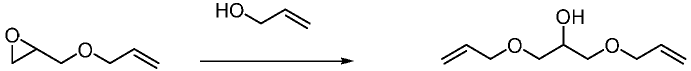

### Synthesis of 1,3-bis(allyloxy)propan-2-ol

Under argon atmosphere, allylglycidyl ether (82.5 mL, 700 mmol, 1.0 equiv.) was dissolved in allyl alcohol (336 mL, 5.0 mol, 7.0 equiv.) and NaHSO₄ (180 g, 1.5 mol, 3.0 equiv.) was added. The reaction was stirred at rt for 1.5 h (TLC control) and the volatiles were removed under reduced vacuum followed by extraction with DCM and H₂O (2x). The combined organic extracts were washed with brine and dried over Na₂SO₄. After filtration, the solvent was evaporated. The crude product was purified by distillation (0.3 mbar, 50°C) and the product was obtained as colorless oil (67.5 g, 390 mmol, 56%)

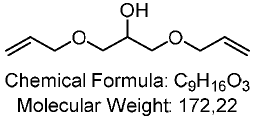

**¹H NMR** (CDCl₃, 500 MHz) δ [ppm]: 5.94 (ddd, 2H), 5.27 (dd, 2H), 5.18 (dd, 2H), 4.01 (ddd, 4H), 4.00 - 3.94 (m, 1H), 3.49 (ddd, 4H). OH is not visible.

¹³**C NMR** (CDCl₃, 125 MHz) δ [ppm]: 134.5 (2C), 117.2 (2C), 72.4 (2C), 71.3 (2C), 69.5 (1C).

### 1.1 Hydrosilylation with different H-Silanes

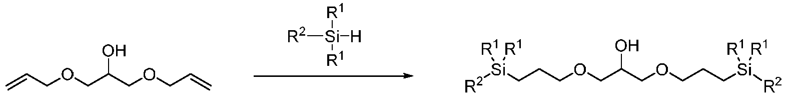

### Synthesis of 9,9,21,21-Tetramethyl-13,17-dioxa-9,21-disila-nonacosan-15-ol

1,3-Bis(allyloxy)propan-2-ol (4.6 g, 21.9 mmol, 1.0 equiv.) was dissolved in toluene (55 mL) under argon atmosphere and Karstedt's catalyst (200 ppm) was added). The mixture was heated to 80°C and octyldimethylsilane (8.19 g, 24.5 mmol, 2.17 equiv.) was added over 6 h. The reaction was stirred at 80°C for 26 h while small portions of catalyst were added (in sum 1000 ppm). After cooling to rt, the solvents were removed under reduced vacuum and the crude product was purified by flash column chromatography on silica (120 g Ecoflex, 85 mL/min, cyclohexane / acetone = 98:2 → 85:15). The product was obtained as colorless oil (4.63 g, 8.59 mmol, 41%). 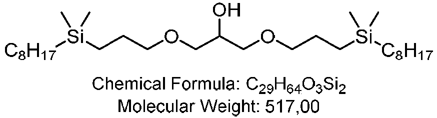 NMR spectra show signals of cyclohexane.

**¹H NMR** (CDCl₃, 500 MHz) δ [ppm]: 3.96 - 3.91 (m, 1H), 3.50 - 3.37 (2x m, 8H), 2.45 (d, OH), 1.59 - 1.50 (m, 4H), 1.32 - 1.19 (m, 24H), 0.86 (t, 6H), 0.49 - 0.41 (2x m, 8H), -0.06 (s, 12H).

¹³**C NMR** (CDCl₃, 125 MHz) δ [ppm]: 74.6 (2C), 71.9 (2C), 69.5 (1C), 33.7 (2C), 32.0 (2C), 29.4 (2C), 29.3 (2C), 24.0 (2C), 23.9 (2C), 22. 7 (2C), 15.2 (2C), 14.1 (2C), 11.1 (2C), -3.5 (4C).

### Synthesis of 9,9,21,21-Tetramethyl-13,17-dioxa-9,21-disila-nonacosan-15-ol

1,3-Bis(allyloxy)propan-2-ol (1.44 g, 8.34 mmol, 1.0 equiv.) was dissolved in toluene (20 mL) under argon atmosphere and Karstedt's catalyst (200 ppm) was added. The mixture was heated to 80°C and dodecyldimethylsilane (3.70 g, 16.19 mmol, 2.17 equiv.) was added over 5 h. The reaction was stirred at 80°C for 72 h while small portions of catalyst were added (in sum 1000 ppm). After cooling to rt, the solvents were removed under reduced pressure and the crude product was purified by flash column chromatography on silica (40 g Ecoflex, 45 mL/min, cyclohexane/ethyl acetate = 98:2 → 65/35). The product was obtained as colorless oil (2.19 g, 3.48 mmol, 47%).

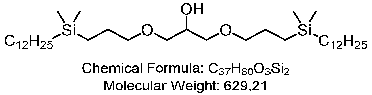

NMR spectra show traces of cyclohexane.

**¹H NMR** (CDCl₃, 500 MHz) δ [ppm] : 3.96 - 3.91 (m, 1H), 3.50 - 3.37 (2x m, 8H), 1.59 - 1.48 (m, 4H), 1.32 - 1.19 (m, 40H), 0.86 (t, 6H), 0.49 - 0.39 (2x m, 8H), -0.06 (s, 12H). OH is not visible.

¹³**C NMR** (CDCl₃, 125 MHz) δ [ppm]: 74.6 (2C), 71.9 (2C), 69.5 (1C), 33.7 (2C), 32.0 (2C), 29.7 (2C), 29.7 (2C), 29.7 (2C), 29.6 (2C), 29. 4 (2C), 29.4 (2C), 24.0 (2C), 23.9 (2C), 22.7 (2C), 15.2 (2C), 14. 1 (2C), 11.1 (2C), -3.5 (4C).

### Synthesis of 13,13,25,25-Tetramethyl-17,21-dioxa-13,15-disila-heptatriacontan-19-ol

1,3-Bis(allyloxy)propan-2-ol (4.6 g, 21.9 mmol, 1.0 equiv.) was dissolved in toluene (25 mL) under argon atmosphere and Karstedt's catalyst (200 ppm) was added. The mixture was heated to 80°C and 2-butyloctyldimethylsilane (3.81 g, 16.7 mmol, 2.0 equiv.) was added over 4 h. The reaction was stirred at 80°C for 28 h while small portions of catalyst were added (in sum 1000 ppm). After cooling to rt, the solvents were removed under reduced vacuum and the crude product was purified by flash column chromatography on silica (40 g Ecoflex, 45 mL/min, cyclohexane/ethyl acetate = 100:0 → 80:20). The product was obtained as colorless oil (1.19 g, 1.89 mmol, 21%).

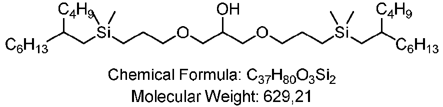

**¹H NMR** (CDCl₃, 500 MHz) δ [ppm] : 3.96 - 3.91 (m, 1H), 3.50 - 3.34 (2x m, 8H), 1.59 - 1.48 (m, 4H), 1.46 - 1.36 (m, 2H), 1.32 - 1.19 (m, 32H), 0.86 (t, 6H), 0.85 (t, 6H), 0.48 (d, 4H), 0.46 - 0.39 (m, 4H), -0.04 (s, 12H). OH is not visible.

¹³**C NMR** (CDCl₃, 125 MHz) δ [ppm]: 74.6 (2C), 71.9 (2C), 69.5 (1C), 36.7 (2C), 36.3 (2C), 33.9 (2C), 32.0 (2C), 29.7 (2C), 28.8 (2C), 26.5 (2C), 24.1 (2C), 23.1 (2C), 22.7 (2C), 20.4 (2C), 14.2 (2C), 14.1 (2C), 12.0 (2C), -2.2 (4C).

### Synthesis of 9,25-dihexyl-11,11,23,23-tetramethyl-15,19-dioxa-11,23-disilatritriacontan-17-ol

1,3-Bis(allyloxy)propan-2-ol (1.03 g, 5.80 mmol, 1.0 equiv.) was dissolved in toluene (20 mL) under argon atmosphere and Karstedt's catalyst (200 ppm) was added. The mixture was heated to 80°C and 2-Hexyldodecyldimethylsilane (3.30 g, 11.6 mmol, 2.0 equiv.) was added over 4 h. The reaction was stirred at 80°C for 26 h while small portions of catalyst were added (in sum 1000 ppm). After cooling to rt, the solvents were removed under reduced vacuum and the crude product was purified by flash column chromatography on silica (40 g Ecoflex, 5 mL/min, *n*-heptane = 100). The product was obtained as colorless oil (920 mg, 1.24 mmol, 21%).

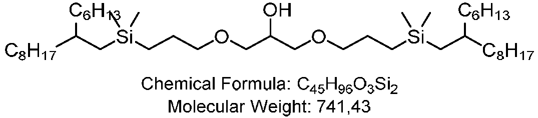

**¹H NMR** (CDCl₃, 500 MHz) δ [ppm] : 3.96 - 3.91 (m, 1H), 3.50 - 3.32 (2x m, 8H), 1.59 - 1.51 (m, 4H), 1.44 - 1.36 (m, 2H), 1.32 - 1.19 (m, 48H), 0.85 (t, 12H), 0.49 (d, 4H), 0.46 - 0.41 (m, 4H), -0.04 (s, 12H). OH is not visible.

¹³**C NMR** (CDCl₃, 125 MHz) δ [ppm]: 74.6 (2C), 71.9 (2C), 69.5 (1C), 36.7 (4C), 34.0 (2C), 32.0 (2C), 31.9 (2C), 30.1 (2C), 29.8 (2C), 29.7 (2C), 29.4 (2C), 26.6 (2C), 26.5 (2C), 24.1 (2C), 22.7 (2x 2C), 20.4 (2C), 14.1 (4C), 12.0 (2C), -2.4 (4C).

### 1.2 Carbonate Formation Process

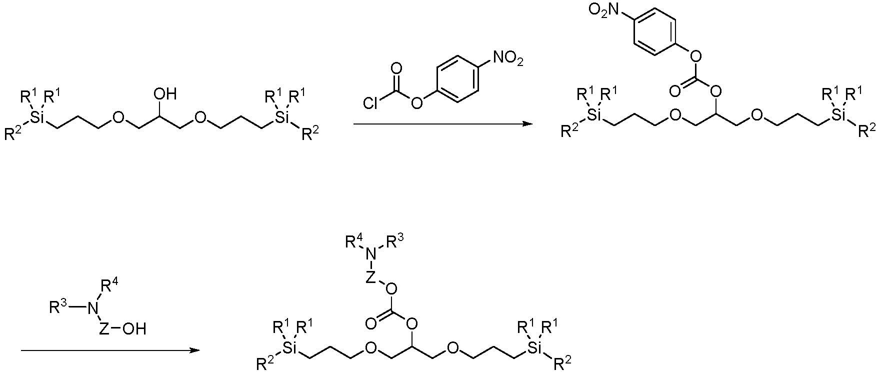

### Synthesis of 4-nitrophenyl carbonate esters

9,9,21,21-Tetramethyl-13,17-dioxa-9,21-disila-nonacosan-15-ol (2.00 g, 4.30 mmol, 1.0 equiv.), 4-DMAP (52.5 mg, 0.43 mmol. 0.1 equiv.) and 4-nitrophenyl chloroformate (953 mg, 4.73 mmol, 1.1 equiv.) were dissolved in DCM (20 mL) in the presence of pyridine (520 µL, 6.45 mmol, 1.5 equiv.) under argon atmosphere. The reaction was stirred at rt overnight (TLC control). The solvent was removed under reduced pressure and the residue was washed with cyclohexane (3 x 30 mL). After solvent removal of the filtrate, the product was obtained as colorless oil (2.36 g, 3.46 mmol, 80%).

### General procedure 1

The 4-nitrophenyl carbonate ester (250 mg, 367 µmol) was dissolved in DCM (5.0 mL) under argon atmosphere. 4-DMAP (4.5 mg, 36.7 µmol, 0.1 equiv.), pyridine (497 mg, 2.2 mmol, 6.0 equiv.) and the corresponding amino alcohol (1.47 mmol, 4.0 equiv.) was added. The reaction was stirred at rt for 16 - 72 h (TLC control). After completion, H₂O (25 mL) was added, and the reaction was extracted with DCM (2x 25 mL). The combined organic phases were washed with brine and after phase separation, dried over Na₂SO₄ and filtered. The solvent was removed under reduced pressure and the crude product was purified by flash column chromatography on silica (4 g Ecoflex, 15 mL/min, cyclohexane/ethyl acetate = 98:2 → 65:35, condition A or CH/EtOAc = 100:0 → 0:100, condition B or DCM/MeOH 100:0 - 95:5, conditions D).

### Example 1: 2-(Diethylamino)ethyl (9,9,21,21-tetramethyl-13,17-dioxa-9,21-disilanonacosan-15-yl) carbonate

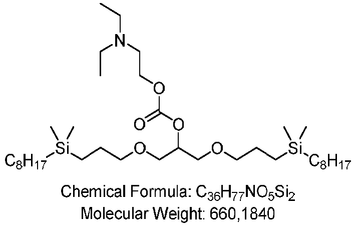

The synthesis followed general procedure 1 using 2-diethylaminoethanol (159 mg, 1.36 mmol, 4.0 equiv.). The product was obtained as yellowish oil (20 mg, 30 µmol, 9%) after flash column chromatography using conditions B.

NMR spectra contain traces of solvents.

**¹H NMR** (CDCl₃, 500 MHz) δ [ppm]: 4.93 (pent, 1H), 4.2 (brs, 2H), 3.62 - 3.53 (m, 4H), 3.44 - 3.30 (m, 4H), 2.75 (brs, 2H), 2.59 (brs, 4H), 1.57 - 1.46 (m, 4H), 1.32 - 1.16 (m, 24H), 1.03 (t, 6H), 0.85 (t, 6H), 0.50 - 0.39 (2xm, 8H), -0.07 (s, 12H).

¹³**C NMR** (CDCl₃, 125 MHz) δ [ppm] : 154.8 (C_{q}), 75.8 (1C), 74.6 (2C), 69.2 (2C), 65.8 (br, 1C), 50.9 (1C), 47.8 (2C), 33.7 (2C), 31.9 (2C), 29.4 (2C), 29.3 (2C), 24.0 (2C), 23.9 (2C), 22.7 (2C), 15.2 (2C), 14.1 (2C), 11.7 (br, 2C), 11.0 (2C), -3.4 (4C).

**HRMS** (+ESI) *m*/*z* calcd for C₃₆H₇₇NO₅Si₂ [M+H]⁺: 660.5415; found: 660.5416.

### Example 2: 3-(Diethylamino)propyl (9,9,21,21-tetramethyl-13,17-dioxa-9,21-disilanonacosan-15-yl) carbonate

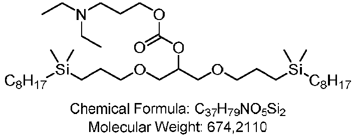

The synthesis followed general procedure 1 using 3-dimethylamino-1-propanol (192 mg, 1.47 mmol, 4.0 equiv.). The product was obtained as yellowish oil (166 mg, 246 µmol, 67%) after flash column chromatography using conditions A.

**¹H NMR** (CDCl₃, 500 MHz) δ [ppm]: 4.93 (pent, 1H), 4.18 (t, 2H), 3.62 - 3.53 (m, 4H), 3.44 - 3.33 (m, 4H), 2.67 (brs, 6H), 1.94 (brs, 2H), 1.58 - 1.47 (m, 4H), 1.32 - 1.16 (m, 24H), 1.11 (brs, 6H), 0.87 (t, 6H), 0.50 - 0.39 (2xm, 8H), -0.06 (s, 12H).

¹³**C NMR** (CDCl₃, 125 MHz) δ [ppm]: 154.8 (C_{q}), 75.6 (1C), 74.6 (2C), 69.1 (2C), 66.5 (1C), 49.1 (1C), 46.9 (2C), 33.7 (2C), 31.9 (2C), 29.4 (2C), 29.3 (2C), 26.2 (1C), 24.0 (2C), 23.8 (2C), 22.7 (2C), 15.1 (2C), 14.1 (2C), 11.5 (2C), 11.0 (2C), -3.4 (4C).

**HRMS** (+ESI) *m*/*z* calcd for C₃₇H₇₉NO₅Si₂ [M+H]⁺: 674.5572; found: 674.5567.

### Example 3: 2-(2-(Dimethylamino)ethoxy)ethyl (9,9,21,21-tetramethyl-13,17-dioxa-9,21-disilanonacosan-15-yl) carbonate

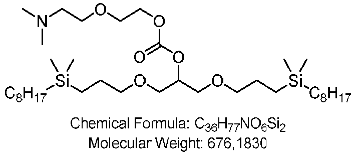

The synthesis followed general procedure 1 using 2-[2-(dimethylamino) ethoxy] ethanol (195 mg, 1.47 mmol, 4.0 equiv.). The product was obtained as yellowish oil (57 mg, 75.4 µmol, 25%) after flash column chromatography using conditions A.

**¹H NMR** (CDCl₃, 500 MHz) δ [ppm]: 4.94 (pent, 1H), 4.29 - 4.24 (m, 2H), 3.70 - 3.62 (m, 4H), 3.62 - 3.54 (m, 4H), 3.44 - 3.32 (m, 4H), 2.63 (t, 2H), 2.37 (s, 6H), 1.58 - 1.47 (m, 2H), 1.32 - 1.16 (m, 26H), 0.87 (t, 6H), 0.50 - 0.39 (2xm, 8H), -0.06 (s, 12H).

¹³**C NMR** (CDCl₃, 125 MHz) δ [ppm]: 154.8 (C_{q}), 75.9 (C), 74.6 (2C), 69.2 (2C), 68.8 (1C), 68.6 (1C), 66.9 (1C), 58.4 (1C), 45.4 (2C), 33.7 (2C), 32.0 (2C), 29.4 (2C), 29.3 (2C), 24.0 (2C), 23.9 (2C), 22. 7 (2C), 15.2 (2C), 14.1 (2C), 11.0 (2C), -3.4 (4C).

**HRMS** (+ESI) *m*/*z* calcd for C₃₆H₇₇NO₆Si₂ [M+H]⁺: 676.5364; found: 676.5362.

### Example 4: 2-Morpholinoethyl (9,9,21,21-tetramethyl-13,17-dioxa-9,21-disilanonacosan-15-yl) carbonate

The synthesis followed general procedure 1 using 4-morpholineethanol (193 mg, 1.47 mmol, 4.0 equiv.). The product was obtained as yellowish oil (47 mg, 70 µmol, 19%) after flash column chromatography using conditions A.

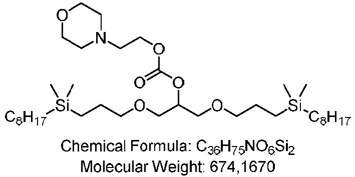

NMR spectrum contains 4-DMAP and residues of the solvent.

**¹H NMR** (CDCl₃, 500 MHz) δ [ppm]: 4.95 (pent, 1H), 4.27 (brs, 2H), 3.71 (brs, 4H), 3.62 - 3.52 (m, 4H), 3.45 - 3.32 (m, 4H), 2.68 (brs, 2H), 2.53 (brs, 4H), 1.58 - 1.48 (m, 4H), 1.30 - 1.18 (m, 24H), 0.87 (t, 6H), 0.49 - 0.39 (2xm, 8H), -0.07 (s, 12H).

¹³**C NMR** (CDCl₃, 125 MHz) δ [ppm] : 154.6 (weak, C_{q}), 76.0 (1C), 74. 6 (2C), 69.1 (2C), 66.3 (br, 2C), 64.3 (br, 1C), 58.8 (1C), 53.6 (2C), 33.7 (2C), 32.0 (2C), 29.4 (2C), 29.3 (2C), 24.0 (2C), 23.9 (2C), 22.7 (2C), 15.2 (2C), 14.1 (2C), 11.1 (2C), -3.4 **(4C).HRMS** (+ESI) *m*/*z* calcd for C₃₆H₇₅NO₆Si₂ [M+H]⁺: 674.5208; found: 674.5199.

### Example 5: 2-(Pyrrolidin-1-yl)ethyl (9,9,21,21-tetramethyl-13,17-dioxa-9,21-disilanonacosan-15-yl) carbonate

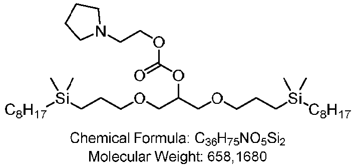

The synthesis followed general procedure 1 using 1-(2-hydroxyethyl)pyrrolidine (192 mg, 1.47 mmol, 4.0 equiv.). The product was obtained as yellowish oil (145 mg, 220 µmol, 61%) after flash column chromatography using conditions A.

**¹H NMR** (CDCl₃, 500 MHz) δ [ppm]: 4.94 (pent, 1H), 4.29 - 4.24 (m, 2H), 3.70 - 3.62 (m, 4H), 3.62 - 3.54 (m, 4H), 3.44 - 3.32 (m, 4H), 2.63 (t, 2H), 2.37 (s, 6H), 1.58 - 1.47 (m, 2H), 1.32 - 1.16 (m, 24H), 0.87 (t, 6H), 0.50 - 0.39 (2xm, 8H), -0.06 (s, 12H).

¹³**C NMR** (CDCl₃, 125 MHz) δ [ppm]: 154.7 (C_{q}), 75. (1C), 74.6 (2C), 69.2 (2C), 66.5 (1C), 54.5 (2C), 54.1 (1C), 33.7 (2C), 32.0 (2C), 29.4 (2C), 29.3 (2C), 24.0 (2C), 23.9 (2C), 23.5 (2C), 22.7 (2C), 15.2 (2C), 14.1 (2C), 11.0 (2C), -3.4 (4C).

**HRMS** (+ESI) *m*/*z* calcd for C₃₆H₇₅NO₅Si₂ [M+H]⁺: 658.5259; found: 658.5253.

### Example 6: 2-(Piperidin-1-yl)ethyl (9,9,21,21-tetramethyl-13,17-dioxa-9,21-disilanonacosan-15-yl) carbonate

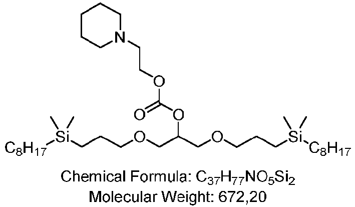

The synthesis followed general procedure 1 using 1-(2-hydroxyethyl)piperidine (169 mg, 1.47 mmol, 4.0 equiv.). The product was obtained as yellowish oil (173 mg, 257 µmol, 70%) after flash column chromatography using conditions A.

**¹H NMR** (CDCl₃, 500 MHz) δ [ppm]: 4.93 (pent, 1H), 4.41 - 4.17 (m, 2H), 3.61 - 3.54 (m, 4H), 3.43 - 3.32 (m, 4H), 2.68 (brs, 2H), 2.47 (brs, 4H), 1.62 (brs, 4H), 1.56 - 1.47 (m, 4H), 1.47 - 1.39 (brs, 2H), 1.31 - 1.16 (m, 24H), 0.87 (t, 6H), 0.50 - 0.39 (2xm, 8H), -0.06 (s, 12H).

¹³**C NMR** (CDCl₃, 125 MHz) δ [ppm]: 154.7 (C_{q}), 75.8 (1C), 74.6 (2C), 69.2 (2C), 65.2 (1C), 57.0 (1C), 54.7 (2C), 33.7 (2C), 32.0 (2C), 29.4 (2C), 29.3 (2C), 25.6 (2C), 23.9 (2C), 23.8 (2C), 23.9 (1C), 22.7 (2C), 15.1 (2C), 14.1 (2C), 11.0 (2C), -3.4 (4C).

**HRMS** (+ESI) *m*/*z* calcd for C₃₇H₇₇NO₅Si₂ [M+H]⁺: 672.5415; found: 672.5419.

### Example 7: 2-(4-Methylpiperazin-1-yl)butyl (9,9,21,21-tetramethyl-13,17-dioxa-9,21-disilanonacosan-15-yl) carbonate

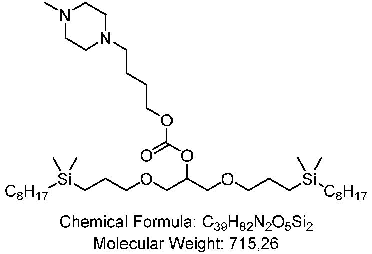

The synthesis followed general procedure 1 using 4-(4-methylpiperazin-1-yl)butan-1-ol (253 mg, 1.47 mmol, 4.0 equiv.). The product was obtained as yellowish oil (122 mg, 171 µmol, 43%) after flash column chromatography using conditions A.

NMR spectra contain unknown impurities.

**¹H NMR** (CDCl₃, 500 MHz) δ [ppm]: 4.93 (pent, 1H), 4.12 (t, 2H), 3.62 - 3.54 (m, 4H), 3.43 - 3.32 (m, 4H), 2.74 (bs, 8H), 2.51 (bs, 5H), 1.72 - 1.64 (m, 2H), 1.56 - 1.45 (m, 4H), 1.33 - 1.16 (m, 26H), 0.87 (t, 6H), 0.50 - 0.39 (2xm, 8H), -0.06 (s, 12H).

¹³**C NMR** (CDCl₃, 125 MHz) δ [ppm]: 154.9 (C_{q}), 75.6 (1C), 74.6 (2C), 69.2 (2C), 67.8 (1C), 57.8 (1C), 54.8 (2C), 52.7 (2C), 45.7 (1C), 33.7 (2C), 32.0 (2C), 29.4 (2C), 29.3 (2C), 26.6 (1C), 24.0 (2C), 23. 9 (2C), 23.0 (1C), 22.7 (2C), 15.2 (2C), 14.1 (2C), 11.1 (2C), -3.4 (4C).

### Example 8: 3-(Dimethylamino)propyl (9,9,21,21-tetramethyl-13,17-dioxa-9,21-disilanonacosan-15-yl) carbonate

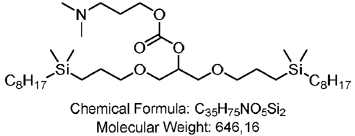

The synthesis followed general procedure 1 using 3-(dimethylamino)-1-propanol (121 mg, 1.17 mmol, 4.0 equiv.). The product was obtained as yellowish oil (37 mg, 57 µmol, 20%) after flash column chromatography using conditions B.

**¹H NMR**(CDCl₃, 500 MHz) δ [ppm]: 4.93 (pent, 1H), 34.18 (t, 2H), 3.62 - 3.54 (m, 4H), 3.44 - 3.32 (m, 4H), 2.60 (t, 2H), 2.22 (s, 6H), 1.96 (pent, 2H), 1.55 - 1.47 (m, 4H), 1.32 - 1.18 (m, 24H), 0.86 (t, 6H), 0.50 - 0.39 (2xm, 8H), -0.07 (s, 12H).

¹³**C NMR** (CDCl₃, 125 MHz) δ [ppm]: 154.7 (C_{q}), 75.7 (1C), 74.6 (2C), 69.1 (2C), 65.7 (1C), 55.8 (1C), 44.5 (2C), 33.7 (2C), 31.9 (2C), 29.3 (2C), 29.2 (2C), 25.9 (1C), 23.9 (2C), 23.8 (2C), 22.6 (2C), 15.1 (2C), 14.1 (2C), 11.0 (2C), -3.5 (4C).

**HRMS** (+ESI) *m*/*z* calcd for C₃₅H₇₅NO₅Si₂ [M+H]⁺: 646.5259; found: 646.5257.

### Example 9: S-(3-(Dimethylamino)propyl) O-(9,9,21,21-tetramethyl-13,17-dioxa-9,21-disilanonacosan-15-yl) carbonothioate

The synthesis followed general procedure 1 using 3-(dimethylamino)-1-propanethiol (139 mg, 1.17 mmol, 4.0 equiv.). The product was obtained as colorless oil (156 mg, 23 µmol, 80%) after flash column chromatography using conditions B.

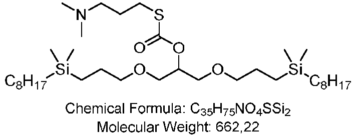

NMR contains traces of cyclohexane.

**¹H NMR** (CDCl₃, 500 MHz) δ [ppm]: 5.12 (pent, 1H), 3.62 - 3.51 (m, 4H), 3.43 - 3.32 (m, 4H), 2.87 (t, 2H), 2.35 (t, 2H), 2.22 (s, 6H), 1.81 (pent, 2H), 1.55 - 1.46 (m, 4H), 1.33 - 1.18 (m, 24H), 0.85 (t, 6H), 0.50 - 0.39 (2xm, 8H), -0.07 (s, 12H).

¹³**C NMR** (CDCl₃, 125 MHz) δ [ppm]: 170.9 (C_{q}), 75.0 (1C), 74.6 (2C), 69.1 (2C), 58.1 (1C), 45.3 (2C), 33.7 (2C), 32.0 (2C), 29.4 (2C), 29.3 (2C), 28.9 (1C), 27.6 (1C), 24.0 (2C), 23.9 (2C), 22.7 (2C), 15.2 (2C), 14.1 (2C), 11.0 (2C), -3.4 (4C).

**HRMS** (+ESI) *m*/*z* calcd for C₃₅H₇₅NO₄SSi₂ [M+H]⁺: 662.5030; found: 662.5030.

### Example 10: 2-((2-(Dimethylamino)ethyl)(methyl) amino) ethyl (9,9,21,21-tetramethyl-13,17-dioxa-9,21-disilanonacosan-15-yl) carbonate

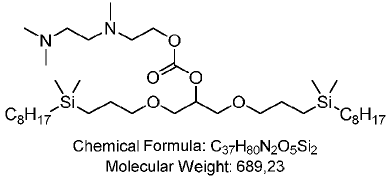

The synthesis followed general procedure 1 using 2-[[2-(dimethylamino)ethyl]methylamino]ethanol (171 mg, 1.17 mmol, 4.0 equiv.). The product was obtained as yellowish oil (25 mg, 36 µmol, 12%) after flash column chromatography using conditions A.

**¹H NMR** (CDCl₃, 500 MHz) δ [ppm]: 4.92 (pent, 1H), 4.20 (t, 2H), 3.62 - 3.54 (m, 4H), 3.45 - 3.32 (m, 4H), 2.68 (t, 2H), 2.59 (t, 2H), 2.49 (t, 2H), 2.32 (s, 6H), 2.29 (s, 3H), 1.55 - 1.48 (m, 4H), 1.33 - 1.18 (m, 24H), 0.85 (t, 6H), 0.50 - 0.39 (2xm, 8H), -0.06 (s, 12H).

¹³**C NMR** (CDCl₃, 125 MHz) δ [ppm]: 154.8 (C_{q}), 75.7 (1C), 74.6 (2C), 69.1 (2C), 65.6 (1C), 57.0 (1C), 55.9 (1C), 55.4 (1C), 45.6 (2C), 43.8 (1C), 33.7 (2C), 31.9 (2C), 29.3 (2C), 29.2 (2C), 24.0 (2C), 23.8 (2C), 22.7 (2C), 15.1 (2C), 14.1 (2C), 11.0 (2C), -3.5 (4C).

**HRMS** (+ESI) *m*/*z* calcd for C₃₇H₈₀N₂O₅Si₂ [M+H]⁺: 689.5681; found: 689.5679.

### Example 11: 3-(Dimethylamino)butyl (9,9,21,21-tetramethyl-13,17-dioxa-9,21-disilanonacosan-15-yl) carbonate

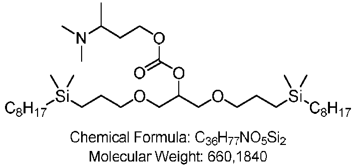

The synthesis followed general procedure 1 using 3-(dimethylamino)-1-butanol (69 mg, 58.8 µmol, 4.0 equiv.). The product was obtained as yellowish oil (6.7 mg, 10 µmol, 7%) after flash column chromatography using conditions D.

NMR spectra contain unknown impurities.

**¹H NMR** (CDCl₃, 500 MHz) δ [ppm]: 4.93 (pent, 1H), 4.26 - 4.12 (m, 2H), 3.64 - 3.52 (m, 4H), 3.45 - 3.32 (m, 4H), 2.86 (brs, 1H), 2.31 (s, 6H), 2.01 (brs, 1H), 1.68 - 1.60 (m, 1H), 1.58 - 1.46 (m, 4H), 1.30 - 1.18 (m, 24H), 1.05 (d, 3H), 0.86 (t, 6H), 0.50 - 0.39 (2xm, 8H), - 0.06 (s, 12H).

**¹³C NMR** (CDCl₃, 125 MHz) δ [ppm]: 154.8 (C_{q}), 75.7 (1C), 74.6 (2C), 69.1 (2C), 65.6 (1C, weak), 56.5 (1C, weak), 40.0 (2C), 33.7 (2C), 32.0 (2C), 31.7 (1C), 29.4 (2C), 29.3 (2C), 24.0 (2C), 23.9 (2C), 22.7 (2C), 15.2 (2C), 14.1 (2C), 13.3 (1C), 11.0 (2C), -3.4 (4C).

**HRMS** (+ESI) *m*/*z* calcd for C₃₆H₇₇NO₅Si₂ [M+H]⁺: 660.5415; found: 660.5418.

### 1.3 Carbamate Formation Process

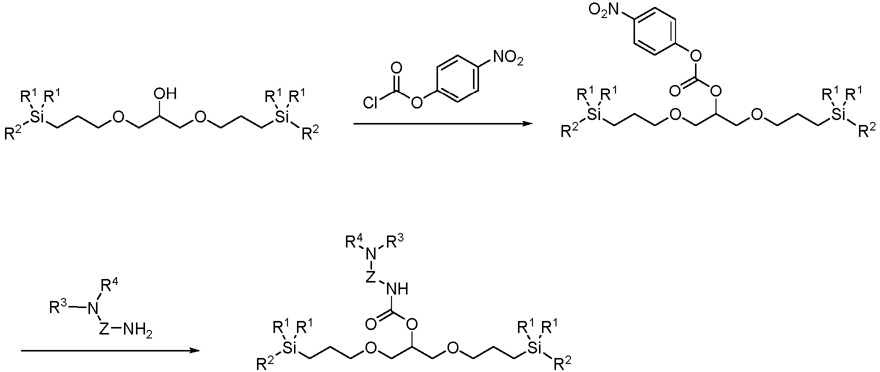

### General procedure 2

The 4-nitrophenyl carbonate ester (200 mg, 293 µmol) was dissolved in DCM (5.0 mL) under argon atmosphere. 4-DMAP (72 mg, 586 µmol, 2.0 equiv.), pyridine (139 mg, 1.76 mmol, 6.0 equiv.) and the corresponding amino amine (1.17 mmol, 4.0 equiv.) was added. The reaction was stirred at rt for 16 - 72 h (TLC control). After completion, the solvent was removed under reduced pressure and heptane (5.0 mL) was added. After filtration of the precipitate, the solvent was removed and the crude product was purified by flash column chromatography on silica (4 g Ecoflex, 15 mL/min, cyclohexane/ethyl acetate = 98:2 → 65:35, condition A or cyclohexane/ethyl acetate = 100:0 → 0:100, condition B or dichloromethane/methanol = 99.5:0.5 - 92:8 condition C).

### Example 12: 9,9,21,21-Tetramethyl-13,17-dioxa-9,21-disilanonacosan-15-yl (3-(diethylamino)propyl)-carbamate

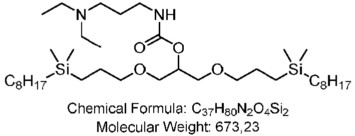

The synthesis followed general procedure 2 using N,N-diethyl-1,3-propanediamine (191 mg, 1.47 mmol, 4.0 equiv.). The product was obtained as clear oil (82 mg, 0.12 mmol, 35%) after flash column chromatography using conditions B.

**¹H NMR** (acetone-d6, 500 MHz) δ [ppm]: 4.92 (pent, 1H), 3.57 - 3.49 (m, 4H), 3.46 - 3.35 (m, 4H), 3.20 (m, 2H), 2.48 (q, 6H), 1.64 (pent, 2H), 1.59 - 1.51 (m, 4H), 1.39 - 1.25 (m, 24H), 1.01 (t, 6H), 0.89 (t, 6H), 0.58 - 0.50 (2xm, 8H), -0.00 (s, 12H). NH is not visible.

¹³**C NMR** (acetone-d6, 125 MHz) δ [ppm]: 155.8 (C_{q}), 73.9 (2C), 71.5 (1C), 69.5 (2C), 51.0 (1C), 46.7 (2C), 39.8 (1C), 33.6 (2C), 31.8 (2C), 29.3 (2C), 29.2 (2C), 27.1 (1C), 24.0 (2C), 23.7 (2C), 22.5 (2C), 14.9 (2C), 13.5 (2C), 11.6 (2C), 10.9 (2C), -4.2 (4C).

**HRMS** (+ESI) *m*/*z* calcd for C₃₇H₈₀N₂O₄Si₂ [M+H]⁺: 673.5732; found: 673.5731.

### Example 13: 9,9,21,21-Tetramethyl-13,17-dioxa-9,21-disilanonacosan-15-yl (3-(dimethylamino)propyl)-carbamate

The synthesis followed general procedure 2 using *N,N-*dimethyl-1,3-propanediamine (102 mg, 1.17 mmol, 4.0 equiv.). The product was obtained as yellowish oil (28 mg, 43 µmol, 14%) after flash column chromatography using conditions B.

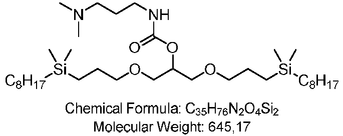

NMR spectra contain unknown impurties.

**¹H NMR** (CDCl₃, 500 MHz) δ [ppm]: 5.56 (brs, NH), 4.98 - 4.90 (m, 1H), 3.60 - 3.49 (m, 4H), 3.43 - 3.32 (m, 6H), 3.09 - 3.01 (m, 2H), 2.78 (s, 6H), 2.07 (pent, 2H), 1.55 - 1.47 (m, 4H), 1.32 - 1.16 (m, 24H), 0.85 (t, 6H), 0.50 - 0.39 (2xm, 8H), -0.06 (s, 12H).

¹³**C NMR** (CDCl₃, 125 MHz) δ [ppm]: 156.4 (C_{q}), 74.6 (2C), 72.4 (1C), 69.5 (2C), 55.4 (1C), 43.0 (2C), 37.7 (1C), 33.7 (2C), 31.9 (2C), 29.4 (2C), 29.3 (2C), 25.0 (1C), 24.0 (2C), 23.9 (2C), 22.7 (2C), 15.2 (2C), 14.1 (2C), 11.1 (2C), -3.5 (4C).

**HRMS** (+ESI) *m*/*z* calcd for C₃₅H₇₆N₂O₄Si₂ [M+H]⁺: 645.5419; found: 645.5416.

### Example 14: 9,9,21,21-Tetramethyl-13,17-dioxa-9,21-disilanonacosan-15-yl (2-(diethylamino)ethyl)-carbamate

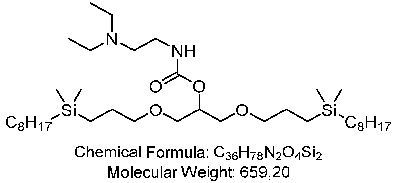

The synthesis followed general procedure 2 using *N,N-*diethylethylenediamine (136 mg, 1.17 mmol, 4.0 equiv.). The product was obtained as yellowish oil (50 mg, 76 µmol, 50%) after flash column chromatography using conditions B.

**¹H NMR** (CDCl₃, 500 MHz) δ [ppm]: 5.41 (brs, NH), 4.96 (pent, 1H), 3.60 - 3.52 (m, 4H), 3.43 - 3.32 (m, 4H), 3.28 - 3.19 (m, 2H), 2.56 (brs, 6H), 1.55 - 1.48 (m, 4H), 1.32 - 1.17 (m, 24H), 1.02 (t, 6H), 0.85 (t, 6H), 0.49 - 0.37 (2xm, 8H), -0.07 (s, 12H).

¹³**C NMR** (CDCl₃, 125 MHz) δ [ppm] : 156.4 (C_{q}), 74. 6 (2C), 72.0 (1C), 69.4 (2C), 51.9 (1C), 46.8 (2C), 38.3 (br, 1C), 33.7 (2C), 31.9 (2C), 29.4 (2C), 29.3 (2C), 24.0 (2C), 23.9 (2C), 22.7 (2C), 15.2 (2C), 14.1 (2C), 11.3 (br, 2C), 11.1 (2C), -3.5 (4C).

**HRMS** (+ESI) *m*/*z* calcd for C₃₆H₇₈N₂O₄Si₂ [M+H]⁺: 659.5575; found: 659.5571.

### Example 15: 9,9,21,21-Tetramethyl-13,17-dioxa-9,21-disilanonacosan-15-yl (4-(diethylamino)butyl)-carbamate

The synthesis followed general procedure 2 using *N*,*N*-diethyl-1,4-butanediamine (84 mg, 588 µmol, 4.0 equiv.). The product was obtained as yellowish oil (44 mg, 64 µmol, 44%) after flash column chromatography using conditions C.

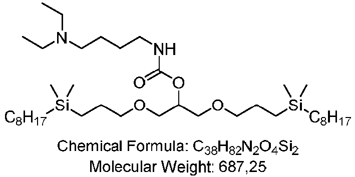

**¹H NMR** (CDCl₃, 500 MHz) δ [ppm]: 5.37 (t, NH), 4.95 (pent, 1H), 3.62 - 3.54 (m, 4H), 3.43 - 3.30 (m, 4H), 3.16 (q, 2H), 2.60 (q, 4H), 2.54 - 2.45 (m, 2H), 1.55 - 1.46 (m, 6H), 1.33 - 1.18 (m, 26H), 1.06 (t, 6H), 0.86 (t, 6H), 0.51 - 0.39 (2xm, 8H), -0.06 (s, 12H).

¹³**C NMR** (CDCl₃, 125 MHz) δ [ppm]: 156.0 (C_{q}), 74.5 (2C), 71.9 (1C), 69.4 (2C), 52.3 (1C), 46.6 (2C), 40.7 (1C), 33.7 (2C), 31.9 (2C), 29.3 (2C), 29.2 (2C), 27.9 (1C), 24.0 (2C), 23.8 (3C), 22.7 (2C), 15.1 (2C), 14.1 (2C), 11.0 (2C), 10.9 (2C), -3.5 (4C).

**HRMS** (+ESI) *m*/*z* calcd for C₃₈H₈₂N₂O₄Si₂ [M+H]⁺: 687.5888; found: 687.5898.

### Example 16: 9,9,21,21-Tetramethyl-13,17-dioxa-9,21-disilanonacosan-15-yl (3-(4-methylpiperazin-1-yl)propyl) carbamate

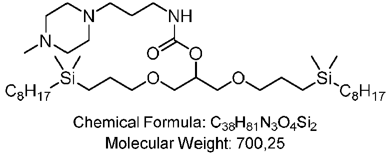

The synthesis followed general procedure 2 using 1-(3-aminopropyl)-4-methylpiperazine (184 mg, 1.17 mmol, 4.0 equiv.). The product was obtained as yellowish oil (30 mg, 43 µmol, 15%) after flash column chromatography using conditions B.

**¹H NMR** (CDCl₃, 500 MHz) δ [ppm]: 5.52 (t, NH), 4.92 (pent, 1H), 3.62 - 3.54 (m, 4H), 3.43 - 3.30 (m, 4H), 3.21 (q, 2H), 2.66 - 2.32 (brs, 8H), 2.39 (t, 2H), 2.28 (s, 3H), 1.64 (pent, 2H), 1.55 - 1.46 (m, 4H), 1.33 - 1.18 (m, 24H), 0.85 (t, 6H), 0.50 - 0.39 (2xm, 8H), -0.06 (s, 12H).

¹³**C NMR** (CDCl₃, 125 MHz) δ [ppm]: 156.0 (C_{q}), 74.5 (2C), 71.9 (1C), 69.5 (2C), 56.4 (1C), 54.9 (2C), 52.8 (2C), 45.8 (1C), 40.1 (1C), 33.7 (2C), 31.9 (2C), 29.3 (2C), 29.2 (2C), 26.3 (1C), 24.0 (2C), 23.8 (2C), 22.7 (2C), 15.1 (2C), 14.1 (2C), 11.0 (2C), -3.5 (4C).

**HRMS** (+ESI) *m*/*z* calcd for C₃₀H₈₁N₃O₄Si₂ [M+H]⁺: 700.5841; found: 700.5834.

### Example 17: 9,9,21,21-Tetramethyl-13,17-dioxa-9,21-disilanonacosan-15-yl (3-(pyrrolidin-1-yl)propyl)-carbamate

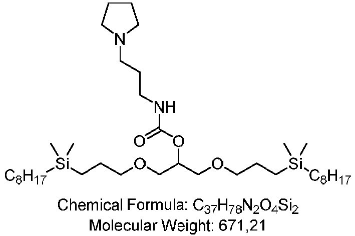

The synthesis followed general procedure 2 using 1-pyrrolidinepropanamine (188 mg, 1.47 mmol, 4.0 equiv.). The product was obtained as yellowish oil (12 mg, 18 µmol, 6%) after flash column chromatography using conditions D.

NMR spectra contain unknown impurities.

**¹H NMR** (CDCl₃, 500 MHz) δ [ppm]: 5.46(t, NH), 4.95 (pent, 1H), 3.59 - 3.51 (m, 4H), 3.44 - 3.19 (m, 4H), 3.27 (q, 2H), 2.72 (brs, 6H), 1.89 (brs, 4H), 1.82 (brs, 2H), 1.56 - 1.45 (m, 4H), 1.32 - 1.17 (m, 24H), 0.86 (t, 6H), 0.49 - 0.37 (2xm, 8H), -0.07 (s, 12H).

¹³**C NMR** (CDCl₃, 125 MHz) δ [ppm]: 156.2 (C_{q}), 74.6 (2C), 72.1 (1C), 69.5 (2C), 54.0 (2C), 53.7 (1C), 39.1 (1C), 33.7 (2C), 31.9 (2C), 29.4 (2C), 29.3 (2C), 27.6 (1C?), 24.0 (2C), 23.9 (2C), 23.4 (2C), 22.7 (2C), 15.2 (2C), 14.1 (2C), 11.1 (2C), -3.4 (4C).

**HRMS** (+ESI) *m*/*z* calcd for C₃₇H₇₈N₂O₄Si₂ [M+H]⁺: 671.5575; found: 671.5571.

### Example 18: 9,9,21,21-Tetramethyl-13,17-dioxa-9,21-disilanonacosan-15-yl (3-(piperidin-1-yl)propyl)-carbamate

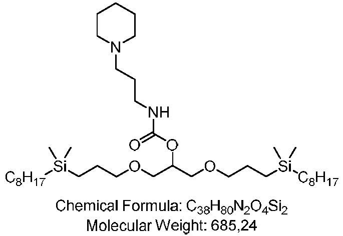

The synthesis followed general procedure 2 using 1-piperidinepropanamine (166 mg, 1.17 mmol, 4.0 equiv.). The product was obtained as yellowish oil (10 mg, 15 µmol, 6%) after flash column chromatography using conditions A. The obtained product contains residues of 4-DMAP.

**¹H NMR** (CDCl₃, 500 MHz) δ [ppm]: 5.80 (t, NH), 4.92 (pent, 1H), 3.60 - 3.47 (m, 4H), 3.42 - 3.28 (m, 4H), 3.19 (q, 2H), 2.31 (app. t, 6H), 1.61 (pent, 2H), 1.55 - 1.44 (2xm, 8H), 1.42 - 1.33 (m, 2H), 1.30 - 1.14 (m, 24H), 0.83 (t, 6H), 0.47 - 0.36 (2xm, 8H), -0.10 (s, 12H).

¹³**C NMR** (CDCl₃, 125 MHz) δ [ppm]: 156.0 (C_{q}), 74.5 (2C), 71.8 (1C), 69.5 (2C), 57.6 (1C), 54.6 (2C), 40.5 (1C), 33.7 (2C), 31.9 (2C), 29.7 (2C), 29.3 (2C), 26.2 (1C), 25.9 (2C), 24.4 (1C), 24.0 (2C), 23.8 (2C), 22.7 (2C), 15.0 (2C), 14.1 (2C), 11.1 (2C), -3.5 (4C).

**HRMS** (+ESI) *m*/*z* calcd for C₃₈H₈₀N₂O₄Si₂ [M+H]⁺: 685.5732; found: 685.5734.

### Example 19: 9,9,21,21-Tetramethyl-13,17-dioxa-9,21-disilanonacosan-15-yl (3-(dibutylamino)propyl)-carbamate

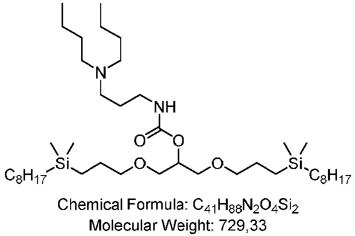

The synthesis followed general procedure 2 using 3-(dibutylamino)propylamine (110 mg, 588 µmol, 4.0 equiv.). The product was obtained as yellowish oil (8 mg, 11 µmol, 7%) after flash column chromatography using conditions C.

**¹H NMR** (CDCl₃, 500 MHz) δ [ppm]: 5.59 (brs, NH), 4.99 (pent, 1H), 3.62 - 3.51 (m, 4H), 3.41- 3.31 (m, 4H), 2.38 (q, 2H), 2.77 (bd, 2H), 2.70 (bs, 4H), 1.86 (brs, 2H), 1.57 (brs, 4H), 1.55 - 1.46 (m, 4H), 1.37 - 1.33 (m, 4H), 1.33 - 1.19 (m, 24H), 0.92 (t, 6H), 0.85 (t, 6H), 0.50 - 0.39 (2xm, 8H), -0.05 (s, 12H).

¹³**C NMR** (CDCl₃, 125 MHz) δ [ppm]: 156.1 (C_{q}), 74.6 (2C), 72.2 (1C), 69.5 (2C), 52.8 (2C), 51.3 (1C), 39.0 (1C), 33.7 (2C), 32.0 (2C), 29.4 (2C), 29.3 (2C), 26.6 (br, 2C), 25.3 (br, 1C), 24.0 (2C), 23.9 (2C), 22.7 (2C), 20.4 (2C), 15.2 (2C), 14.1 (2C), 13.8 (2C), 11.1 (2C), -3.4 (4C).

**HRMS** (+ESI) *m*/*z* calcd for C₄₁H₈₈N₂O₄Si₂ [M+H]⁺: 729.6358; found: 729.6363.

### Example 20: 9,9,21,21-Tetramethyl-13,17-dioxa-9,21-disilanonacosan-15-yl (2-(azepan-1-yl)ethyl)-carbamate

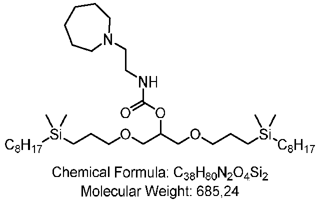

The synthesis followed general procedure 2 using 2-(azepan-1-yl)ethanamine (83 mg, 588 µmol, 4.0 equiv.). The product was obtained as yellowish oil (12 mg, 18 µmol, 12%) after flash column chromatography using conditions C.

**¹H NMR** (CDCl₃, 500 MHz) δ [ppm]: 5.59 (brs, NH), 4.99 (pent, 1H), 3.62 - 3.51 (m, 4H), 3.45 - 3.28 (m, 6H), 2.77 (bd, 6H), 1.72 (brs, 4H), 1.62 (brs, 4H), 1.55 - 1.46 (m, 4H), 1.33 - 1.19 (m, 24H), 0.88 (t, 6H), 0.50 - 0.39 (2xm, 8H), -0.05 (s, 12H).

¹³**C NMR** (CDCl₃, 125 MHz) δ [ppm]: 156.1 (C_{q}), 74.5 (2C), 72.2 (1C), 69.4 (2C), 56.6 (1C), 55.3 (2C), 38.0 (1C), 33.7 (2C), 31.9 (2C), 29.4 (2C), 29.3 (2C), 26.9 (2x 2C), 24.0 (2C), 23.8 (2C), 22.7 (2C), 15.1 (2C), 14.1 (2C), 11.0 (2C), -3.5 (4C).

**HRMS** (+ESI) *m*/*z* calcd for C₃₈H₈₀N₂O₄Si₂ [M+H]⁺: 685.5732; found: 685.5743.

### Example 21: 9,9,21,21-Tetramethyl-13,17-dioxa-9,21-disilanonacosan-15-yl (4-(dimethylamino)butyl)-carbamate

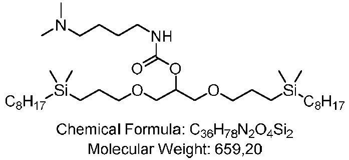

The synthesis followed general procedure 2 using *N,N*-dimethyl-1,5-pentanediamine (68 mg, 588 µmol, 4.0 equiv.). The product was obtained as yellowish oil (42 mg, 64 µmol, 44%) after flash column chromatography using conditions C.

**¹H NMR** (CDCl₃, 500 MHz) δ [ppm]: 5.17 (brt, NH), 4.94 (pent, 1H), 3.59 - 3.49 (m, 4H), 3.43 - 3.30 (m, 4H), 3.19 - 3.11 (m, 2H), 2.48 (t, 2H), 2.36 (s, 6H), 1.63 - 1.56 (m, 2H), 1.57 - 1.46 (m, 6H), 1.29 - 1.17 (m, 24H), 0.85 (t, 6H), 0.48 - 0.38 (2xm, 8H), -0.08 (s, 12H).

¹³**C NMR** (CDCl₃, 125 MHz) δ [ppm]: 156.1 (C_{q}), 74.5 (2C), 71.9 (1C), 69.4 (2C), 58.5 (1C), 44.5 (2C), 40.7 (1C), 33.7 (2C), 31.9 (2C), 29.3 (2C), 29.2 (2C), 27.5 (1C), 24.0 (2C), 23.8 (2C), 23.7 (1C), 22.6 (2C), 15.1 (2C), 14.1 (2C), 11.0 (2C), -3.5 (4C).

**HRMS** (+ESI) *m*/*z* calcd for C₃₆H₇₈N₂O₄Si₂ [M+H]⁺: 659.5575; found: 659.5586.

### 1.4 Esterification Process

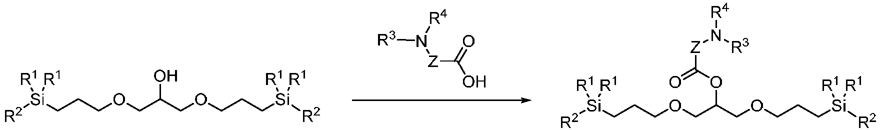

### General procedure 3

The substrate (0.58 mmol) was dissolved in DCM (6.0 mL) in a vial under argon atmosphere. Two mixtures were prepared. Mixture A contains the carboxylic acid (1.5 equiv.) and DIPEA (4.5 equiv. or 6.0 equiv. if HCl salt was used) in DCM. Mixture B contains EDC-HCl (1.5 equiv.) and 4-DMAP (0.2 equiv.) in DCM. After the addition of mixture A and B to the substrate, the reaction was stirred at rt for 2 - 8d (TLC control). If required, the additional carboxylic acid was added after 1 - 4d (0.3 equiv.) After completion, celite (1.0 g) was added and the volatiles were removed under reduced pressure. The product was purified by flash column chromatography on silica (4 g Ecoflex, 15 mL/min, CH/ EtOAc = 98:2 → 65:35, condition A or CH/EtOAc = 100:0 → 0:100, condition B).

### Example 22: 9,9,21,21-Tetramethyl-13,17-dioxa-9,21-disilanonacosan-15-yl 4-(dimethylamino)butanoate

The synthesis followed general procedure 3 using *4-(N,N-*dimethylamino)butanoic acid hydrochloride (146 mg, 0.87 mmol, 1.5 equiv.). The product was obtained as yellowish oil (200 mg, 317 µmol, 55%) after flash column chromatography using conditions A.

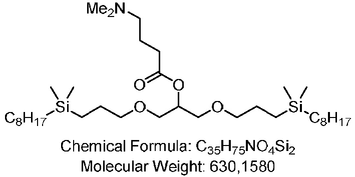

**¹H NMR** (CDCl₃, 500 MHz) δ [ppm]: 5.12 (pent, 1H), 3.59 - 4.51 (m, 4H), 3.44 - 3.32 (m, 4H), 2.36 (t, 2H), 2.30 (t, 2H), 2.21 (s, 6H), 1.88 (pent, 2H), 1.56 - 1.48 (m, 4H), 1.32 - 1.18 (m, 24H), 0.87 (t, 6H), 0.50 - 0.39 (2xm, 8H), -0.06 (s, 12H).

**¹³C NMR** (CDCl₃, 125 MHz) δ [ppm]: 172.9 (C_{q}), 74.6 (2C), 71.6 (1C), 69.3 (2C), 54.6 (1C), 44.1 (2C), 33.7 (2C), 32.1 (1C), 32.0 (2C), 29.4 (2C), 29.3 (2C), 26.9 (1C), 24.0 (2C), 23.9 (2C), 22.7 (2C), 15.2 (2C), 14.1 (2C), 11.1 (2C), -3.4 (4C).

**HRMS** (+ESI) *m*/*z* calcd for C₃₆H₇₇NO₅Si₂ [M+H]⁺: 660.5310; found: 687.5307.

### Example 23: 9,9,21,21-tetramethyl-13,17-dioxa-9,21-disilanonacosan-15-yl 3-(dimethylamino)propanoate

The synthesis followed general procedure 3 using *3-(N,N-*dimethylamino)propionic acid hydrochloride (134 mg, 87 µmol, 1.5 equiv.). The product was obtained as colorless oil (116 mg, 184 µmol, 32%) after flash column chromatography using conditions B.

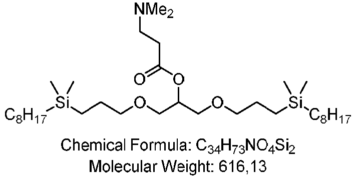

**¹H NMR** (CDCl₃, 500 MHz) δ [ppm]: 5.13 (pent, 1H), 3.59 - 4.51 (m, 4H), 3.44 - 3.32 (m, 4H), 2.78 - 2.66 (m, 2H), 2.64 - 2.53 (m, 2H), 2.3 (s, 6H), 1.56 - 1.48 (m, 4H), 1.32 - 1.18 (m, 24H), 0.87 (t, 6H), 0.50 - 0.39 (2xm, 8H), -0.06 (s, 12H).

**¹³C NMR** (CDCl₃, 125 MHz) δ [ppm]: 171.6 (C_{q}), 74.5 (2C), 71.9 (1C), 69.2 (2C), 54.4 (1C), 44.9 (2C), 33.7 (2C), 31.9 (2C), 29.4 (2C), 29.3 (2C), 26.9 (1C), 24.0 (2C), 23.8 (2C), 22.7 (2C), 15.2 (2C), 14. 1 (2C), 11.1 (2C), -3.4 (4C).

### Example 24: 9,9,21,21-Tetramethyl-13,17-dioxa-9,21-disilanonacosan-15-yl 3-(pyrrolidin-1-yl)propanoate

The synthesis followed general procedure 3 using 1-pyrrolidinepropanoic acid hydrochloride (156 mg, 0.87 mmol, 1.5 equiv.). The product was obtained as colorless oil (157 mg, 244 µmol, 42%) after flash column chromatography using conditions B.

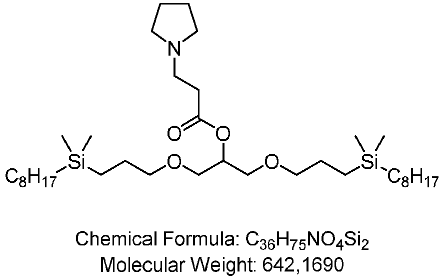

**¹H NMR** (CDCl₃, 500 MHz) δ [ppm]: 5.14 (pent, 1H), 3.59 - 4.51 (m, 4H), 3.44 - 3.32 (m, 4H), 2.92 (brs, 2H), 2.69 (brs, 6H), 1.85 (brs, 4H), 1.56 - 1.48 (m, 4H), 1.33 - 1.18 (m, 24H), 0.87 (t, 6H), 0.50 - 0.39 (2xm, 8H), -0.06 (s, 12H).

**¹³C NMR** (CDCl₃, 125 MHz) δ [ppm]: 171.8 (C_{q}), 74.5 (2C), 71.7 (1C), 69.1 (2C), 54.6 (2C), 51.1 (1C), 34.2 (1C), 33.7 (2C), 31.9 (2C), 29.4 (2C), 29.3 (2C), 24.0 (2C), 23.8 (2C), 23.4 (2C), 22.7 (2C), 15.2 (2C), 14.1 (2C), 11.1 (2C), -3.4 (4C).

**HRMS** (+ESI) *m*/*z* calcd for C₃₆H₇₅NO₄Si₂ [M+H]⁺: 642.5310; found: 642.5318.

### Example 25: 9,9,21,21-Tetramethyl-13,17-dioxa-9,21-disilanonacosan-15-yl 3-(piperidin-1-yl)propanoate

The synthesis followed general procedure 3 using 1-piperidinepropanoic acid (137 mg, 0.87 mmol, 1.5 equiv.). The crude product was obtained as yellowish oil (261 mg, 40 µmol, 69%) and was applied without further purification. 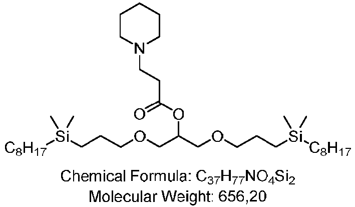 NMR spectra contain impurities.

**¹H NMR** (CDCl₃, 500 MHz) δ [ppm] : 5.11 (pent, 1H), 3.57 - 4.51 (m, 4H), 3.44 - 3.32 (m, 4H), 2.69 (brs, 2H), 2.56 (brs, 2H), 2.3 (brs, 4H), 1.56 - 1.46 (m, 8H), 1.41 (brs, 2H), 1.33 - 1.18 (m, 24H), 0.86 (t, 6H), 0.49- 0.39 (2xm, 8H), -0.06 (s, 12H).

**¹³C NMR** (CDCl₃, 125 MHz) δ [ppm]: 172.1 (C_{q}, weak) 74.5 (2C), 71.9 (1C), 69.3 (2C), 54.1 (3C), 33.7 (2C), 32.1 (br, 1C), 31.9 (2C), 29.4 (2C), 29.3 (2C), 25.7 (br, 2C), 24.1 (br, 1C), 24.0 (2C), 23.9 (2C), 22.7 (2C), 15.2 (2C), 14.1 (2C), 11.1 (2C), -3.4 (4C).

**HRMS** (+ESI) *m*/*z* calcd for C₃₇H₇₇NO₄Si₂ [M+H]⁺: 656.5466; found: 656.5472.

### Example 26: 9,9,21,21-Tetramethyl-13,17-dioxa-9,21-disilanonacosan-15-yl 3-(4-methylpiperazin-1-yl)-propanoate

The synthesis followed general procedure 3 using 4-methyl-1-piperazinepropanoic acid (150 mg, 0.87 mmol, 1.5 equiv.). The product was obtained as yellowish oil (145 mg, 22 µmol, 35%) after flash column chromatography using conditions B.

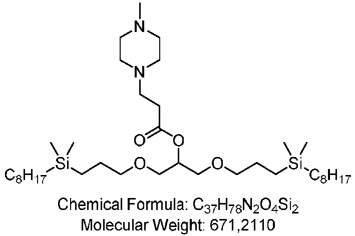

**¹H NMR** (CDCl₃, 500 MHz) δ [ppm]: 5.10 (pent, 1H), 3.59 - 3.51 (m, 4H), 3.42 - 3.32 (m, 4H), 2.72 (t, 2H), 2.68 - 2.46 (m, 8H), 2.52 (t, 2H), 2.36 (brs, 3H), 1.56 - 1.48 (m, 4H), 1.33 - 1.18 (m, 24H), 0.87 (t, 6H), 0.50 - 0.39 (2xm, 8H), -0.06 (s, 12H).

**¹³C NMR** (CDCl₃, 125 MHz) δ [ppm]: 171.9 (C_{q}), 74.5 (2C), 71.7 (1C), 69.2 (2C), 54.9 (2C), 53.4 (1C), 52.5 (2C), 45.7 (1C), 33.7 (2C), 32.4 (1C), 31.9 (2C), 29.3 (2C), 29.2 (2C), 24.0 (2C), 23.8 (2C), 22.7 (2C), 15.2 (2C), 14.1 (2C), 11.1 (2C), -3.4 (4C).

**HRMS** (+ESI) *m*/*z* calcd for C₃₇H₇₄N₂O₄Si₂ [M+H]⁺: 671.5575; found: 671.5581.

### Example 27: 9,9,21,21-Tetramethyl-13,17-dioxa-9,21-disilanonacosan-15-yl 4-(4-methylpiperazin-1-yl)-butanoate

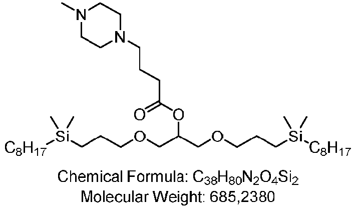

The synthesis followed general procedure 3 using 1-piperazinebutanoic acid, 4-methyl-, hydrochloride (194 mg, 871 µmol, 1.5 equiv.). The product was obtained as white solid (35 mg, 51 µmol, 13%) after flash column chromatography using conditions A.

NMR spectra contain unknown impurities.

**¹H NMR** (CDCl₃, 500 MHz) δ [ppm]: 5.08 (pent, 1H), 3.57 - 3.49 (m, 4H), 3.44 - 3.30 (m, 4H), 2.90 - 2.53 (brs, 8H), 2.50 - 2.41 (m, 2H), 2.47 (s, 3H), 2.36 (t, 2H), 1.83 (pent, 2H), 1.58 - 1.46 (m, 4H), 1.31 - 1.14 (m, 24H), 0.85 (t, 6H), 0.48 - 0.38 (2xm, 8H), -0.07 (s, 12H).

**¹³C NMR** (CDCl₃, 125 MHz) δ [ppm]: 172.8 (C_{q}), 74.6 (2C), 71.8 (1C), 69.3 (2C), 56.9 (1C), 54.1 (2C), 51.3 (2C), 44.9 (1C), 33.7 (2C), 32.0 (1C), 31.9 (2C), 29.4 (2C), 29.3 (2C), 24.0 (2C), 23.9 (2C), 22.7 (2C), 21.7 (1C), 15.2 (2C), 14.1 (2C), 11.1 (2C), -3.4 (4C).

**HRMS** (+ESI) *m*/*z* calcd for C₃₈H₈₀N₂O₄Si₂ [M+H]⁺: 685.5732; found: 685.5725.

### Example 28: 9,9,21,21-Tetramethyl-13,17-dioxa-9,21-disilanonacosan-15-yl 1-methylpiperidine-4-carboxylate

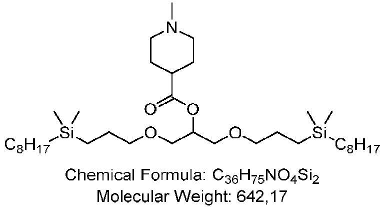

The synthesis followed general procedure 3 using 1-methylpiperidine-4-carboxylic acid hydrochloride (156 mg, 871 µmol, 1.5 equiv.). The product was obtained as yellowish oil (80 mg, 12 µmol, 32%) after flash column chromatography using conditions A.

**¹H NMR** (CDCl₃, 500 MHz) δ [ppm]: 5.13 (pent, 1H), 3.57 - 3.47 (m, 4H), 3.43 - 3.30 (m, 4H), 2.85 - 2.77 (m, 2H), 2.39 - 2.26 (m, 1H), 2.28 (s, 3H), 2.17 - 1.98 (m, 2H), 1.98 - 1.88 (m, 2H), 1.88 - 1.74 (m, 2H), 1.56 - 1.47 (m, 4H), 1.33 - 1.12 (m, 24H), 0.86 (t, 6H), 0.50 - 0.38 (2xm, 8H), -0.07 (s, 12H).

**¹³C NMR** (CDCl₃, 125 MHz) δ [ppm]: 174.3 (C_{q}), 74.5 (2C), 71.5 (1C), 69.3 (2C), 54.6 (2C), 46.1 (1C), 40.0 (1C), 33.7 (2C), 31.9 (2C), 29.3 (2C), 29.2 (2C), 27.9 (2C), 24.0 (2C), 23.9 (2C), 22.6 (2C), 15.1 (2C), 14.1 (2C), 11.1 (2C), -3.5 (4C).

**HRMS** (+ESI) m/z calcd for C₃₆H₇₅NO₄Si₂ [M+H]⁺: 642.5310; found: 642.5312.

### Example 29: 9,9,21,21-Tetramethyl-13,17-dioxa-9,21-disilanonacosan-15-yl 4-(pyrrolidin-1-yl)butanoate

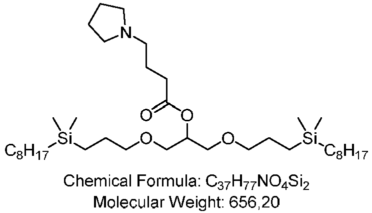

The synthesis followed general procedure 3 using 1-pyrrolidinebutanoic acid hydrochloride (58 mg, 300 µmol, 1.5 equiv.). The product was obtained as yellowish oil (16 mg, 24 µmol, 12%) after flash column chromatography using conditions B.

**¹H NMR** (CDCl₃, 500 MHz) δ [ppm]: 5.10 (pent, 1H), 3.57 - 3.49 (m, 4H), 3.43 - 3.32 (m, 4H), 2.76 - 2.49 (m, 6H), 2.40 (t, 2H), 1.96 - 1.87 (m, 2H), 1.83 (brs, 4H), 1.56 - 1.47 (m, 4H), 1.33 - 1.12 (m, 24H), 0.86 (t, 6H), 0.50 - 0.39 (2xm, 8H), -0.07 (s, 12H).

**¹³C NMR** (CDCl₃, 125 MHz) δ [ppm]: 171.8 (C_{q}), 74.5 (2C), 72.1 (1C), 69.2 (2C), 54.5 (1C), 53.6 (2C), 33.7 (2C), 31.9 (2C), 31.2 (1C), 29.3 (2C), 29.2 (2C), 24.0 (2C), 23.8 (2C), 23.4 (2C), 22.6 (2C), 21.3 (1C), 15.1 (2C), 14.1 (2C), 11.0 (2C), -3.5 (4C).

**HRMS** (+ESI) *m*/*z* calcd for C₃₇H₇₇NO₄Si₂ [M+H]⁺: 656.5466; found: 656.5468.

### Example 30: 9,9,21,21-Tetramethyl-13,17-dioxa-9,21-disilanonacosan-15-yl 4-(piperidin-1-yl)butanoate

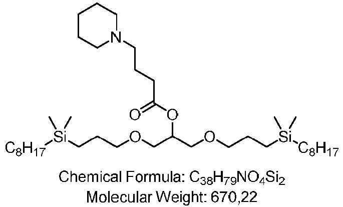

The synthesis followed general procedure 3 using 1-piperidinebutanoic acid hydrochloride (62 mg, 300 µmol, 1.5 equiv.). The product was obtained as yellowish oil (16 mg, 24 µmol, 12%) after flash column chromatography using conditions B.

**¹H NMR** (CDCl₃, 500 MHz) δ [ppm]: 5.10 (pent, 1H), 3.57 - 3.49 (m, 4H), 3.43 - 3.30 (m, 4H), 2.47 (brs, 6H), 2.39 (t, 2H), 1.90 (brs, 2H), 1.67 (brs, 4H), 1.56 - 1.47 (m, 4H), 1.45 (brs, 2H), 1.33 - 1.12 (m, 24H), 0.86 (t, 6H), 0.50 - 0.38 (2xm, 8H), -0.07 (s, 12H).

**¹³C NMR** (CDCl₃, 125 MHz) δ [ppm] : 172.7 (C_{q}), 74.5 (2C), 71.7 (1C), 69.4 (2C), 57.9 (1C), 54.1 (2C), 40.8 (1C), 33.7 (2C), 32.1 (1C), 31.9 (2C), 29.4 (2C), 29.3 (2C), 25.1 (2C), 24.0 (2C), 23.8 (2C), 22.7 (2C), 21.3 (1C), 16.1 (2C), 14.1 (2C), 11.1 (2C), -3.5 (4C).

**HRMS** (+ESI) *m*/*z* calcd for C₃₈H₇₉NO₄Si₂ [M+H]⁺: 670.5623; found: 670.5626.

### Example 31: 9,9,21,21-Tetramethyl-13,17-dioxa-9,21-disilanonacosan-15-yl 4-(diethylamino)butanoate

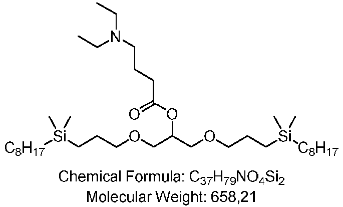

The synthesis followed general procedure 3 using *4-(N,N-*diethylamino)butanoic acid hydrochloride (59 mg, 300 µmol, 1.5 equiv.). The product was obtained as yellowish oil (93 mg, 138 µmol, 69%) after flash column chromatography using conditions A.

**¹H NMR** (CDCl₃, 500 MHz) δ [ppm]: 5.10 (pent, 1H), 3.57 - 3.49 (m, 4H), 3.43 - 3.30 (m, 4H), 2.54 brs, 4H), 2.47 (brs, 2H), 2.36 (t, 2H), 1.83 - 1.73 (m, 2H), 1.58 - 1.47 (m, 4H), 1.33 - 1.12 (m, 24H), 1.02 (t, 6H), 0.86 (t, 6H), 0.50 - 0.38 (2xm, 8H), -0.07 (s, 12H).

**¹³C NMR** (CDCl₃, 125 MHz) δ [ppm] : 172.7 (C_{q}), 74.5 (2C), 71.8 (1C), 69.5 (2C), 51.8 (1C), 46.9 (2C), 33.7 (2C), 32.2 (1C), 31.9 (2C), 29.4 (2C), 29.3 (2C), 24.0 (2C), 23.8 (2C), 22.7 (2C), 21.9 (1C), 15.2 (2C), 14.1 (2C), 11.5 (2C), 11.1 (2C), -3.5 (4C).

**HRMS** (+ESI) *m*/*z* calcd for C₃₇H₇₉NO₄Si₂ [M+H]⁺: 658.5623; found: 658.5626.

### Example 32: 9,9,21,21-Tetramethyl-13,17-dioxa-9,21-disilanonacosan-15-yl 5-(dimethylamino)pentanoate

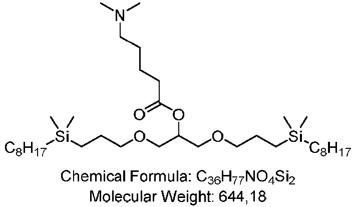

The synthesis followed general procedure 3 using *5-(N,N-*dimethylamino)pentanoic acid hydrochloride (200 mg, 1.10 mmol, 2.0 equiv.). The product was obtained as clear oil (92 mg, 14 µmol, 26%) after flash column chromatography using conditions A.

**¹H NMR** (CDCl₃, 500 MHz) δ [ppm]: 5.13 (pent, 1H), 3.57 - 3.47 (m, 4H), 3.43 - 3.30 (m, 4H), 2.34 (t, 2H), 2.28 (t, 2H), 2.22 (s, 6H), 1.63 (pent, 2H), 1.56 - 1.45 (m, 6H), 1.33 - 1.12 (m, 24H), 0.86 (t, 6H), 0.50 - 0.38 (2xm, 8H), -0.07 (s, 12H).

**¹³C NMR** (CDCl₃, 125 MHz) δ [ppm] : 173.0 (C_{q}), 74.5 (2C), 71.5 (1C), 69.2 (2C), 45.2 (1C), 34.2 (1C), 33.7 (2C), 31.9 (2C), 29.4 (2C), 29.3 (2C), 26.7 (1C), 24.0 (2C), 23.8 (2C), 22.8 (1C), 22.7 (1C), 15.1 (2C), 14.1 (2C), 11.1 (2C), -3.5 (4C).

**HRMS** (+ESI) *m*/*z* calcd for C₃₆H₇₇NO₄Si₂ [M+H]⁺: 644.5466; found: 644.5473.

### 1.5 Linker Systems

### General procedure 4

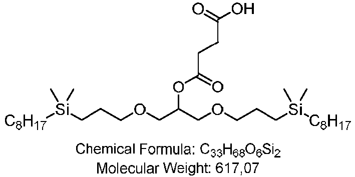

1,3-Bis(allyloxy)propan-2-ol (1.55 g, 3.0 mmol, 1.0 equiv.), succinic anhydride (600 mg, 6.0 mmol, 2.0 equiv.) and 4-DMAP (367 mg, 3.0 mmol, 1.0 equiv.) were dissolved in a mixture of THF, DCM and pyridine (2:2:1, 50 mL). The reaction was stirred overnight at room temperature. The reaction was diluted with ethyl acetate and extracted (2x 200 mL) with 1 M aqueous HCl solution (100 mL). The combined organic layers were washed with brine (100 mL) and dried over Na₂SO₄. After filtration and solvent removal, the crude product was purified by flash column chromatography on silica (25 g Ecoflex,32 mL/min, CH/EtOAc = 86:14 → 72:28) to afford the product as clear colorless oil (1054 mg, 1.71 mmol, 57%)

**¹H NMR** (CDCl₃, 500 MHz) δ [ppm]: 5.11 (pent, 1H), 3.58 - 3.50 (m, 4H), 3.42 - 3.31 (m, 4H), 2.67 - 2.56 (m, 4H), 1.55 - 1.46 (m, 4H), 1.33 - 1.15 (m, 24H), 0.85 (t, 6H), 0.50 - 0.39 (2xm, 8H), -0.07 (s, 12H). CO₂H is not visible.

**¹³C NMR** (CDCl₃, 125 MHz) δ [ppm]: 177.7 (C_{q}), 171.7 (C_{q}), 74.5 (2C), 72.1 (1C), 69.1 (2C), 33.7 (2C), 31.9 (2C), 29.4 (2C), 29.3 (2C), 29.0 (1C), 28.9 (1C), 24.0 (2C), 23.9 (2C), 22.7 (2C), 15.1 (2C), 14.1 (2C), 11.1 (2C), -3.4 (4C).

The acid (200 mg, 324 µmol, 1.0 equiv.), EDC-HCl (81 mg, 421 µmol, 1.3 equiv.), 4-DMAP (8.0 mg, 64.8 µmol, 0.2 equiv.), DIPEA (221 µL, 1.30 mmol, 4.0 equiv.) and the corresponding amino alcohol or amino amine (486 µmol, 1.5 equiv.) were placed under argon atmosphere into a vial. DCM (5 mL). The reaction was stirred at room temperature (2 - 5 d) until completion (TLC control). The reaction was diluted with dichloromethane (20 mL) and extracted (2x 25 mL) with an unsaturated aqueous NaHCO₃ solution. The combined organic phases were washed with brine (25 mL), dried over Na₂SO₄ and filtered. After solvent removal the crude product was purified by flash column chromatography on silica (4 g Ecoflex, 15 mL/min, CH/EtOAc = 98:2 → 65:35, condition A or CH/EtOAc = 100:0 → 0:100, condition B or DCM/MeOH = 99.5:0.5 - 92:8 condition C).

### Example 33: 3-(Dimethylamino)propyl (9,9,21,21-tetramethyl-13,17-dioxa-9,21-disilanonacosan-15-yl) succinate

The synthesis followed general procedure 4 using *N,N-*dimethylethanolamine (50 mg, 486 µmol, 1.5 equiv.). The product was obtained as yellowish oil (34 mg, 48 µmol, 15%) after flash column chromatography using conditions B.

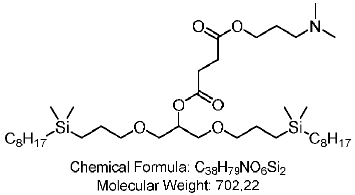

**¹H NMR** (CDCl₃, 500 MHz) δ [ppm]: 4.99 (pent, 1H), 4.12 (t, 2H), 3.58 - 3.50 (m, 4H), 3.42 - 3.31 (m, 4H), 2.67 - 2.56 (m, 4H), 2.41 (t, 2H), 2.27 (s, 6H), 1.83 (pent, 2H), 1.55 - 1.46 (m, 4H), 1.33 - 1.15 (m, 24H), 0.85 (t, 6H), 0.48 - 0.38 (2xm, 8H), -0.07 (s, 12H).

**¹³C NMR** (CDCl₃, 125 MHz) δ [ppm]: 172.2 (C_{q}), 171.1 (C_{q}), 74.6 (2C), 72.1 (1C), 69.1 (2C), 62.8 (1C), 56.1 (1C), 45.1 (2C), 33.7 (2C), 32.0 (2C), 29.4 (2C), 29.3 (2C), 29.1 (2C), 26.5 (1C), 24.0 (2C), 23.9 (2C), 22.7 (2C), 15.2 (2C), 14.1 (2C), 11.1 (2C), -3.4 (4C).

**HRMS** (+ESI) *m*/*z* calcd for C₃₈H₇₉NO₆Si₂ [M+H]⁺: 702.5521; found: 702.5518.

### 1.6 Different Silicon-Containing Side Chains

### General procedure 5

The 4-nitrophenyl carbonate ester (100 mg, 126 µmol) was dissolved in DCM (5.0 mL) under argon atmosphere. 4-DMAP (1.5 mg, 12.6 µmol, 0.1 equiv.), pyridine (60 mg, 755 µmol, 6.0 equiv.) and the corresponding amino alcohol (504 µmol, 4.0 equiv.) was added. The reaction was stirred at rt for 24 - 168 h (TLC control). After completion, H₂O (2 mL) and n-pentane (4 mL) were added, and the mixture was stirred at rt for 60 minutes. This process was repeated (2x) and the solvent was removed of combined organic layers.

### Example 34: 7,23-Dibutyl-9,9,21,21-tetramethyl-13,17-dioxa-9,21-disilanonacosan-15-yl 4-(dimethyl-amino)butanoate

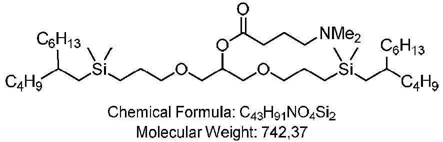

The synthesis followed general procedure 3 using *4-(N,N-*dimethylamino)butanoic acid hydrochloride (97 mg, 574 µmol, 1.5 equiv.). The product was obtained as colorless oil (172 mg, 231 µmol, 60%) after flash column chromatography using conditions A.

**¹H NMR** (CDCl₃, 500 MHz) δ [ppm]: 5.10 (pent, 1H), 3.57 - 4.51 (m, 4H), 3.42 - 3.31 (m, 4H), 2.38 - 2.32 (m, 2H), 2.37 (t, 2H), 2.26 (s, 6H), 1.82 (pent, 2H), 1.56 - 1.48 (m, 4H), 1.44 - 1.35 (m, 2H), 1.32 - 1.18 (m, 32H), 0.86 (2xt, 12H), 0.47 (d, 4H), 0.45 - 0.38 (m, 4H), -0.05 (s, 12H).

**¹³C NMR** (CDCl₃, 125 MHz) δ [ppm]: 172.8 (C_{q}), 74.6 (2C), 71.6 (1C), 69.3 (2C), 58.6 (1C), 45.1 (2C), 36.7 (2C), 36.4 (2C), 33.9 (2C), 31.9 (1C), 31.9 (2C), 29.7 (2C), 28.8 (2C), 26.5 (2C), 24.0 (2C), 23.1 (2C), 22.8 (2C), 22.5 (1C), 20.4 (2C), 14.2 (2C), 14.1 (2C), 11.9 (2C), -2.4 (4C).

**HRMS** (+ESI) *m*/*z* calcd for C₄₃H₉₁NO₄Si₂ [M+H]⁺: 742.6562; found: 742.6558.

### Example 35: 13,13,25,25-Tetramethyl-17,21-dioxa-13,25-disilaheptatriacontan-19-yl 4-(dimethylamino)-butanoate

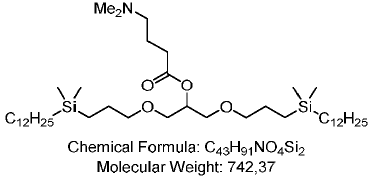

The synthesis followed general procedure 3 using *4-(N,N-*dimethylamino)butanoic acid hydrochloride (97 mg, 574 µmol, 1.5 equiv.). The product was obtained as yellowish oil (123 mg, 165 µmol, 41%) after flash column chromatography using conditions A.

NMR spectra show unknown impurities.

**¹H NMR** (CDCl₃, 500 MHz) δ [ppm]: 5.10 (pent, 1H), 3.58 - 4.48 (m, 4H), 3.43 - 3.30 (m, 4H), 2.39 - 2.32 (m, 2H), 2.37 (t, 2H), 2.26 (s, 6H), 1.82 (pent, 2H), 1.56 - 1.47 (m, 4H), 1.32 - 1.18 (m, 40H), 0.86 (t, 6H), 0.48 - 0.38 (2xm, 8H), -0.05 (s, 12H).

**¹³C NMR** (CDCl₃, 125 MHz) δ [ppm]: 172.9 (C_{q}), 74.6 (2C), 71.6 (1C), 69.3 (2C), 58.6 (1C), 45.1 (2C), 33.7 (2C), 32.1 (1C), 31.9 (2C), 29.7 (2C), 29.7 (2C), 29.7 (2C), 29.6 (2C), 29.4 (2C), 29.3 (2C), 24. 0 (2C), 23.9 (2C), 22.7 (2C), 22.6 (1C), 15.2 (2C), 14.1 (2C), 11.1 (2C), -3.4 (4C).

**HRMS** (+ESI) m/z calcd for C₄₃H₉₁NO₄Si₂ [M+H]⁺: 742.6562; found: 742.6557.

### Example 36: 13,13,25,25-Tetramethyl-17,21-dioxa-13,25-disilaheptatriacontan-19-yl (2-(diethylamino)-ethyl)carbamate

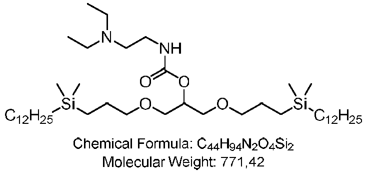

The synthesis followed general procedure 5 using *N,N-*diethylethylenediamine (58.1 mg, 0.504 mmol mg, 504 µmol, 4.0 equiv.). The product was obtained as slightly yellow oil (93 mg, 121 µmol, 96%) after extraction.

NMR spectra show unknown impurities.

**¹H NMR** (acetone-d6, 500 MHz) δ [ppm] : 5.94 (t, NH), 4. 93 (pent, 1H), 3.61 - 3.53 (m, 4H), 3.44 - 3.30 (m, 4H), 3.17 (t, 2H), 2.52 (q+t, 6H), 1.60 - 1.51 (m, 4H), 1.39 - 1.24 (m, 40H), 1.00 (t, 6H), 0.90 (t, 6H), 0.60 - 0.49 (2xm, 8H), 0.00 (s, 12H).

**¹³C NMR** (acetone-d6, 125 MHz) δ [ppm] : 155.9 (C_{q}), 73.9 (2C), 71. 6 (1C), 69.4 (2C), 52.3 (1C), 47.0 (2C), 38.9 (1C), 33.5 (2C), 31.7 (2C), 29.5 (multiple signals), 29.2 (2C), 24.0 (2C), 23.7 (2C), 22.5 (2C), 14.9 (2C), 13.6 (2C), 11.7 (2C), 10.9 (2C), -4.1 (4C).

**HRMS** (+ESI) *m*/*z* calcd for C₄₄H₉₄N₂O₂Si₂ [M+H]⁺: 771.6827; found: 771. 6842.

### Example 37: 7,23-Dibutyl-9,9,21,21-tetramethyl-13,17-dioxa-9,21-disilanonacosan-15-yl (2-(diethylamino)-ethyl)carbamate

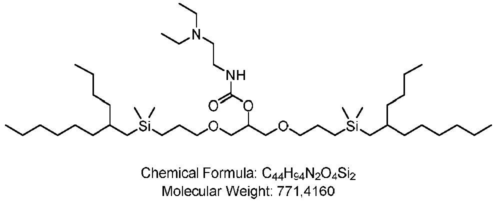

The synthesis followed general procedure 5 using *N,N-*diethylethylenediamine (58.1mg, 0.504 mmol mg, 504 µmol, 4.0 equiv.). The product was obtained as slightly yellow oil (91 mg, 120 µmol, 95%) extraction.

NMR spectra show unknown impurities.

**¹H NMR** (acetone-d6, 500 MHz) δ [ppm]: 5.90 (t, NH), 4.89 (pent, 1H), 3.61 - 3.53 (m, 4H), 3.44 - 3.30 (m, 4H), 3.13 (t, 2H), 2.49 (q+t, 6H), 1.58 - 1.46 (m, 6H), 1.38 - 1.19 (m, 32H), 0.97 (t, 6H), 0.85 (2xt, 12H), 0.55 (d, 4H), 0.53 - 0.49 (m, 4H), 0.00 (s, 12H).

**¹³C NMR** (acetone-d6, 125 MHz) δ [ppm]: 156.8 (C_{q}), 73.9 (2C), 71.6 (1C), 69.4 (2C), 52.3 (1C), 47.0 (2C), 38.9 (1C), 36.6 (2C), 36.3 (2C), 33.8 (2C), 31.8 (2C), 29.6 (2C), 28.8 (2C), 26.4 (2C), 24.0 (2C), 22.9 (2C), 22.5 (2C), 20.3 (2C), 13.6 (2C), 13.5 (2C), 11.8 (2C), 11.7 (2C), -2.8 (4C). Two signals (28.8 and 29.6 are hidden below the acetone signal and were identified by the interpretation of the HSQC NMR spectrum).

**HRMS** (+ESI) *m*/*z* calcd for C₄₄H₉₄N₂O₂Si₂ [M+H]⁺: 771.6827; found: 771. 6838.

### Example 38: 4-(4-Methylpiperazin-1-yl)butyl (13,13,25,25-tetramethyl-17,21-dioxa-13,25-disilaheptatria-contan-19-yl) carbonate

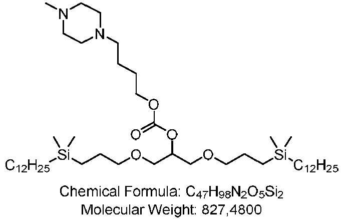

The synthesis followed general procedure 5 using 4-N-methyl-1-piperazinebutanol (86 mg, 0.504mmol86 mg, 504 µmol, 4.0 equiv.). The product was obtained as slightly yellow oil (97 mg, 122 µmol, 97%) after extraction.

NMR spectra show unknown impurities.

**¹H NMR** (CDCl₃, 500 MHz) δ [ppm]: 4.93 (pent, 1H), 4.11 (t, 2H), 3.62 - 3.53 (m, 4H), 3.44 - 3.30 (m, 4H), 2.72 - 2.27 (brm, 8H), 2.31 (t, 2H), 2.24 (s, 3H), 1.70 - 1.61 (m, 2H), 1.57 - 1.46 (m, 4H), 1.32 - 1.16 (m, 42H), 0.85 (t, 6H), 0.50 - 0.39 (2xm, 8H), -0.07 (s, 12H).

**¹³C NMR** (CDCl₃, 125 MHz) δ [ppm]: 154.9 (C_{q}), 75.5 (1C), 74.6 (2C), 69.1 (2C), 67.4 (1C), 57.9 (1C), 55.1 (2C), 53.1 (2C), 46.0 (1C), 33.7 (2C), 31.9 (2C), 29.7 (multiple C), 29.3 (multiple C), 26.6 (1C), 24.0 (2C), 23.9 (2C), 23.1 (1C), 22.7 (2C), 15.2 (2C), 14.1 (2C), 11.0 (2C), -3.5 (4C) .

**HRMS** (+ESI) *m*/*z* calcd for C₄₅H₉₄N₂O₅Si₂ [M+H]⁺: 827.7089; found: 827.7097.

### Example 39: 7,23-Dibutyl-9,9,21,21-tetramethyl-13,17-dioxa-9,21-disilanonacosan-15-yl (4-(4-methyl-piperazin-1-yl)butyl) carbonate

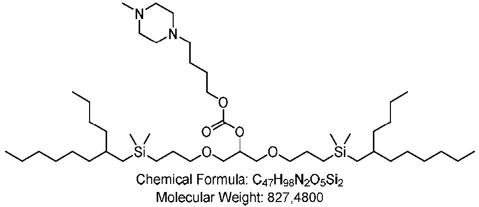

The synthesis followed general procedure 5 using 4-N-methyl-1-piperazinebutanol (86 mg, 0.504 mmol, 4.0 equiv.). The product was obtained as slightly yellow oil (93 mg, 117 µmol, 93%) after extraction.

**¹H NMR** (CDCl₃, 500 MHz) δ [ppm]: 4.93 (pent, 1H), 4.10 (t, 2H), 3.61 - 3.53 (m, 4H), 3.44 - 3.30 (m, 4H), 2.72 - 2.27 (brm, 8H), 2.31 (t, 2H), 2.25 (s, 3H), 1.70 - 1.62 (m, 2H), 1.58 - 1.46 (m, 4H), 1.44 - 1.35 (m, 2H), 1.30 - 1.12 (m, 34H), 0.85 (2xt, 12H), 0.47 (d, 4H), 0.44 - 0.39 (m, 4H), -0.05 (s, 12H).

**¹³C NMR** (CDCl₃, 125 MHz) δ [ppm]: 154.9 (C_{q}), 75.5 (1C), 74.6 (2C), 69.4 (2C), 67.9 (1C), 58.0 (1C), 55.1 (2C), 53.1 (2C), 46.0 (1C), 36.6 (2C), 36. 3 (2C), 33.9 (2C), 31.9 (2C), 29.7 (2C), 28.8 (2C), 26. 6 (1C), 26.5 (2C), 24.0 (2C), 23.2 (1C), 23.1 (2C), 22.7 (2C), 20. 4 (2C), 14.2 (2C), 14.1 (2C), 11.9 (2C), -2.8 (4C).

**HRMS** (+ESI) *m*/*z* calcd for C₄₇H₉₈N₂O₅Si₂ [M+H]⁺: 827.7089; found: 827.7093.

### Example 40: 3-(Dimethylamino)propyl (13,13,25,25-tetramethyl-17,21-dioxa-13,25-disilaheptatriacontan-19-yl) carbonate

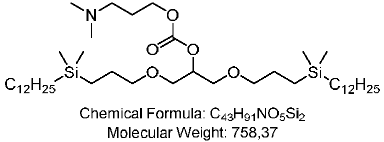

The synthesis followed general procedure 5 using 3-(*N,N-*dimethylamino)-1-propanol (67 mg, 0.504 mmol (67 mg, 504 µmol, 4.0 equiv.). The product was obtained as slightly yellow oil (67 mg, 88 µmol, 70%) after extraction.

**¹H NMR** (CDCl₃, 500 MHz) δ [ppm]: 4.93 (pent, 1H), 4.18 (t, 2H), 3.61 - 3.53 (m, 4H), 3.44 - 3.30 (m, 4H), 2.53 (brs, 2H), 2.34 (brs, 6H), 1.94 (brs, 2H), 1.58 - 1.46 (m, 4H), 1.30 - 1.12 (m, 40H), 0.86 (t, 6H), 0.50 - 0.37 (2xm, 8H), -0.07 (s, 12H).

**¹³C NMR** (CDCl₃, 125 MHz) δ [ppm] : 154.8 (C_{q}), 75.6 (1C), 74.6 (2C), 71.9 (1C), 69.1 (2C), 55.9 (1C), 44.7 (2C), 33.7 (4C), 31.9 (4C), 33.7 (2C), 31.9 (2C), 29.6 (multiple C), 29.4 (2C), 29.3 (2C), 24.0 (1C), 22.7 (2C), 15.2 (2C), 14.1 (2C), 11.1 (2C), -3.4 (4C).

**HRMS** (+ESI) *m*/*z* calcd for C₄₃H₉₁NO₅Si₂ [M+H]⁺: 758.6511; found: 758.6519.

### Example 41: 7,23-Dibutyl-9,9,21,21-tetramethyl-13,17-dioxa-9,21-disilanonacosan-15-yl (3-(dimethyl-amino)propyl) carbonate

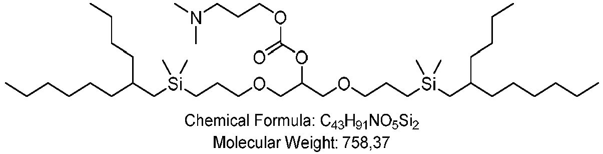

The synthesis followed general procedure 5 using *3-(N,N-*dimethylamino)-1-propanol (67 mg, 504 µmol, 4.0 equiv.). The product was obtained as slightly yellow oil (50 mg, 66 µmol, 52%) after extraction.

**¹H NMR** (CDCl₃, 500 MHz) δ [ppm] : 4.93 (pent, 1H), 4.16 (t, 2H), 3.61 - 3.53 (m, 4H), 3.44 - 3.30 (m, 4H), 2.37 (t, 2H), 2.22 (s, 6H), 1.83 (pent, 2H), 1.58 - 1.46 (m, 4H), 1.44 - 1.35 (m, 2H), 1.30 - 1.12 (m, 32H), 0.85 (2xt, 12H), 0.47 (d, 4H), 0.44 - 0.39 (m, 4H), -0.05 (s, 12H).

**¹³C NMR** (CDCl₃, 125 MHz) δ [ppm] : 154.8 (C_{q}), 75.6 (1C), 74.6 (2C), 69.1 (2C), 66.3 (1C), 55.9 (1C), 45.3 (2C), 36.7 (2C), 36.3 (2C), 34.0 (2C), 31.9 (2C), 29.7 (2C), 28.8 (2C), 26.7 (1C), 26.5 (2C), 24.0 (2C), 23.1 (2C), 22.7 (2C), 20.4 (2C), 14.1 (2C), 14.0 (2C), 11.9 (2C), -2.8 (4C).

**HRMS** (+ESI) *m*/*z* calcd for C₄₃H₉₁NO₅Si₂ [M+H]⁺: 758.6511; found: 758.6519.

### Example 42: 2-(2-(Dimethylamino)ethoxy)ethyl (13,13,25,25-tetramethyl-17,21-dioxa-13,25-disilaheptatria-contan-19-yl)

### carbonate

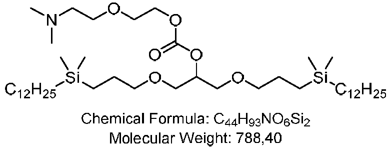

The synthesis followed general procedure 5 using 2-[2-*(N,N-*dimethylamino)ethoxy]ethanol (67 mg, 504 µmol, 4.0 equiv.). The product was obtained as slightly yellow oil (67 mg, 882 µmol, 70%) after extraction.

NMR spectra show unknown impurities.

**¹H NMR** (CDCl₃, 500 MHz) δ [ppm]: 4.93 (pent, 1H), 4.25 (t, 2H), 3.66 (t, 2H), 3.62 - 3.53 (m, 4H), 3.50 - 3.30 (m, 6H), 2.55 (t, 2H), 2.25 (s, 6H), 1.56 - 1.46 (m, 4H), 1.32 - 1.16 (m, 40H), 0.85 (t, 6H), 0.50 - 0.39 (2xm, 8H), -0.07 (s, 12H).

**¹³C NMR** (CDCl₃, 125 MHz) δ [ppm]: 154.9 (C_{q}), 75.8 (1C), 74.6 (2C), 69.1 (2C), 69.0 (1C), 68.7 (1C), 66.9 (1C), 58.6 (1C), 45.6 (2C), 33.7 (2C), 31.9 (2C), 29.7 (multiple C), 29.3 (multiple C), 24.0 (2C), 23.9 (2C), 22.7 (2C), 15.2 (2C), 14.1 (2C), 11.0 (2C), -3.5 (4C).

**HRMS** (+ESI) *m*/*z* calcd for C₄₄H₉₃NO₆Si₂ [M+H]⁺: 788.6616; found: 788.6624.

### Example 43: 7,23-Dibutyl-9,9,21,21-tetramethyl-13,17-dioxa-9,21-disilanonacosan-15-yl (2-(2-(dimethyl-amino)ethoxy)ethyl) carbonate

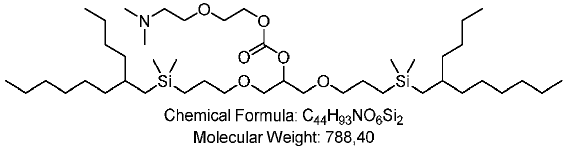

The synthesis followed general procedure 5 using 2-[2-(*N,N-*dimethylamino)ethoxy]ethanol (67 mg, 504 µmol, 4.0 equiv.). The product was obtained as slightly yellow oil (32 mg, 42 µmol, 33%) after extraction.

**¹H NMR** (CDCl₃, 500 MHz) δ [ppm]: 4.93 (pent, 1H), 4.25 (t, 2H), 3.66 (t, 2H), 3.62 - 3.53 (m, 6H), 3.43 - 3.30 (m, 4H), 2.51 (t, 2H), 2.25 (s, 6H), 1.56 - 1.46 (m, 4H), 1.45 - 1.36 (m, 2H), 1.32 - 1.16 (m, 32H), 0.85 (2xt, 12H), 0.47 (d, 4H), 0.44 - 0.38 (m, 4H), -0.04 (s, 12H) .

**¹³C NMR** (CDCl₃, 125 MHz) δ [ppm]: 154.9 (C_{q}), 75.8 (1C), 74.7 (2C), 69.3 (1C), 69.1 (2C), 68.7 (1C), 67.0 (1C), 58.6 (1C), 45.8 (2C), 36.7 (2C), 36.4 (2C), 34.0 (2C), 32.0 (2C), 29.8 (2C), 28.8 (2C), 26.7 (2C), 24.0 (2C), 23.1 (2C), 22.7 (2C), 20.5 (2C), 14.2 (2C), 14.1 (2C), 11.9 (2C), -2.8 (4C).

HRMS (+ESI) *m*/*z* calcd for C₄₄H₉₃NO₆Si₂ [M+H]⁺: 788.6616; found: 788.6625.

### Example 44: 19,19,31,31-Tetramethyl-23,27-dioxa-19,31-disilanonatetracontan-25-yl 4-(dimethylamino)-butanoate)

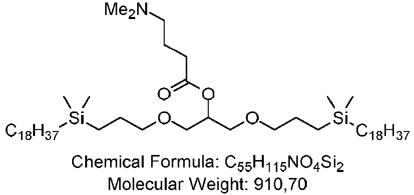

The synthesis followed general procedure 3 using 4-(*N,N-*dimethylamino)butanoic acid hydrochloride (94 mg, 564 µmol, 1.5 equiv.). The product was obtained as clear oil (81 mg, 90 µmol, 24%) after flash column chromatography using conditions A.

NMR spectra show unknown impurities.

**¹H NMR** (CDCl₃, 500 MHz) δ [ppm]: 5.10 (pent, 1H), 3.58 - 4.48 (m, 4H), 3.43 - 3.30 (m, 4H), 2.51 - 2.42 (m, 2H), 2.41 - 2.35 (m, 2H), 2.33 (s, 6H), 1.87 (pent, 2H), 1.56 - 1.47 (m, 4H), 1.32 - 1.18 (m, 64H), 0.86 (t, 6H), 0.48 - 0.38 (2xm, 8H), -0.05 (s, 12H).

**¹³C NMR** (CDCl₃, 125 MHz) δ [ppm]: 172.9 (C_{q}), 74.7 (2C), 71.7 (1C), 69.3 (2C), 58.4 (1C), 44.7 (2C), 33.7 (2C), 32.1 (1C), 31.9 (2C), 29.8 - 29.3 (multiple signals), 24.0 (2C), 23.9 (2C), 22.7 (2C), 22.1 (1C) 15.2 (2C), 14.1 (2C), 11.1 (2C), -3.4 (4C).

### Example 45: 9,9,13,13,25,25,29,29-Octamethyl-17,21-dioxa-9,13,25,29-tetrasilaheptatriacontan-19-yl 4-(dimethylamino)butanoate

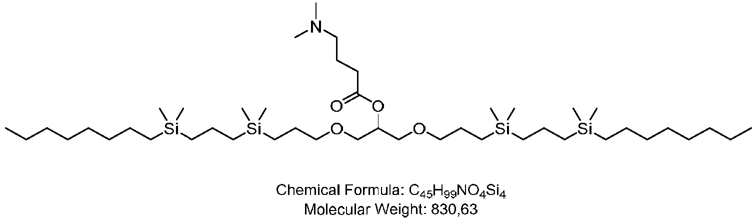

The synthesis followed general procedure 2 using *4-(N,N-*dimethylamino)butanoic acid hydrochloride (52 mg, 1.14 mmol, 1.1 equiv.). The product was obtained as clear oil (20 mg, 24 µmol, 8%) after flash column chromatography using conditions A.

NMR spectra show unknown impurities.

**¹H NMR** (CDCl₃, 500 MHz) δ [ppm] : 5.10 (pent, 1H), 3.58 - 4.48 (m, 4H), 3.42 - 3.31 (m, 4H), 2.36 (t, 2H), 2.33 (t, 2H), 2.23 (s, 6H), 1.80 (pent, 2H), 1.55 - 1.47 (m, 4H), 1.32 - 1.18 (m, 28H), 0.86 (t, 6H), 0.55 - 0.48 (2m, 8H), 0.46 - 0.38 (2xm, 8H), -0.07 (s, 12H), -0.09 (s, 12H).

**¹³C NMR** (CDCl₃, 125 MHz) δ [ppm] : 172.9 (C_{q}), 74.5 (2C), 71.5 (1C), 69.3 (2C), 58.6 (1C), 45.1 (2C), 34.0 (2C), 32.1 (1C), 31.9 (2C), 29.3 (2C), 29.2 (2C), 24.0 (2C), 23.9 (2C), 22.7 (3C), 20.0 (2C), 19.9 (2C), 18.3 (2C), 15.2 (2C), 14.1 (2C), 11.1 (2C), -3.3 (4C), -3.4 (4C).

**HRMS** (+ESI) m/z calcd for C₄₅H₉₉NO₄Si₄ [M+H]⁺: 830.6726; found: 830.6733.

### 2 1,2,6-Trihexanol Framework

### 2.1 Precursor Synthesis

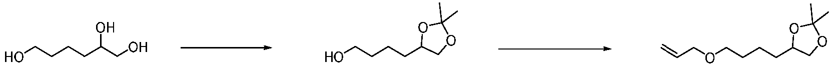

### 4-(4-Allyloxybutyl)-2,2-dimethyl-1,3-dioxolane

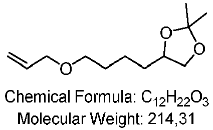

NaH (3.85 g, 160 mmol, 2.8 equiv.) was placed in a flask and dry THF (120 mL), mixed with the acetal (10.0 g, 57.39 mmol, 1 equiv.) and stirred for 3 h at rt. Allyl bromide (6.45 mL, 74.6 mmol, 1.3 equiv.) was then added to the solution and stirred overnight at rt (20 h). The solution was quenched with sat. NH₄Cl solution and diethyl ether (80 mL) was added. The organic phase was washed with saturated NH₄Cl solution (80 mL), water (80 mL) and brine (80 mL), dried over MgSO₄ and concentrated under reduced pressure. The crude product was purified by flash column chromatography (FP Ecoflex Si 120 g, 85 mL/min, cyclohexane / ethyl acetate = 0% → 40%) to afford the product as a colorless oil (10.7 g, 47.9 mmol, 84%).

**¹H NMR** (CDCl₃, 500 MHz) δ [ppm]: 5.89 (ddt, 1H), 5.25 (dd, 1H), 5.15 (dd, 1H), 4.10 - 4.02 (m, 1H), 4.02 - 4.00 (m, 1H), 3.94 (d, 2H), 3.49 (t, 1H), 3.42 (t, 2H), 1.68 - 1.57 (m, 4H), 1.55 - 1.44 (m, 2H), 1.39 (s, 3H), 1.33 (s, 3H).

**¹³C NMR** (CDCl₃, 125 MHz) δ [ppm]: 135.0 (1C), 116.8 (1C), 108.6 (1C), 76.0 (1C), 71.8 (1C), 70.1 (1C), 69.5 (1C), 33.4 (1C), 29.8 (1C), 26.9 (1C), 25.7 (1C), 22.5 (1C).

### 3-[4-(2,2-dimethyl-1,3-dioxolan-4-yl)butoxy]propyl-dimethyl-octylsilane

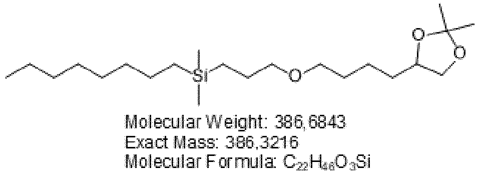

4-(4-Allyloxybutyl)-2,2-dimethyl-1,3-dioxolane (4.00 g, 18.67 mmol, 1.0 equiv.) was solved in dry toluene (20 mL) and 1/3 of the dimethyldodecylsilane (3.86 g, 22.40 mmol, 1.2 equiv. of a 90% pure substrate) and 1/3 of the Karstedt's catalyst (9 µL) were transferred into the flask. The flask was heated to reflux and another 1/3 of the substrate was added every 30 min (1 h in total for complete addition).

The solution was refluxed overnight and allowed to cool down. The solvent was removed under reduced pressure and the crude product was purified by flash column chromatography (FP Ecoflex Si 120 g, 85 ml/min, cyclohexane / acetone = 1%) to afford a colorless liquid (5.98 g, 15.5 mmol, 83%).

**¹H NMR** (CDCl₃, 500 MHz) δ [ppm]: 4.10 - 4.03 (m, 1H), 4.03 (dd, 1H), 3.49 (dd, 1H), 3.40 (t, 2H), 3.34 (t, 2H), 1.69 - 1.43 (m, 6H), 1.42 - 1.31 (m, 2H), 1.39 (s, 3H), 1.34 (s, 3H), 1.31 - 1.21 (m, 12H), 0.87 (t, 3H), 0.50 - 0.42 (m, 4H), -0.05 (s, 6H).

**¹³C NMR** (CDCl₃, 125 MHz) δ [ppm] : 108.6 (1C), 76.1 (1C), 74.1 (1C), 70.6 (1C), 69.5 (1C), 33.7 (1C), 33.4 (1C), 32.0 (1C), 29.8 (1C), 29.3 (1C), 29.3 (1C), 27.0 (1C), 25.9 (1C), 24.2 (1C), 23.9 (1C), 22.7 (1C), 22.5 (1C), 15.2 (1C), 14.1 (1C), 11.2 (1C), -3.4 (2C).

**²⁹Si NMR** (CDCl₃, 99 MHz) δ [ppm]: 2.78.

### 6-[3-[Dimethyl (octyl) silyl]propoxy]hexane-1,2-diol

The acetal derivative (3.25 g, 8.40 mmol, 1.0 equiv.) was dissolved in MeOH (15 mL), mixed with aq. HCl (15 mL, 32%) and stirred for 4 h at 50°C. The solution was then extracted with DCM (3 x 15 mL) and the combined organic phases were washed with sat. NaHCO₃ solution., dried over MgSO₄ and concentrated under reduced pressure to obtain the product as a pale white solid (1.88 g, 5.42 mmol, 65%).

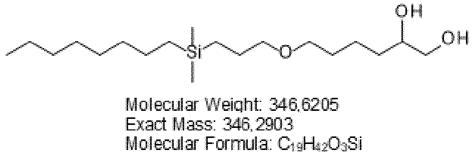

**¹H NMR** (CDCl₃, 500 MHz) δ [ppm]: 3.70 (m, 1H), 3.64 (ddd, 1H), 3.42 (ddd, 1H), 3.41 (t, 2H), 3.34 (m, 2H), 2.27 (d, 1H, OH), 2.00 (t, 1H, OH), 1.63 - 1.38 (m, 8H), 1.32 - 1.21 (m, 12H), 0.87 (t, 3H), 0.50 - 0.42 (m, 4H), -0.05 (s, 6H).

**¹³C NMR** (CDCl₃, 125 MHz) δ [ppm]: 74.1 (1C), 72.2 (1C), 70.6 (1C), 66.8 (1C), 33.7 (1C), 32.9 (1C), 32.0 (1C), 29.6 (1C), 29.4 (1C), 29.3 (1C), 24.1 (1C), 23.9 (1C), 22.7 (1C), 22.3 (1C), 15.2 (1C), 14.1 (1C), 11.2 (1C), -3.42 (2C).

**²⁹Si NMR** (CDCl₃, 99 MHz) δ [ppm] : 2.78.

### 2.2 Esterification Process

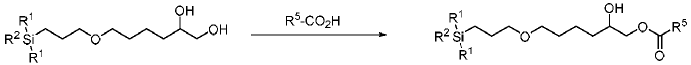

### General Procedure 6:

A solution of diol **6** (1.0 equiv.) in DCM (8 mL for 3.00 mmol starting material) was treated with a base (1.05 equiv.), EDC-HCl (1.05 equiv.) and 4-DMAP (0.1 equiv.). The carboxylic acid (1.0 equiv.) dissolved in DCM (2 mL) was added slowly into the reaction mixture via a syringe. The solution was stirred at rt overnight and concentrated afterwards. The crude product was purified by flash chromatography (FP Ecoflex 40 g Si, 45 mL/min, cyclohexane / ethyl acetate: 0% → 30%) to obtain the product. Regioisomers were obtained.

### [6-[3-[Dimethyl (octyl)silyl]propoxy]2-2-hydroxy-hexyl]nonanoate

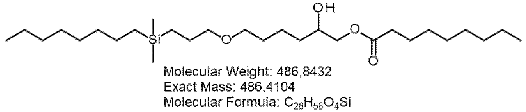

The synthesis followed general procedure 6 using diol (1.04 g, 3.00 mmol, 1.0 equiv.), NEt₃ (0.44 mL, 3.15 mmol, 1.05 equiv.), EDC-HCl (0.60 g, 3.15 mmol, 1.05 equiv.), 4-DMAP (4 mg, 0.30 mmol, 0.1 equiv.) and nonanoic acid (0.52 mL, 0.47 g, 3.00 mmol, 1.0 equiv.), to obtain the product as colorless oil after column chromatography (0.96 g, 1.98 mmol, 66%).

**¹H NMR** (CDCl₃, 500 MHz) δ [ppm]: 4.13 (dd, 1H), 3.95 (dd, 1H), 3.86 - 3.80 (m, 1H), 3.40 (t, 2H), 3.34 (t, 2H), 2.33 (t, 2H), 2.09 (d, 1H), 1.65 - 1.57 (m, 4H), 1.56 - 1.46 (m, 6H), 1.34 - 1.19 (m, 22H), 0.87 (t, 6H), 0.50 - 0.42 (m, 4H), -0.05 (s, 6H).

### [6-[3-[Dimethyl(octyl)silyl]propoxy]2-hydroxy-hexyl]octadecanoate

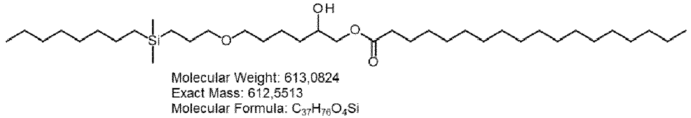

The synthesis followed general procedure 6 using diol (0.52 g, 1.49 mmol, 1.0 equiv.), stearic acid (0.42 g, 1.49 mmol, 1.0 equiv.), EDC-HCl (0.30 g, 1.56 mmol, 1.05 equiv.) and 4-DMAP (18 mg, 0.15 mmol, 0.1 equiv.) to obtain the product as a colorless oil after purification by column chromatography (0.73 g, 1.19 mmol, 80%). No additional base was used for this reaction.

**¹H NMR** (CDCl₃, 500 MHz) δ [ppm]: 4.14 (dd, 1H), 3.95 (dd, 1H), 3.85 - 3.78 (m, 1H), 3.41 (t, 2H), 3.34 (t, 2H), 2.33 (t, 2H), 1.84 (s, 1H), 1.66 - 1.57 (m, 4H), 1.56 - 1.47 (m, 6H), 1.46 - 1.40 (m, 2H), 1.33 - 1.20 (m, 38H), 0.87 (t, 6H), 0.50 - 0.42 (m, 4H), -0.05 (s, 6H).

### [6-[3-[Dimethyl(octyl)silyl]propoxy]-2hydroxy-hexyl] (9Z,12Z)-octadeca-9,12-dienoate

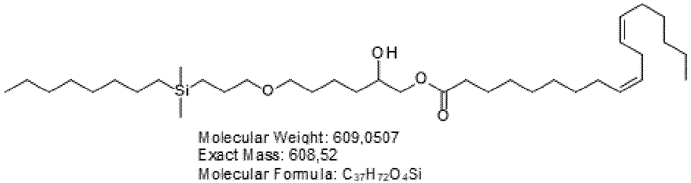

The synthesis followed general procedure 6 using diol (0.31 g, 0.89 mmol, 1.0 equiv.), linoleic acid (0.33 mL, 0.89 mmol, 1.0 equiv., 85%), EDC-HCl (0.18 g, 0.94 mmol, 1.05 equiv.) and 4-DMAP (11 mg, 0.09 mmol, 0.1 equiv.) to obtain the product as a colorless oil after column chromatography (0.26 g, 0.42 mmol, 48%).

**¹H NMR** (CDCl₃, 500 MHz) δ [ppm]: 5.40 - 5.28 (m, 4H), 4.13 (dd, 1H), 3.95 (dd, 1H), 3.86 - 3.79 (m, 1H), 3.40 (t, 2H), 3.34 (t, 2H), 2.76 (t, 2H), 2.33 (t, 2H), 2.04 (q, 4H), 1.66 - 1.56 (m, 4H), 1.55 - 1.39 (m, 6H), 1.38 - 1.21 (m, 26H), 0.89 - 0.85 (m, 6H), 0.50 - 0.42 (m, 4H), -0.05 (s, 6H). OH is not visible.

### [6-[3-[Dimethyl(octyl)silyl)]propoxy]-2-hydroxy-hexyl](2-hexyldecyl) hexanedioate

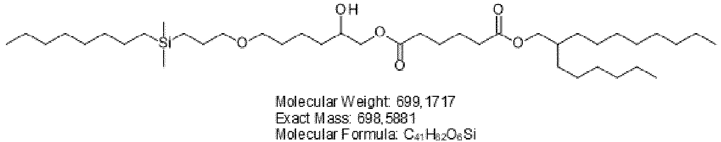

The synthesis followed general procedure 6 using diol (0.50 g, 1.44 mmol, 1.0 equiv.), 6-(2-hexyldecoxy)-6-oxo-hexanoicacid (0.54 g, 1.44 mmol, 1.0 equiv.), EDC-HCl (0.34 g, 1.73 mmol, 1.2 equiv.), 4-DMAP (90 mg, 0.72 mmol, 0.5 equiv.) and DIPEA (0.30 mL, 0.22 g, 1.73 mmol, 1.2 equiv.) to obtain the product as colorless oil after column chromatography (0.43 g, 0.61 mmol, 42%).

**¹H NMR** (CDCl₃, 500 MHz) δ [ppm]: 4.14 (dd, 1H), 3.96 (d, 2H), 3.95 (dd, 1H), 3.86 - 3.80 (m, 1H), 3.40 (t, 2H), 3.34 (t, 2H), 2.40 - 2.29 (m, 4H), 1.70 - 1.63 (m, 4H), 1.63 - 1.45 (m, 7H), 1.33 - 1.20 (m, 38H), 0.87 (t, 9H), 0.50 - 0.42 (m, 4H), -0.05 (s, 6H). OH is not visible.

### [6-[3-[dimethyl(octyl)silyl]propoxy]-2-hydroxy-hexyl] 4,5-bis(octylsulfanyl)pentanoate

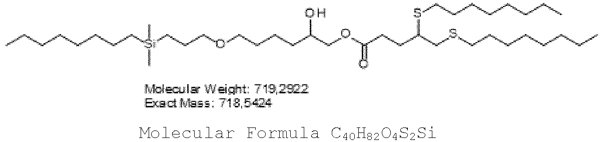

The synthesis followed general procedure 6 using diol (0.20 g, 0.58 mmol, 1.0 equiv.), 4,5-bis(octylsulfanyl)pentanoic acid (0.23 g, 0.58 mmol, 1.0 equiv.), EDC-HCl (0.13 g, 0.69 mmol, 1.2 equiv.), 4-DMAP (36 mg, 0.29 mmol, 0.5 equiv.) and DIPEA (0.16 mL, 0.12 g, 0.92 mmol, 1.6 equiv.) to obtain the product as colorless oil after column chromatography (0.29 g, 0.41 mmol, 71%).

**¹H NMR** (CDCl₃, 500 MHz) δ [ppm]: 4.18 - 4.11 (m, 1H), 3,98 - 3.91 (m, 1H), 3.87 - 3.81 (m, 1H), 3.40 (t, 2H), 3.34 (t, 2H), 2.86 (dd, 1H), 2.78 - 2.69 (m, 1H), 2.64 - 2.47 (m, 7H), 2.27 - 2.18 (m, 1H), 1,98 (bs, 1H), 1.78 - 1.68 (m, 1H), 1.64 - 1.47 (m, 10H), 1.45 - 1.18 (m, 34H), 0.87 (t, 9H), 0.50 - 0.41 (m, 4H), -0.06 (s, 6H).

### 2.3 Installation of the Head Group

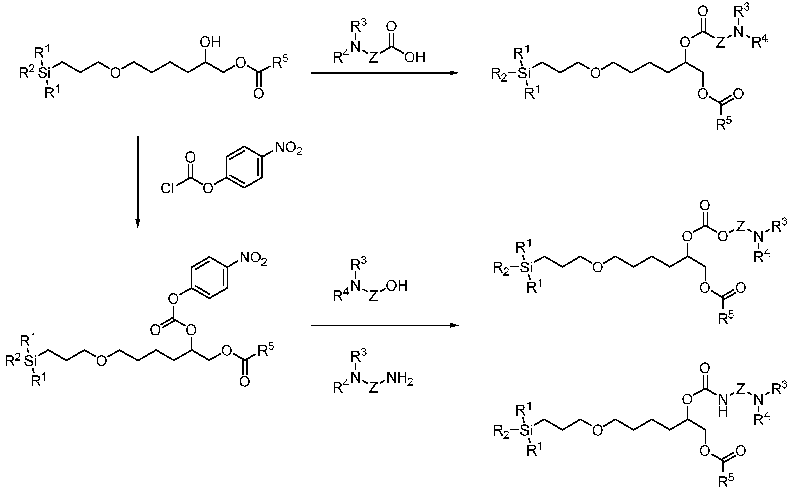

### General Procedure 7:

A solution of monoester (1.0 equiv.) in DCM (5 mL for 0.60 mmol diol) was treated with pyridine (1.5 equiv.), 4-DMAP (0.1 equiv.) and 4-nitrophenyl chloroformate (1.2 equiv.) under argon atmosphere and stirred at rt overnight (TLC control). The solvent was removed under reduced pressure and the residue was extracted with n-pentane (4 x 15 mL). Byproducts were precipitated with n-pentane and filtered off. The solvent was removed under reduced pressure and the crude product was used as such for the subsequent reaction (General Procedure 8).

### General Procedure 8:

A solution of the 4-nitrophenyl carbonate ester (1.0 equiv.) in DCM (3 mL for 0.12 mmol **13)** was treated with pyridine (6.0 equiv.), 4-DMAP (2.0 equiv.) and the corresponding amin oalcohol or aminoamine (4.0 equiv.) under argon atmosphere. The reaction was stirred at rt for 24 - 48 h (TLC control). After completion, the solvent was removed under reduced pressure and the residue was dissolved in n-pentane (10 mL), washed with H₂O (1 x 10 mL) and water containing one drop of MeOH (1 x 10 mL). n-Pentane was removed and the crude product was purified by flash chromatography (FP Ecoflex 4 g Si, 15 mL/min, DCM/MeOH: 0% → 10%) to obtain the product.

### Example 46: [2-[2-(Dimethylamino)ethoxycarbonyloxy]-6-[3-[diethyl(octyl)silyl]propoxy]hexyl] nonanoate

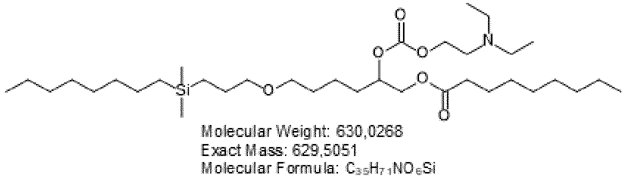

The synthesis followed general procedure 8 using 4-nitrophenyl carbonate ester (0.10 g, 0.15 mmol, 1.0 equiv.), 4-DMAP (40 mg, 0.30 mmol, 2.0 equiv.), pyridine (70 µL, 0.90 mmol, 6.0 equiv.) and 2-(*N,N*-diethylamino)ethanol (80 µL, 0.60 mmol, 4.0 equiv.) to obtain the product as a yellow oil after column chromatography (31 mg, 0.05 mmol, 33%).

NMR spectra contain a mixture of regioisomers. The NMR data of the major isomer are given.

**¹H NMR** (CDCl₃, 500 MHz) δ [ppm]: 4.92 - 4.86 (m, 1H), 4.26 (dd, 1H), 4.18 (t, 2H), 4.04 (dd, 1H), 3.38 (t, 2H), 3.33 (t, 2H), 2.72 (t, 2H), 2.56 (q, 4H), 2.29 (t, 2H), 1.71 - 1.49 (m, 8H), 1.49 - 1.35 (m, 2H), 1.33 - 1.19 (m, 22H), 1.02 (t, 6H), 0.87 (t, 6H), 0.50 - 0.41 (m, 4H), -0.05 (s, 6H).

**¹³C NMR** (CDCl₃, 125 MHz) δ [ppm]: 173.5 (C_{q}), 154.9 (C_{q}), 75.7 (1C), 74.1 (1C), 70.4 (1C), 66.2 (1C), 64.7 (1C), 51.1 (1C), 47.7 (2C), 34.1 (1C), 33.7 (1C), 32.0 (1C), 31.8 (1C), 30.6 (1C), 29.6 (1C), 29.4 (1C), 29.3 (1C), 29.2 (1C), 29.1 (2C), 24.8 (1C), 24.1 (1C), 23.9 (1C), 22.7 (1C), 22.6 (1C), 21.9 (1C), 15.2 (1C), 14.1 (1C), 14.1 (1C), 11.9 (2C), 11.2 (1C), -3.4 (2C).

**²⁹Si NMR** (CDCl₃, 99 MHz) δ [ppm]: 2.80.

**HRMS** (+ESI) *m*/*z* calcd for C₃₅H₇₁NO₆Si [M+H]⁺: 630.5130; found: 630.5161.

### Example 47: [2-[2-(Dimethylamino(ethoxycarbonyloxy]-6-[3-[diethyl(octyl)silyl]propoxy]hexyl] octadecanoate

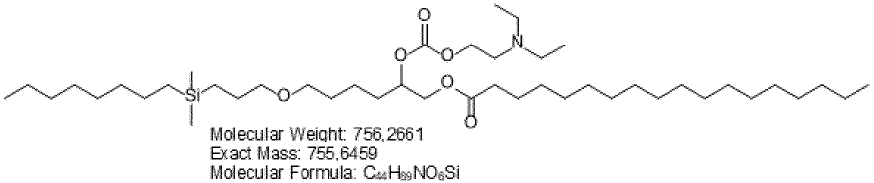

The synthesis followed general procedure 8 using 4-nitrophenyl carbonate ester (0.10 g, 0.13 mmol, 1.0 equiv.), 4-DMAP (31 mg, 0.26 mmol, 2.0 equiv.), pyridine (63 µL, 0.78 mmol, 4.0 equiv.) and 2-(N,N-diethylamino) ethanol (69 µL, 0.52 mmol, 4.0 equiv.) to obtain the product as a white solid after flash chromatography (75 mg, 0.10 mmol, 76%).

**¹H NMR** (CDCl₃, 500 MHz) δ [ppm] : 4.91 - 4.85 (m, 1H), 4.26 (dd, 1H), 4.22 (t, 2H), 4.04 (dd, 1H), 3.38 (t, 2H), 3.33 (t, 2H), 2.78 (t, 2H), 2.61 (q, 4H), 2.29 (t, 2H), 1.70 - 1.49 (m, 8H), 1.49 - 1.34 (m, 2H), 1.34 - 1.19 (m, 44H), 1.04 (t, 2H), 0.87 (t, 6H), 0.50 - 0.41 (m, 4H), -0.05 (s, 6H).

**¹³C NMR** (CDCl₃, 125 MHz) δ [ppm] : 173.5 (C_{q}), 154.8 (C_{q}), 75.8 (1C), 74.1 (1C), 70.4 (1C), 65.6 (1C), 64.6 (1C), 50.6 (1C), 47.3 (2C), 34.1 (1C), 33.7 (1C), 31.9 (1C), 31.9 (1C), 30.6 (1C), 29.7 (4C), 29.7 (2C), 29.6 (1C), 29.5 (1C), 29.5 (1C), 29.4 (1C), 29.3 (1C), 29.3 (1C), 29.3 (1C), 29.2 (1C), 29.1 (1C), 25.0 (1C), 24.8 (1C), 24.1 (1C), 23.8 (1C), 22.7 (2C), 21.8 (1C), 15.2 (1C), 14.1 (2C), 11.3 (1C), 11.2 (1C), -3.4 (2C).

**²⁹Si NMR** (CDCl₃, 99 MHz) δ [ppm]: 2.79.

**HRMS** (+ESI) *m*/*z* calcd for C₄₄H₈₉NO₆Si [M+H]⁺: 756.6538; found: 756.6542.

### General Procedure 9:

A solution of monoester (1.0 equiv.) in DCM (3 mL for 0.14 mmol 13) was treated with pyridine (2.0 equiv.) under argon. A solution of T3P^{®} (2.0 equiv., 50% in DMF) was added to the reaction and the reaction was stirred overnight at rt. Afterwards the organic phase was washed with a saturated aqeuous solution of NH₄Cl and brine, dried over anhydrous Na₂SO₄, filtered and concentrated under reduced pressure.

The crude product was purified by flash chromatography (FP Ecoflex 4 g Si, 15 mL/min, DCM/MeOH : 0% → 10%) to obtain the product.

### Example 48: [2-[4-(Dimethylamino)butanoyloxy]-6-[3-[dimethyl(octyl)silyl]propoxy]hexyl] nonanoate

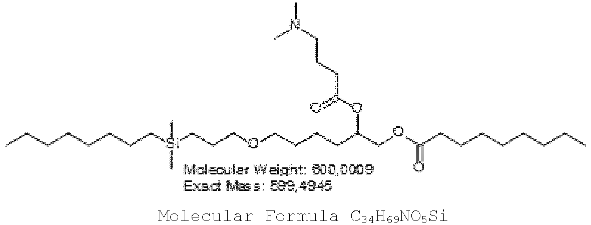

The synthesis followed general procedure 9 using **13 (Si)** (222 mg, 0.45 mmol, 1.0 equiv.), pyridine (91 µL, 1.13 mmol, 2.5 equiv.), 4-(N,N-dimethylamino)butanoic acid hydrochloride (113 mg, 0.68 mmol, 1.5 equiv.) and T3P^{®} solution (0.27 mL, 0.91 mmol, 2.0 equiv.) to obtain the product as a colorless oil after column chromatography (185 mg, 0.31 mmol, 69%).

**¹H NMR** (CDCl₃, 500 MHz) δ [ppm]: 5.04 - 4.97 (m, 1H), 4.30 (dd, 1H), 3.96 (dd, 1H), 3.45 (t, 2H), 3.40 - 3.35 (m, 2H), 3.32 (t, 2H), 2.94 - 2.85 (brm, 2H), 2.67 (brs, 6H), 2.42 (t, 2H), 2.28 (t, 2H), 2.07 (brquint., 2H), 1.65 - 1.48 (m, 8H), 1.43 - 1.18 (m, 22H), 0.85 (t, 6H), 0.49 - 0.39 (m, 4H), -0.07 (s, 6H).

**¹³C NMR** (CDCl₃, 125 MHz) δ [ppm]: 173.6 (1C), 172.0 (1C), 74.1 (1C), 72.3 (1C), 70.4 (1C), 69.3 (1C), 64.4 (1C), 57.3 (1C), 43.4 (2C), 34.1 (1C), 33.7 (1C), 31.9 (1C), 31.8 (2C), 31.1 (1C), 30.5 (1C), 29.3 (1C), 29.2 (1C), 29.1 (1C+1C), 24.9 (1C), 24.1 (1C), 23.8 (1C), 22.6 (2C), 22.0 (1C), 22.3 (1C), 14.1 (2C), 11.15 (2C), -3.4 (2C).

**HRMS** (+ESI) *m*/*z* calcd for C₃₄H₆₉NO₅Si [M+H]⁺: 600.5024; found: 600.5040.

### Example 49: [2-[4-(dimethylamino)butanoyloxy]-6-[3-[dimethyl(octyl)silyl]propoxy]hexyl] octadecanoate

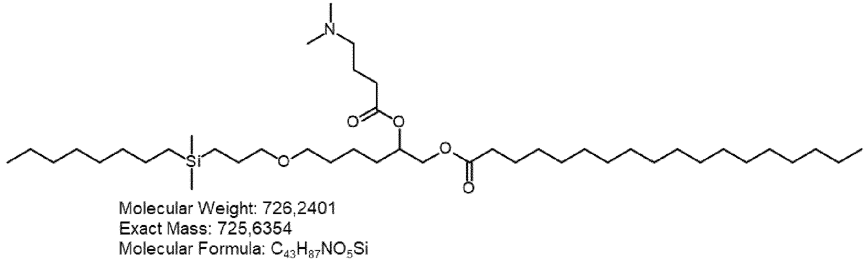

The synthesis followed general procedure 9 using **13 (Si)** (100 mg, 0.14 mmol, 1.0 equiv.), pyridine (23 µL, 0.28 mmol, 2.0 equiv.), 4-(N,N-dimethylamino)butanoic acid hydrochloride (35 mg, 0.21 mmol, 1.5 equiv.) and T3P^{®} solution (0.08 mL, 0.28 mmol, 2.0 equiv.) to obtain the product as a colorless oil after column chromatography (90 mg, 0.12 mmol, 90%).

**¹H NMR** (CDCl₃, 500 MHz) δ [ppm]: 5.11 - 5.03 (m, 1H), 4.23 (dd, 1H), 4.00 (dd, 1H), 3.37 (t, 2H), 3.32 (t, 2H), 2.41 - 2.30 (m, 2H), 2.34 (t, 2H), 2.27 (s, 6H), 1.82 (p, 2H), 1.64 - 1.49 (m, 8H), 1.45 - 1.17 (m, 44H), 0.86 (t, 6H), 0.50 - 0.41 (m, 4H), -0.06 (s, 6H).

**¹³C NMR** (CDCl₃, 125 MHz) δ [ppm]: 173.5 (Cq), 172.9 (Cq), 74.1 (1C), 71.5 (1C), 70.4 (1C), 64.8 (1C), 58.6 (1C), 45.2 (2C), 34.2 (1C), 33.7 (1C), 32.0 (3C), 30.6 (1C), 29.7 (7C), 29.6 (3C), 29.5 (1C), 29.4 (2C), 29.3 (2C), 29.2 (1C), 24.9 (1C), 24.2 (1C), 23.9 (1C), 22.7 (2C), 22.0 (1C), 15.2 (1C), 14.1 (2C), 11.2 (1C), -3.4 (2C).

**HRMS** (+ESI) m/z calcd for C₄₃H₈₇NO₅Si [M+H]⁺: 726.6433; found: 726.6460.

### Example 50: [2-[4-(Dimethylamino)butanoyloxy]-6-[3-[dimethyl(octyl)silyl]propoxy]hexyl] (9Z,12Z)-octa-deca-9,12-dienoate

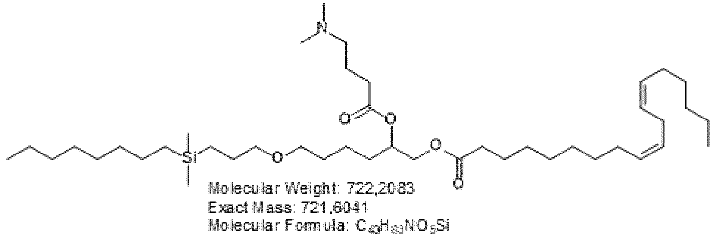

The synthesis followed general procedure 9 using **13 (Si)** (80 mg, 0.13 mmol, 1.0 equiv.), pyridine (21 µL, 0.26 mmol, 2.0 equiv.), 4-(N,N-dimethylamino)butanoic acid hydrochloride (33 mg, 0.20 mmol, 1.5 equiv.) and T3P^{®} solution (76 µL, 0.26 mmol, 2.0 equiv.) to obtain the product as a colorless liquid after column chromatography (80 mg, 0.11 mmol, 85%).

**¹H NMR** (CDCl₃, 500 MHz) δ [ppm]: 5.38 - 5.25 (m, 4H), 5.02 - 4.96 (m, 1H), 4.30 (dd, 1H), 3.95 (dd, 1H), 3.36 (t, 2H), 3.30 (t, 2H), 3.02 - 2.96 (m, 2H), 2.74 (s, 6H), 2.73 (t, 2H), 2.45 - 2.40 (m, 2H), 2.27 (t, 2H), 2.15 - 2.07 (m, 2H), 2.01 (q, 4H), 1.63 - 1.47 (m, 8H), 1.39 - 1.19 (m, 28H), 0.87 - 0.82 (m, 6H), 0.48 - 0.39 (m, 4H), -0.08 (s, 6H) .

**¹³C NMR** (CDCl₃, 125 MHz) δ [ppm]: 173.5 (C_{q}), 171.6 (C_{q}), 130.2 (1C), 130.0 (1C), 128.1 (1C), 127.9 (1C), 74.1 (1C), 72.6 (1C), 70.3 (1C), 64.3 (1C), 56.9 (1C), 42.8 (1C+1C), 34.1 (1C), 33.7 (1C), 31.9 (1C), 31.5 (1C), 30.8 (1C), 30.5 (1C), 29.6 (1C), 29.5 (1C), 29.3 (2C), 29.3 (1C), 29.2 (1C), 29.1 (1C+1C), 27.2 (1C+1C), 25.6 (1C), 24.9 (1C), 24.1 (1C), 23.8 (1C), 22.7 (1C+1C), 22.1 (1C), 19.5 (1C), 15.2 (1C), 14.1 (1C), 14.0 (1C), 11.2 (1C), -3.4 (2C).

**²⁹Si NMR** (CDCl₃, 99 MHz) δ [ppm]: 2.77

**HRMS** (+ESI) *m*/*z* calcd for C₄₃H₈₃NO₅Si [M+H]⁺: 722.6120; found: 722.6086.

### Example 51: [2-[4-(Dimethylamino)butanoyloxy]-6-[3-[dimethyl(octyl)silyl]propoxy]hexyl] (2-hexyldecyl) hexanedioate

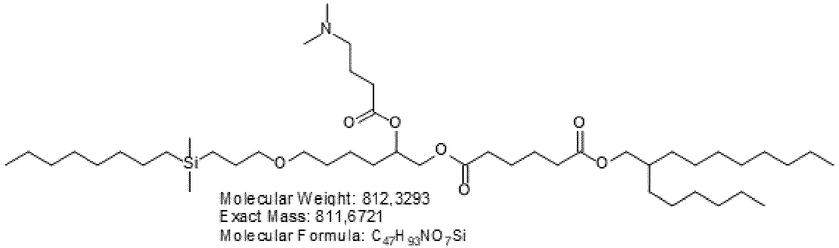

The synthesis followed general procedure 9 using (100 mg, 0.14 mmol, 1.0 equiv.), pyridine (23 µL, 0.28 mmol, 2.0 equiv.), *4-(N,N-*dimethylamino) butanoic acid hydrochloride (35 mg, 0.21 mmol, 1.5 equiv.) and T3P^{®} solution (82 µL, 0.28 mmol, 2.0 equiv.) to obtain the product as a colorless oil after column chromatography (91 mg, 0.11 mmol, 79%).

**¹H NMR** (CDCl₃, 500 MHz) δ [ppm] : 5.09 - 5.02 (m, 1H), 4.24 (dd, 1H), 3.99 (dd, 1H), 3.95 (d, 2H), 3.37 (2H), 3.32 (t, 2H), 2.50 - 2.40 (brm, 1H), 2.38 - 2.27 (brm, 11H), 1.85 (quint., 2H), 1.67 - 1.49 (m, 11H), 1.44 - 1.32 (m, 2H), 1.32 - 1.18 (m, 38H), 0.86 (t, 9H), 0.49 - 0.41 (m, 4H), -0.06 (s, 6H).

**¹³C NMR** (CDCl₃, 125 MHz) δ [ppm] : 173.5 (C_{q}), 173.0 (C_{q}), 172.8 (C_{q}), 74.1 (1C), 71.6 (1C), 70.4 (1C), 67.2 (1C), 64.9 (1C), 58.4 (1C), 44.9 (2C), 37.3 (1C), 33.9 (1C), 33.7 (1C+1C), 32.0 (1C), 31.9 (1C+1C), 31.8 (1C), 31.1 (2C), 30.6 (1C), 30.0 (1C), 29.6 (1C+2C), 29.3 (1C+1C+1C), 26.7 (1C+1C), 24.4 (1C+1C), 24.3 (1C), 24.1 (1C), 23.9 (1C), 22.7 (2C+1C), 22.0 (1C), 15.2 (1C), 14.1 (2C+1C), 11.2 (1C), - 3.4 (2C).

**²⁹Si NMR** (CDCl₃, 99 MHz) δ [ppm] : 2.77.

**HRMS** (+ESI) *m*/*z* calcd for C₄₇H₉₃NO₇Si [M+H]⁺: 812.6800; found: 812.6825.

### Example 52: [2-[4-(dimethylamino)butanoyloxy]-6-[3-[dimethyl(octyl)silyl]propoxy]hexyl] 4,5-bis(octyl sulfanyl)pentanoate

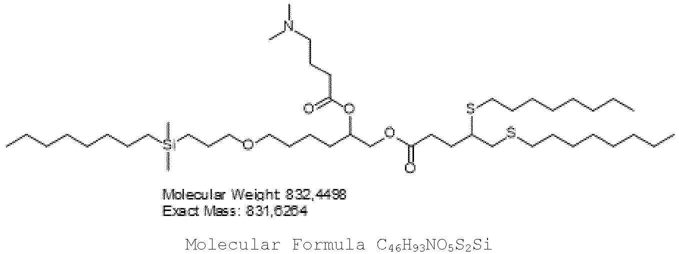

The synthesis followed general procedure 9 using **13 (Si)** (100 mg, 0.14 mmol, 1.0 equiv.), pyridine (23 µL, 0.28 mmol, 2.0 equiv.), 4-(*N,N*-dimethylamino) butanoic acid hydrochloride (35 mg, 0.21 mmol, 1.5 equiv.) and T3P^{®} solution (82 µL, 0.28 mmol, 2.0 equiv.) to obtain the product as a colorless oil after column chromatography (72 mg, 0.09 mmol, 64%).

**¹H NMR** (CDCl₃, 500 MHz) δ [ppm]: 5.10 - 5.03 (m, 1H), 4.26 - 4.20 (m, 1H), 4.04 - 3.98 (m, 1H), 3.37 (t, 2H), 3.32 (t, 2H), 2.84 (dd, 1H), 2.76 - 2.69 (m, 1H), 2.64 - 2.55 (m, 1H), 2.57 - 2.43 (m, 6H), 2.42 - 2.32 (m, 2H), 2.35 (t, 2H), 2.28 (s, 6H), 2.23 - 2.13 (m, 1H), 1.87 - 1.78 (m, 2H), 1.77 - 1.67 (m. 1H), 1.65 - 1.49 (m, 10H), 1.45 - 1.17 (m, 34H), 0.86 (t, 9H), 0.50 - 0.41 (m, 4H), -0.06 (s, 6H).

**¹³C NMR** (CDCl₃, 125 MHz) δ [ppm]: 173.0 (Cq), 172.9 (Cq), 70.1 (1C), 71.4 (1C), 70.4 (1C), 65.0 (1C), 58.6 (1C), 45.1 (3C), 38.3 (1C), 33.7 (1C), 33.0 (1C), 32.0 (2C), 31.8 (2C), 31.3 (1C), 30.8 (1C), 30.7 (1C), 29.9 (1C), 29.8 (1C), 29.6 (1C), 29.3 (2C), 29.2 (5C), 29.0 (1C), 28.9 (1C), 28.3 (1C), 24.1 (1C), 23.9 (1C), 22.7 (3C), 22.0 (1C), 15.2 (1C), 14.1 (3C), 11.2 (1C), -3.41 (2C).

**HRMS** (+ESI) *m*/*z* calcd for C₄₆H₉₃NO₅S₂Si [M+H]⁺: 832.6343; found: 832.6363.

All synthesized compounds are summarized in the following table.

**Table 1: Synthesized compounds**

| | |
|---|---|
| 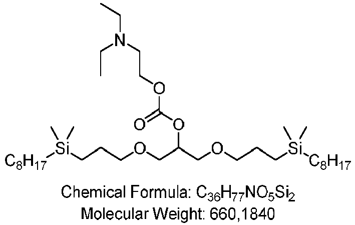 | **Example 1** |
| | 2-(Diethylamino)ethyl (9,9,21,21-tetramethyl-13,17-dioxa-9,21-disilanonacosan-15-yl) carbonate |
| | Formula (IV) |
| 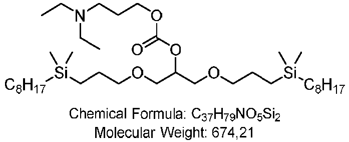 | **Example 2** |
| | 3-(Diethylamino)propyl(9,9,21,21-tetramethyl-13,17-dioxa-9,21-disilanonacosan-15-yl)carbonate |
| | Formula (V) |
| 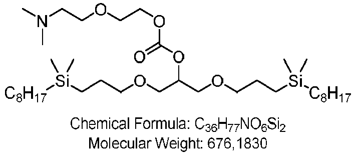 | **Example 3** |
| | 2-(2-(Dimethylamino)ethoxy)ethyl (9,9,21,21-tetramethyl-13,17-dioxa-9,21-disilanonacosan-15-yl)carbonate |
| | Formula (VI) |
| 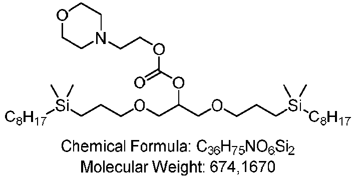 | **Example** 4 |
| | 2-Morpholinoethyl (9,9,21,21-tetramethyl-13,17-dioxa-9,21-disilanonacosan-15-yl)carbonate |
| | Formula (VII) |
| 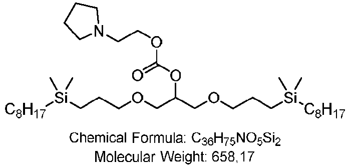 | **Example 5** |
| | 2-(Pyrrolidin-1-yl)ethyl (9,9,21,21-tetramethyl-13,17-dioxa-9,21-disilanonacosan-15-vl) carbonate |
| | Formula (VIII) |
| 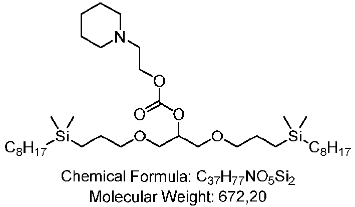 | **Example 6** |
| | 2-(Piperidin-1-yl)ethyl (9,9,21,21-tetramethyl-13,17-dioxa-9,21-disilanonacosan-15-yl)carbonate |
| | Formula (IX) |
| 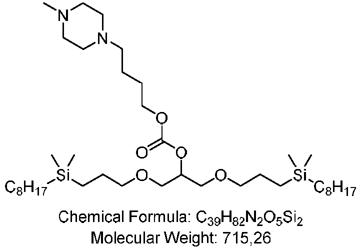 | **Example 7** |
| | 2-(4-Methylpiperazin-1-yl)butyl (9,9,21,21-tetramethyl-13,17-dioxa-9,21-disilanonacosan-15-yl)carbonate |
| | Formula (X) |
| 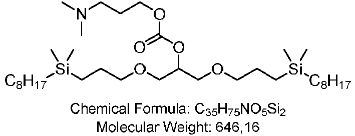 | **Example 8** |
| | 3-(Dimethylamino)propyl (9,9,21,21-tetramethyl-13,17-dioxa-9,21-disilanonacosan-15-yl)carbonate |
| | Formula (XI) |
| 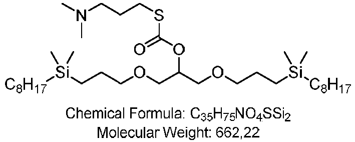 | **Example 9** |
| | S-(3-(Dimethylamino)propyl) O-(9,9,21,21-tetramethyl-13,17-dioxa-9,21-disilanonacosan-15-yl) carbonothioate |
| | Formula (XII) |
| 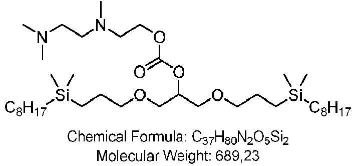 | **Example 10** |
| | 2-((2-(Dimethylamino)ethyl)(methyl)amino)eth yl(9,9,21,21-tetramethyl-13,17-dioxa-9,21-disilanonacosan-15-yl)carbonate |
| | Formula (XIII) |
| 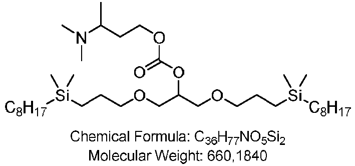 | **Example 11** |
| | 3-(Dimethylamino)butyl(9,9,21,21-tetramethyl-13,17-dioxa-9,21-disilanonacosan-15-yl)carbonate |
| | Formula (XIV) |
| 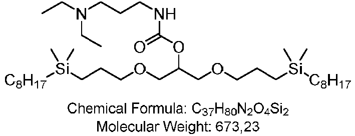 | **Example 12** |
| | 9,9,21,21-Tetramethyl-13,17-dioxa-9,21-disilanonacosan-15-yl(3-(diethylamino)propyl)-carbamate |
| | Formula (XV) |
| 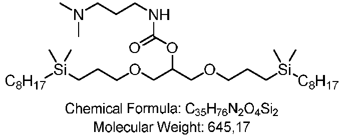 | **Example 13** |
| | 9,9,21,21-Tetramethyl-13,17-dioxa-9,21-disilanonacosan-15-yl (3-(dimethylamino)propyl)-carbamate |
| | Formula (XVI) |
| 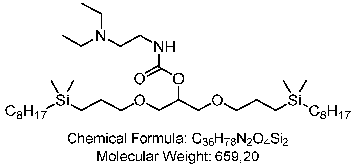 | **Example 14** |
| | 9,9,21,21-Tetramethyl-13,17-dioxa-9,21-disilanonacosan-15-yl (2-(diethylamino)ethyl)-carbamate |
| | Formula (XVII) |
| 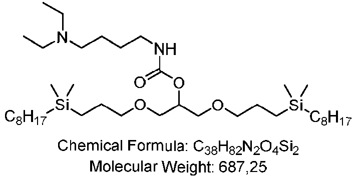 | **Example 15** |
| | 9,9,21,21-Tetramethyl-13,17-dioxa-9,21-disilanonacosan-15-yl (4-(diethylamino)butyl)-carbamate |
| | Formula (XVIII) |
| 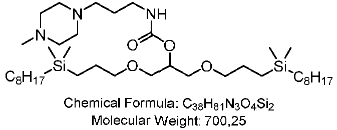 | **Example 16** |
| | 9,9,21,21-Tetramethyl-13,17-dioxa-9,21-disilanonacosan-15-yl (3-(4-methylpiperazin-1-yl)propyl) carbamate |
| | Formula (XIX) |
| 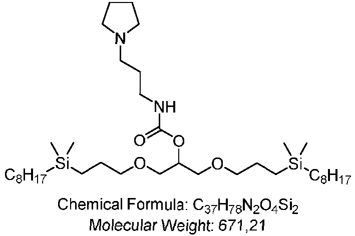 | **Example 17** |
| | 9,9,21,21-Tetramethyl-13,17-dioxa-9,21-disilanonacosan-15-yl (3-(pyrrolidin-1-yl)propyl)-carbamate |
| | Formula (XX) |
| 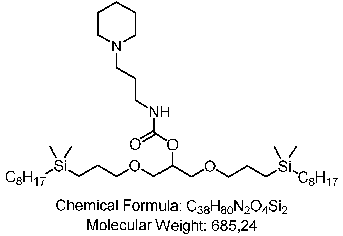 | **Example 18** |
| | 9,9,21,21-Tetramethyl-13,17-dioxa-9,21-disilanonacosan-15-yl (3-(piperidin-1-yl)propyl)-carbamate |
| | Formula (XXI) |
| 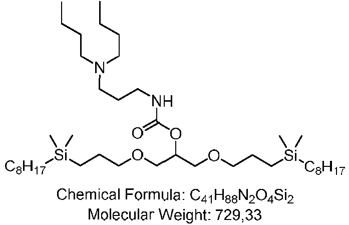 | **Example 19** |
| | 9,9,21,21-Tetramethyl-13,17-dioxa-9,21-disilanonacosan-15-yl (3-(dibutylamino)propyl)-carbamate |
| | Formula (XXII) |
| 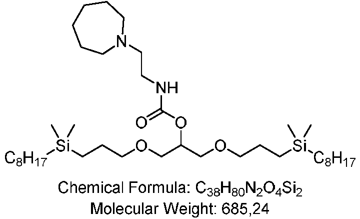 | **Example 20** |
| | 9,9,21,21-Tetramethyl-13,17-dioxa-9,21-disilanonacosan-15-yl (2-(azepan-1-yl)ethyl)-carbamate |
| | Formula (XXIII) |
| 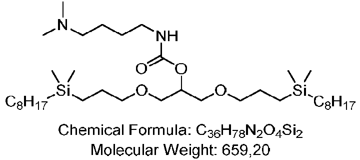 | **Example 21** |
| | 9,9,21,21-Tetramethyl-13,17-dioxa-9,21-disilanonacosan-15-yl (4-(dimethylamino)butyl)-carbamate |
| | Formula (XXIV) |
| 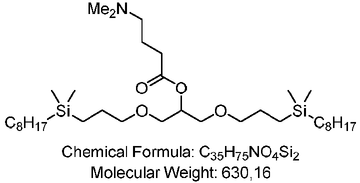 | **Example 22** |
| | 9,9,21,21-Tetramethyl-13,17-dioxa-9,21-disilanonacosan-15-yl 4-(dimethylamino)butanoate |
| | Formula (XXV) |
| 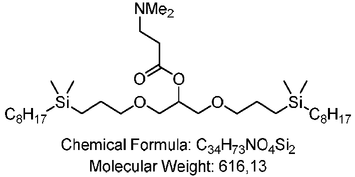 | **Example 23** |
| | 9,9,21,21-tetramethyl-13,17-dioxa-9,21-disilanonacosan-15-yl 3-(dimethylamino)propanoate |
| | Formula (XXVI) |
| 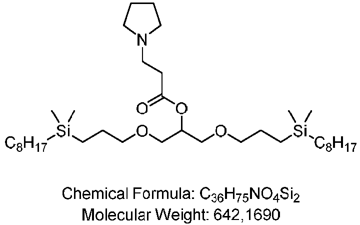 | **Example 24** |
| | 9,9,21,21-Tetramethyl-13,17-dioxa-9,21-disilanonacosan-15-yl 3-(pyrrolidin-1-yl)propanoate |
| | Formula (XXVII) |
| 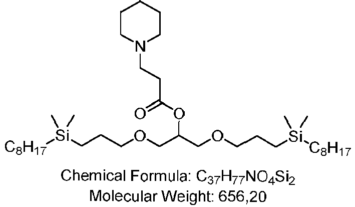 | **Example 25** |
| | 9,9,21,21-Tetramethyl-13,17-dioxa-9,21-disilanonacosan-15-yl 3-(piperidin-1-yl)propanoate |
| | Formula (XXVIII) |
| 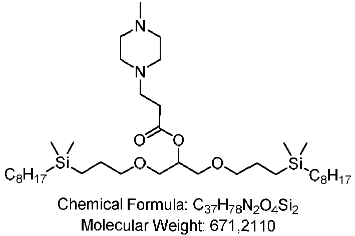 | **Example 26** |
| | 9,9,21,21-Tetramethyl-13,17-dioxa-9,21-disilanonacosan-15-yl 3-(4-methylpiperazin-1-yl)-propanoate |
| | Formula (XXIX) |
| 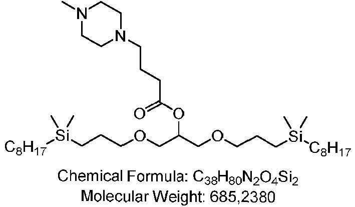 | **Example 27** |
| | 9,9,21,21-Tetramethyl-13,17-dioxa-9,21-disilanonacosan-15-yl 4-(4-methylpiperazin-1-yl)-butanoate |
| | Formula (XXX) |
| 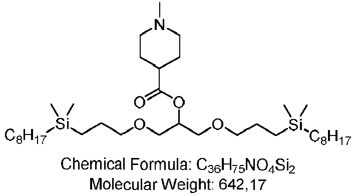 | **Example 28** |
| | 9,9,21,21-Tetramethyl-13,17-dioxa-9,21-disilanonacosan-15-yl 1-methylpiperidine-4-carboxylate |
| | Formula (XXXI) |
| 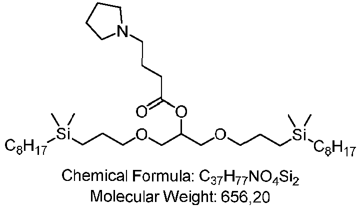 | **Example 29** |
| | 9,9,21,21-Tetramethyl-13,17-dioxa-9,21-disilanonacosan-15-yl 4-(pyrrolidin-1-yl)butanoate |
| | Formula (XXXII) |
| 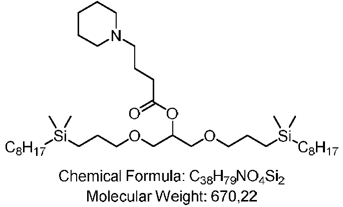 | **Example 30** |
| | 9,9,21,21-Tetramethyl-13,17-dioxa-9,21-disilanonacosan-15-yl 4-(piperidin-1-yl)butanoate |
| | Formula (XXXIII) |
| 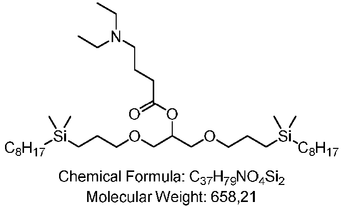 | **Example 31** |
| | 9,9,21,21-Tetramethyl-13,17-dioxa-9,21-disilanonacosan-15-yl 4-(diethylamino)butanoate |
| | Formula (XXXIV) |
| 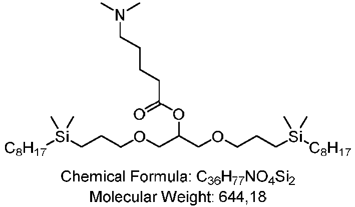 | **Example 32** |
| | 9,9,21,21-Tetramethyl-13,17-dioxa-9,21-disilanonacosan-15-yl 5-(dimethylamino)pentanoate |
| | Formula (XXXV) |
| 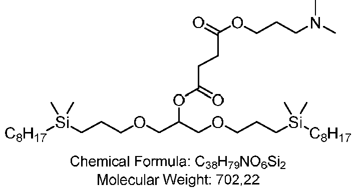 | **Example 33** |
| | 3-(Dimethylamino)propyl (9,9,21,21-tetramethyl-13,17-dioxa-9,21-disilanonacosan-15-yl) succinate |
| | Formula (XXXVI) |
| 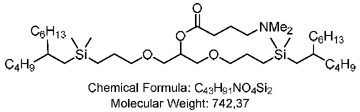 | **Example 34** |
| | 7,23-Dibutyl-9,9,21,21-tetramethyl-13,17-dioxa-9,21-disilanonacosan-15-yl 4-(dimethyl-amino)butanoate |
| | Formula (XXXVII) |
| 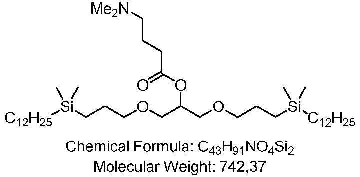 | **Example 35** |
| | 13,13,25,25-Tetramethyl-17,21-dioxa-13,25-disilaheptatriacontan-19-yl 4-(dimethylamino)-butanoate |
| | Formula (XXXVIII) |
| 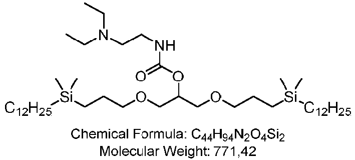 | **Example 36** |
| | 13,13,25,25-Tetramethyl-17,21-dioxa-13,25-disilaheptatriacontan-19-yl (2-(diethylamino)-ethyl)carbamate |
| | Formula (XXXIX) |
| 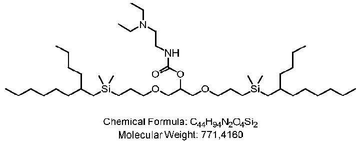 | **Example 37** |
| | 7,23-Dibutyl-9,9,21,21-tetramethyl-13,17-dioxa-9,21-disilanonacosan-15-yl (2-(diethylamino)-ethyl)carbamate |
| | Formula (XL) |
| 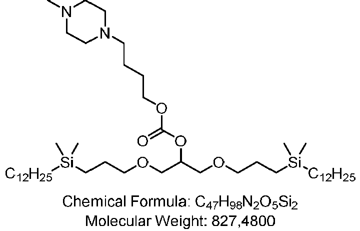 | **Example 38** |
| | 4-(4-Methylpiperazin-1-yl)butyl (13,13,25,25-tetramethyl-17,21-dioxa-13,25-disilaheptatria-contan-19-yl) carbonate |
| | Formula (XLI) |
| 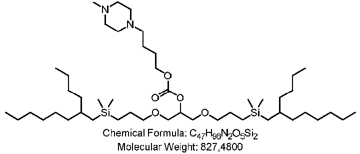 | **Example 39** |
| | 7,23-Dibutyl-9,9,21,21-tetramethyl-13,17-dioxa-9,21-disilanonacosan-15-yl (4-(4-methyl-piperazin-1-yl)butyl) carbonate |
| | Formula (XLII) |
| 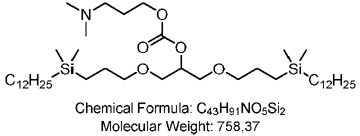 | **Example 40** |
| | 3-(Dimethylamino)propyl (13,13,25,25-tetramethyl-17,21-dioxa-13,25-disilaheptatriacontan-19-yl) carbonate |
| | Formula (XLIII) |
| 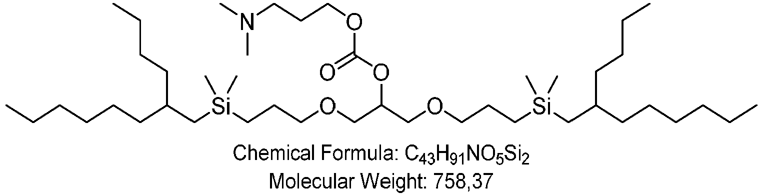 | |
| **Example 41** | |
| 7,23-Dibutyl-9,9,21,21-tetramethyl-13,17-dioxa-9,21-disilanonacosan-15-yl (3-(dimethyl-amino)propyl) carbonate | |
| Formula (XLIV) | |
| 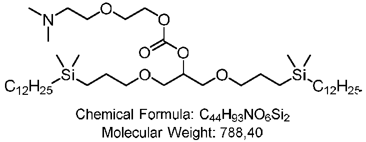 | **Example 42** |
| | 2-(2-(Dimethylamino)ethoxy)ethyl (13,13,25,25-tetramethyl-17,21-dioxa-13,25-disilaheptatria-contan-19-yl) carbonate |
| | Formula (XLV) |
| 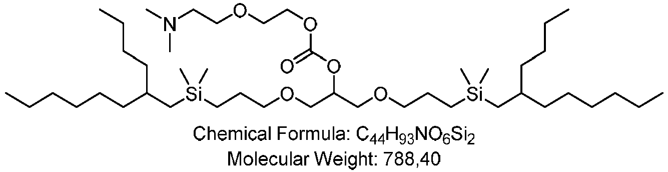 | |
| **Example 43** | |
| 7,23-Dibutyl-9,9,21,21-tetramethyl-13,17-dioxa-9,21-disilanonacosan-15-yl (2-(2-(dimethyl-amino)ethoxy)ethyl) carbonate | |
| Formula (XLVI) | |
| 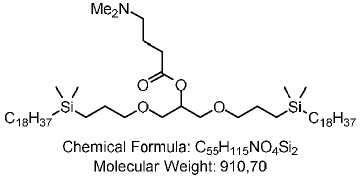 | **Example 44** |
| | 19,19,31,31-Tetramethyl-23,27-dioxa-19,31-disilanonatetracontan-25-yl 4-(dimethylamino)-butanoate) |
| | Formula (XLVII) |
| 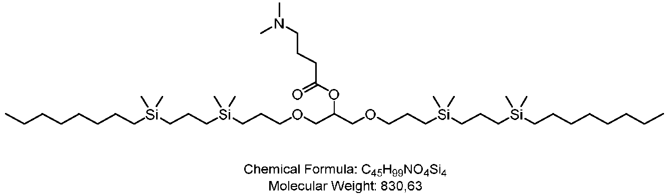 | |
| **Example 45** | |
| 9,9,13,13,25,25,29,29-Octamethyl-17,21-dioxa-9,13,25,29-tetrasilaheptatriacontan-19-yl 4-(dimethylamino)butanoate | |
| Formula (XLVIII) | |
| 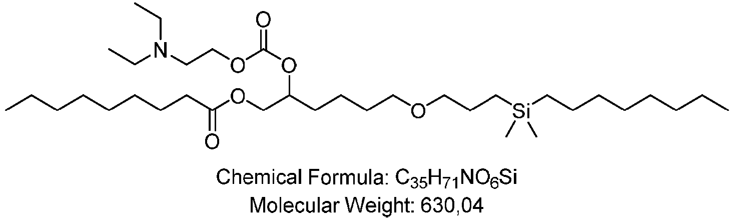 | |
| **Example 46** | |
| [2-[2-(Dimethylamino)ethoxycarbonyloxy]-6-[3-[diethyl(octyl)silyl]propoxy]hexyl] nonanoate | |
| Formula (XLIX) | |
| 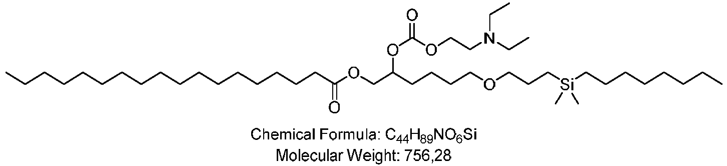 | |
| **Example 47** | |
| [2-[2-(Dimethylamino(ethoxycarbonyloxy]-6-[3-[diethyl(octyl)silyl]propoxy]hexyl] octadecanoate | |
| Formula (L) | |
| 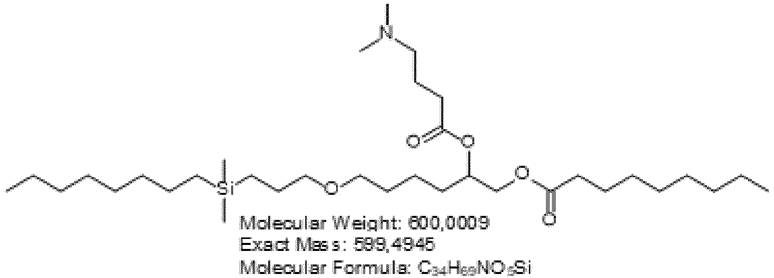 | |
| **Example 48** | |
| [2-[4-(Dimethylamino)butanoyloxy]-6-[3-[dimethyl(octyl)silyl]propoxy]hexyl] nonanoate | |
| Formula (LI) | |
| 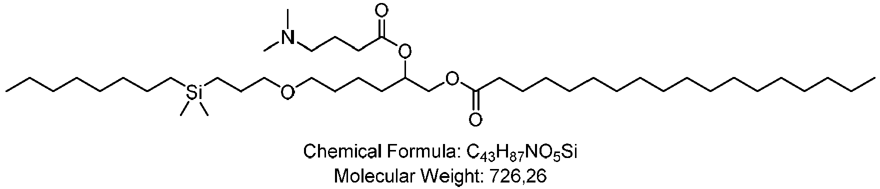 | |
| **Example 49** | |
| [2-[4-(dimethylamino)butanoyloxy]-6-[3-[dimethyl(octyl)silyl]propoxy]hexyl] octadecanoate | |
| Formula (LII) | |
| 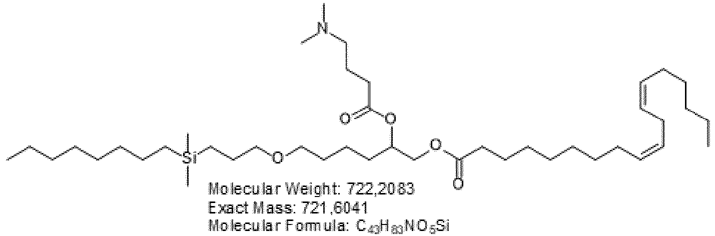 | |
| **Example 50** | |
| [2-[4-(Dimethylamino)butanoyloxy]-6-[3-[dimethyl(octyl)silyl]propoxy]hexyl] (9Z,12Z)-octa-deca-9,12-dienoate | |
| Formula (LIII) | |
| 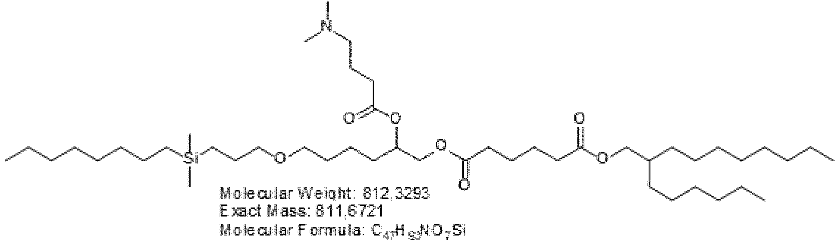 | |
| **Example 51** | |
| [2-[4-(Dimethylamino)butanoyloxy]-6-[3-[dimethyl(octyl)silyl]propoxy]hexyl] (2-hexyldecyl) hexanedioate | |
| Formula (LIV) | |
| 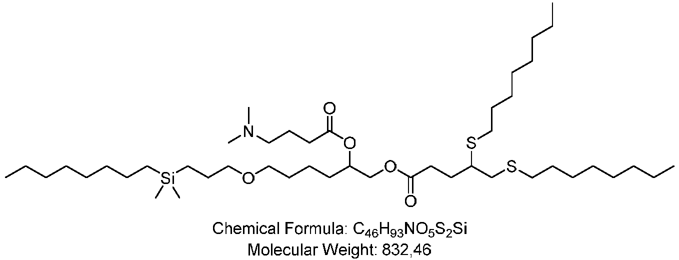 | |
| **Example 52** | |
| [2-[4-(dimethylamino)butanoyloxy]-6-[3-[dimethyl(octyl)silyl]propoxy]hexyl] 4,5-bis(octyl sulfanyl)pentanoate | |
| Formula (LV) | |

### 3 LNP production

### LNP production using screening approach:

LNPs for screening were prepared by pipette mixing in 96-well plates. Fluc encoding mRNA was formulated in a base formulation consisting of ionizable lipid/cholesterol/DSPC/DMG-PEG 2000 at a molar ration of 50/38.5/10/1.5 mol%, cₜₒₜₐₗ (lipid) = 12.5 mM, flow rate ratio (FRR) = 3 and N/P ratio = 6. DLin-MC3-DMA (MC3) based LNPs served as a frame of reference for nanoparticle formation, physicochemical characterization and transfection in vitro, due to its commercial availability and extended characterization in the literature.

In brief, lipid components were dissolved in abs. EtOH with the specified molar ratios above and rapidly mixed by pipetting Fluc encoding mRNA in 50 mM citrate buffer (pH 4). The resulting mixture was diluted with 2.5-fold with 50 mM citrate buffer (pH 4) and mixed with PBS (pH 7.4).

### LNP production using microfluidics:

LNPs for hit validation were prepared by microfluidic mixing using a microfluidic mixing device (NanoAssemblr Ignite, PNI, Canada). Fluc encoding mRNA was formulated in a base formulation consisting of ionizable lipid/cholesterol/DSPC/DMG-PEG 2000 at a molar ration of 50/38.5/10/1.5 mol%, cₜₒₜₐₗ (lipid) = 12.5 mM, flow rate ratio (FRR) = 3 and N/P ratio = 6. DLin-MC3-DMA (MC3) based LNPs served as a frame of reference for nanoparticle formation, physicochemical characterization and transfection in vitro, due to its commercial availability and extended characterization in the literature.

In brief, lipid components were dissolved in abs. EtOH with the specified molar ratios above and mixed with Fluc encoding mRNA in 50 mM citrate buffer (pH 4) using a total flow rate = 18 ml/min. The resulting mixture was diluted with 2.5-fold with 50 mM citrate buffer (pH 4). Subsequently, the mixture is dialyzed against PBS (pH 7.4) using cassette (Slide-A-Lyzer^{™} 10K MWCO, Thermo Fisher).

### Characterization of LNP:

Particle size and poly dispersity index (PDI) were determined by dynamic light scattering (DLS) in 1:100 dilutions in PBS as duplicates in a plate reader III (DynaPro^{®} Plate Reader III, Wyatt, USA).

The encapsulation efficiency (EE%) was determined using Quant-iT^{™} RiboGreen^{™} RNA Assay Kit (Invitrogen^{™}, Thermo Fisher Scientific, Waltham, MA, USA) with a modified protocol. Briefly, 100 µL of the diluted fluorescent dye (1:200) was added to 100 µL of diluted nanoparticle suspension in the presence or absence of 2% (w/v) Triton-X and incubated in absence of light according to the manufacturer's protocol.

### 4 Cell Culture Assays

### Luciferase

To determine luciferase activity, cells were seeded at different densities (HEK293: 3·10⁴ cells/well, HepG2: 4·10⁴ cells/well, THP-1: 4·10⁴ cells/well) and attached overnight. Cells were transfected with LNPs at a concentration of 1 µg/mL. Luciferase activity was determined using the Bright-Glo^{™} Luciferase assay system from Promega according to manufacturer's instructions after 6 and 24 hours. Luminescence was measured on the Variolux Skan multimode plate reader from ThermoFisher. The evaluation of luciferase activity in relative luminescence units (RLU) was carried out using Microsoft Excel.

To ensure appropriate evaluation of luciferase activity and thus lipid functionality, a negative and a positive control were included. The negative control did not contain any LNPs, but was nevertheless measured with the Bright-Glo^{™} Luciferase assay system to determine background signal. As positive control a commercially available Si-free benchmark lipid was applied. The used commercially available Si-free benchmark lipid was O-(Z, Z, Z, Z-heptatriaconta-6, 9, 26, 29-tetraen-19-yl)-4-(N, N-dimethylamino) purchased from from Hölzel Biotech as D-Lin-MC3-DMA(MC3). The postive control was set to 100% and the Luciferase activity of the LNPs containing novel lipids was determined accordingly.

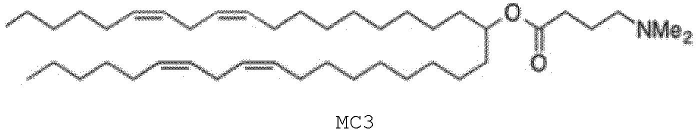

### Viability

To determine cellular viability, cells were seeded at different densities (HEK293: 3·10⁴ cells/well, HepG2: 4·10⁴ cells/well, THP-1: 4·10⁴ cells/well) and attached overnight. Cells were transfected with LNPs at a concentration of 1 µg/mL. In parallel, the corresponding reagents of the RealTime-Glo^{™} MT Cell Viability Assay from Promega were added according to manufacturer's instructions after 6 and 24 hours. Luminescence was measured on the Variolux Skan multimode plate reader from ThermoFisher. The evaluation of luciferase activity in relative luminescence units (RLU) was carried out using Microsoft Excel.

Results of the LNP characterization as well as the cell culture assays are summarized in the following table.

**Table 2: LNP characterization and results of cell culture assays**

| **No.** | **EE [%]** | **Luc. act. HEK293 [RLU@6/ 24h]** | **Luc. act. HepG2 [RLU@6/ 24h]** | **Luc. act. THP-1 [RLU@6/ 24h]** | **Cell Viability** |
|---|---|---|---|---|---|
| **1** | 57 | 6h | | | 6h |
| | | - 0,00 | | | - 0,9 |
| | | - 0,00 | | | - 0,9 |
| | | 24h | | | 24h |
| | | - 0,00 | | | - 1,34 |
| | | - 0,00 | | | - 0,97 |
| **2** | 98 | 6h | | | 6h |
| | | - 0,13 | | | - 1,88 |
| | | 24h | | | 24h |
| | | - 0,34 | | | - 1,47 |
| **3** | 98 | 6h | | | 6h |
| | | - 0,30 | | | - 1,84 |
| | | - 2,19 | | | - 1,13 |
| | | - 0,45 | | | - 1,06 |
| | | - 0,83 | | | - 0,92 |
| | | - 0,46 | | | - 1,01 |
| | | 24h | | | 24h |
| | | - 0,75 | | | - 1,63 |
| | | - 1,28 | | | - 1,07 |
| | | - 0,55 | | | - 1,09 |
| | | - 0,43 | | | - 0,88 |
| | | - 0,52 | | | - 0,68 |
| **4** | 20 | 6h | | | 6h |
| | | - 0,00 | | | - 1,78 |
| | | 24h | | | 24h |
| | | - 0,00 | | | - 1,52 |
| **5** | 77 | 6h | | | 6h |
| | | - 0,01 | | | - 1,80 |
| | | 24h | | | 24h |
| | | - 0,04 | | | - 1,54 |
| **6** | 40 | 6h | | | 6h |
| | | - 0,00 | | | - 1,93 |
| | | 24h | | | 24h |
| | | - 0,00 | | | - 1,64 |
| **7** | 97 | 6h | | | 6h |
| | | - 0,52 | | | - 1,22 |
| | | - 1,39 | | | - 0,90 |
| | | - 0,45 | | | - 1,00 |
| | | - 0,90 | | | - 0,96 |
| | | - 0,19 | | | - 0,85 |
| | | 24h | | | 24h |
| | | - 0,67 | | | - 1,31 |
| | | - 3,7 | | | - 1,05 |
| | | - 0,47 | | | - 0,95 |
| | | - 4,82 | | | - 0,93 |
| | | - 0,76 | | | - 1,00 |
| **8** | 95 | 6h | | | 6h |
| | | - 1,14 | | | - 1,18 |
| | | - 1,01 | | | - 1,06 |
| | | - 0,88 | | | - 0,95 |
| | | - 0,42 | | | - 1,11 |
| | | - 0,55 | | | - 0,85 |
| | | 24h | | | 24h |
| | | - 1,00 | | | - 1,06 |
| | | - 5,67 | | | - 1,01 |
| | | - 0,91 | | | - 0,98 |
| | | - 0,55 | | | - 1,00 |
| | | - 0,36 | | | - 0,62 |
| **9** | 78 | 6h | | | 6h |
| | | - 0,24 | | | - 1,29 |
| | | - 0,10 | | | - 0,97 |
| | | 24h | | | 24h |
| | | - 0,29 | | | - 1,18 |
| | | - 0,09 | | | - 0,86 |
| **10** | 97 | 6h | | | 6h |
| | | - 0,69 | | | - 1,23 |
| | | - 0,79 | | | - 1,10 |
| | | - 0,63 | | | - 1,00 |
| | | - 0,20 | | | - 1,13 |
| | | - 0,04 | | | - 0,84 |
| | | 24h | | | 24h |
| | | - 1,61 | | | - 1,04 |
| | | - 5,15 | | | - 1,08 |
| | | - 0,63 | | | - 1,05 |
| | | - 0,50 | | | - 1,10 |
| | | - 0,18 | | | - 0,40 |
| **11** | 94 | 6h | | | 6h |
| | | - 0,08 | | | - 0,94 |
| | | - 0,54 | | | - 1,05 |
| | | 24h | | | 24h |
| | | - 0,10 | | | - 0,89 |
| | | - 0,28 | | | - 0,88 |
| **12** | 99 | 6h | | | 6h |
| | | - 0,70 | | | - 0,79 |
| | | - 0,12 | | | - 0,89 |
| | | - 0,17 | | | - 0,96 |
| | | 24h | | | 24h |
| | | - 0,69 | | | - 0,42 |
| | | - 0,02 | | | - 0,66 |
| | | - 0,20 | | | - 0,61 |
| **13** | 95 | 6h | | | 6h |
| | | - 0,04 | | | - 0,91 |
| | | 24h | | | 24h |
| | | - 0,05 | | | - 0,71 |
| **14** | 96 | 6h | | | 6h |
| | | - 1,26 | | | - 1,21 |
| | | - 1,07 | | | - 1,14 |
| | | - 1,21 | | | - 0,99 |
| | | - 0,11 | | | - 1,16 |
| | | - 0,51 | | | - 0,89 |
| | | 24h | | | 24h |
| | | - 0,81 | | | - 1,10 |
| | | - 1,93 | | | - 1,05 |
| | | - 0,95 | | | - 0,90 |
| | | - 1,09 | | | - 1,02 |
| | | - 0,28 | | | - 0,64 |
| **15** | 98 | 6h | | | 6h |
| | | - 0,41 | | | - 1,04 |
| | | 24h | | | 24h |
| | | - 0,28 | | | - 0,66 |
| **16** | 96 | 6h | | | 6h |
| | | - 1,51 | | | - 1,06 |
| | | 24h | | | 24h |
| | | - 0,26 | | | - 0,65 |
| **17** | 97 | 6h | | | 6h |
| | | - 0,18 | | | - 0,96 |
| | | 24h | | | 24h |
| | | - 0,05 | | | - 0,81 |
| **18** | 97 | 6h | | | 6h |
| | | - 0,17 | | | - 0,96 |
| | | 24h | | | 24h |
| | | - 0,18 | | | - 0,89 |
| **19** | 83 | 6h | | | 6h |
| | | - 0,01 | | | - 0,88 |
| | | - 0,00 | | | - 1,03 |
| | | 24h | | | 24h |
| | | - 0,01 | | | - 0,84 |
| | | - 0,02 | | | - 1,18 |
| **20** | 95 | 6h | | | 6h |
| | | - 0,02 | | | - 1,12 |
| | | 24h | | | 24h |
| | | - 0,03 | | | - 0,90 |
| **21** | 96 | 6h | | | 6h |
| | | - 0,53 | | | - 0,84 |
| | | 24h | | | 24h |
| | | - 0,05 | | | - 0,54 |
| **22** | 98 | 6h | 6h | 6h | 6h |
| | | - 0,09 | - 1,85 | - 1,18 | - 0,9 (HEK) |
| | | - 0,65 | 24h | 24h | - 0,9 (HEK) |
| | | - 2,48 | - 1,84 | - 2,46 | - 0,9 (HEK) |
| | | 24h | | | - 0,76 (HepG2) |
| | | - 0,78 | | | |
| | | - 1,07 | | | - 1,04 (THP1) |
| | | - 2,22 | | | |
| | | | | | 24h |
| | | | | | - 0,9 (HEK) |
| | | | | | - 0,9 (HEK) |
| | | | | | - 0,9 (HEK) |
| | | | | | - 1,47 (HepG2) |
| | | | | | - 0,73 (THP1) |
| **23** | 97 | 6h | | | 6h |
| | | - 0,07 | | | - 0,8 |
| | | 24h | | | 24h |
| | | - 0,13 | | | - 0,88 |
| **24** | 90 | 6h | | | 6h |
| | | - 0,17 | | | - 0,9 |
| | | 24h | | | 24h |
| | | - 0,13 | | | - 1,02 |
| **25** | 57 | 6h | | | 6h |
| | | - 0,00 | | | - 0,9 |
| | | 24h | | | 24h |
| | | - 0,00 | | | - 0,83 |
| **26** | 98 | 6h | | | 6h |
| | | - 0,42 | | | - 0,8 |
| | | - 0,52 | | | - 1,0 |
| | | - 1,29 | | | - 0,9 |
| | | 24h | | | 24h |
| | | - 0,59 | | | - 0,56 |
| | | - 0,25 | | | - 1,07 |
| | | - 0,70 | | | - 0,68 |
| **27** | 89 | 6h | | | 6h |
| | | - 0,19 | | | - 1,16 |
| | | 24h | | | 24h |
| | | - 0,55 | | | - 1,08 |
| **28** | 95 | 6h | | | 6h |
| | | - 0,88 | | | - 0,94 |
| | | - 0,68 | | | - 0,99 |
| | | - 1,27 | | | - 1,01 |
| | | - 0,32 | | | - 1,33 |
| | | 24h | | | 24h |
| | | - 0,52 | | | - 0,70 |
| | | - 0,37 | | | - 0,92 |
| | | - 2,89 | | | - 1,07 |
| | | - 0,95 | | | - 1,15 |
| **29** | 96 | 6h | | | 6h |
| | | - 0,49 | | | - 0,95 |
| | | - 0,53 | | | - 0,88 |
| | | 24h | | | 24h |
| | | - 0,54 | | | - 0,99 |
| | | - 0,48 | | | - 0,83 |
| **30** | 93 | 6h | | | 6h |
| | | - 0,08 | | | - 0,93 |
| | | 24h | | | 24h |
| | | - 0,16 | | | - 0,96 |
| **31** | 96 | 6h | | | 6h |
| | | - 0,35 | | | - 0,93 |
| | | - 0,23 | | | - 0,98 |
| | | 24h | | | 24h |
| | | - 0,50 | | | - 0,95 |
| | | - 0,46 | | | - 0,95 |
| **32** | 97 | 6h | | | 6h |
| | | - 0,38 | | | - 0,99 |
| | | - 0,39 | | | - 0,99 |
| | | - 0,39 | | | - 0,99 |
| | | 24h | | | 24h |
| | | - 0,73 | | | - 0,83 |
| | | - 0,33 | | | - 1,00 |
| | | - 0,36 | | | - 1,14 |
| **33** | 97 | 6h | | | 6h |
| | | - 0,20 | | | - 0,93 |
| | | - 1,25 | | | - 0,84 |
| | | - 0,24 | | | - 1,20 |
| | | 24h | | | 24h |
| | | - 0,83 | | | - 0,72 |
| | | - 0,22 | | | - 0,85 |
| | | - 1,40 | | | - 1,27 |
| **34** | 91 | 6h | | | 6h |
| | | - 2,85 | | | - 1,15 |
| | | - 0,66 | | | - 1,04 |
| | | - 0,26 | | | - 1,28 |
| | | 24h | | | 24h |
| | | - 4,58 | | | - 1,20 |
| | | - 0,80 | | | - 1,13 |
| | | - 0,32 | | | - 1,30 |
| **35** | 88 | 6h | | | 6h |
| | | - 5,61 | | | - 1,14 |
| | | - 0,29 | | | - 0,95 |
| | | - 0,91 | | | - 1,04 |
| | | - 0,57 | | | - 1,28 |
| | | 24h | | | 24h |
| | | - 12,88 | | | - 1,17 |
| | | - 0,53 | | | - 0,95 |
| | | - 1,76 | | | - 1,05 |
| | | - 0,67 | | | - 1,10 |
| **36** | 91 | 6h | | | 6h |
| | | - 0,03 | | | - 0,93 |
| | | - 0,20 | | | - 0,90 |
| | | 24h | | | 24h |
| | | - 0,17 | | | - 1,10 |
| | | - 0,40 | | | - 0,82 |
| **37** | 97 | 6h | | | 6h |
| | | - 0,02 | | | - 0,95 |
| | | 24h | | | 24h |
| | | - 0,08 | | | - 1,02 |
| **38** | 85 | 6h | | | 6h |
| | | - 0,21 | | | - 0,99 |
| | | 24h | | | 24h |
| | | - 0,02 | | | - 0,90 |
| **39** | 93 | 6h | | | 6h |
| | | - 0,04 | | | - 0,92 |
| | | 24h | | | 24h |
| | | - 0,03 | | | - 1,16 |
| **40** | 87 | 6h | | | 6h |
| | | - 0,05 | | | - 0,98 |
| | | 24h | | | 24h |
| | | - 0,04 | | | - 1,08 |
| **41** | 96 | 6h | | | 6h |
| | | - 0,11 | | | - 1,00 |
| | | - 0,11 | | | - 0,91 |
| | | 24h | | | 24h |
| | | - 0,05 | | | - 1,28 |
| | | - 0,09 | | | - 1,05 |
| **42** | 94 | 6h | | | 6h |
| | | - 0,00 | | | - 0,98 |
| | | - 0,02 | | | - 0,91 |
| | | 24h | | | 24h |
| | | - 0,05 | | | - 0,92 |
| | | - 0,09 | | | - 0,51 |
| **43** | 98 | 6h | | | 6h |
| | | - 0,00 | | | - 0,71 |
| | | - 0,27 | | | - 0,95 |
| | | 24h | | | 24h |
| | | - 0,22 | | | - 1,33 |
| | | - 0,77 | | | - 1,01 |
| **44** | 91 | 6h | | | 6h |
| | | - 0,01 | | | - 0,85 |
| | | 24h | | | 24h |
| | | - 0,00 | | | - 0,84 |
| **45** | 96 | 6h | | | 6h |
| | | - 0,22 | | | - 0,96 |
| | | - 0,23 | | | - 0,92 |
| | | 24h | | | 24h |
| | | - 0,14 | | | - 1,09 |
| | | - 0,45 | | | - 0,77 |
| **46** | 95 | 6h | | | 6h |
| | | - 0,02 | | | - 0,86 |
| | | - 0,00 | | | - 0,86 |
| | | - 0,00 | | | - 1,12 |
| | | - 0,01 | | | - 1,34 |
| | | 24h | | | 24h |
| | | - 0,02 | | | - 0,83 |
| | | - 0,00 | | | - 0,86 |
| | | - 0,00 | | | - 0,98 |
| | | - 0,00 | | | - 1,40 |
| **47** | 54 | 6h | | | 6h |
| | | - 0,00 | | | - 0,88 |
| | | 24h | | | 24h |
| | | - 0,02 | | | - 0,99 |
| **48** | 97 | 6h | | | 6h |
| | | - 0,03 | | | - 0,94 |
| | | - 0,01 | | | - 0,97 |
| | | - 0,00 | | | - 0,89 |
| | | - 0,00 | | | - 0,92 |
| | | 24h | | | 24h |
| | | - 0,01 | | | - 0,54 |
| | | - 0,02 | | | - 0,90 |
| | | - 0,00 | | | - 0,76 |
| | | - 0,00 | | | - 0,87 |
| **49** | 97 | | | | |
| **50** | 93 | 6h | | | 6h |
| | | - 0,00 | | | - 0,73 |
| | | - 0,00 | | | - 0,87 |
| | | - 0,00 | | | - 0,67 |
| | | - 0,00 | | | - 0,86 |
| | | 24h | | | 24h |
| | | - 0,00 | | | - 0,29 |
| | | - 0,00 | | | - 1,02 |
| | | - 0,00 | | | - 0,40 |
| | | - 0,00 | | | - 0,89 |
| **51** | 98 | 6h | | | 6h |
| | | - 0,92 | | | - 1,01 |
| | | 24h | | | 24h |
| | | - 1,00 | | | - 1,07 |
| **52** | 97 | | | | |

Furthermore, Luciferase activity and cellular viability for LNPs containing the lipids according to the invention have been compared to the reference lipid. Results are summarized in the following table.

**Table 3: comparison of cell culture results with benchmark lipid**

| **No.** | Luc. Act. comparison to reference | Cell viability comparison to reference |
|---|---|---|
| 3 | 0.71 | 1.07 |
| 7 | 2.08 | 1.05 |
| 8 | 1.70 | 0.93 |
| 10 | 1.61 | 0.93 |
| 14 | 1.01 | 0.94 |
| 22 | 1.34 | 0.90 |
| 26 | 0.51 | 0.77 |
| 28 | 1.45 | 0.96 |
| 33 | 1.12 | 0.95 |
| 34 | 1.87 | 1.21 |
| 35 | 0.99 | 1.07 |
| 43 | 0.54 | 1.17 |
| 49 | 1.74 | 0.99 |
| 51 | 1.59 | 1.07 |
| 52 | 0.95 | 0.98 |

Since the benchmark lipid used for comparison achieves very high Luciferase activities, reaching an activity amounting to at least 50 % compared to the benchmark is acceptable in terms of the present invention, preferably amounting to at least 100 %, in particular amounting to at least 150 %.

The above-mentioned lipids were tested in robust in vitro cell culture experimental settings and therefore provide strong evidence for their superior performance compared to the FDA-approved benchmark lipid MC3. Moreover, the lipids showed very high Luciferase activities, as a measure for transfection, as well as comparably low toxicity. In addition, all lipids in table 3 achieved very high encapsulation efficiencies in nanoparticle formulations in comparison to the benchmark LNP.

The present invention is furthermore characterized by the following items.
1. Compound according to chemical formula (I) or its isomeric form 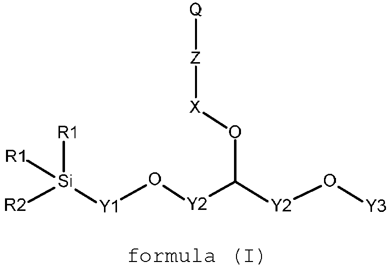 wherein
- **R1**: represents independently from each other H, OH, or a C1-C16 hydrocarbon residue, which can be linked to the Si atom by an O atom,
preferably H, OH, or a C1-C6 hydrocarbon residue, which can be linked to the Si atom by an O atom,
more preferably H, OH, or a C1-C6 alkyl group, which can be linked to the Si atom by an O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl linking group, in particular -CH₂-CH₂-CH₂-;
- **Y2**: represents independently from each other a C1-C10 hydrocarbon linking group, preferably a C1-C6 hydrocarbon linking group, more preferably a C1-C4 alkyl linking group, more preferably -CH₂- or -CH₂-(CH₂)₂-CH₂-, in particular -CH₂-;
- **Y3**: represents
-**Y1**-Si(**R1**)₂**R2** with the definitions given above, or
-(C=O)-**R5**, wherein **R5** represents an organic residue comprising at least 5 C atoms, preferably at least 8 C atoms, in particular 8 to 21 C atoms,
   wherein the organic residue **R5** can be branched or linear and/or
   wherein the organic residue **R5** can be saturated or unsaturated, preferably saturated and/or
   wherein the organic residue **R5** can contain further heteroatoms selected from O, N or S, preferably O and S;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **Q**: represent an organic residue comprising 2 to 20 C-atoms, preferably 2 to 12 C atoms, and at least one N atom, wherein the organic residue can be saturated or unsaturated, preferably saturated.
2. Compound according to item 1, which is a compound according to chemical formula (Ia) or its isomeric form 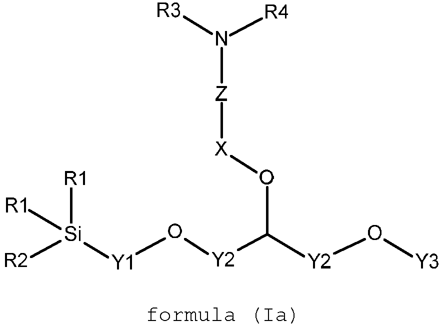 wherein
- **R1**: represents independently from each other H, OH, or a C1-C16 hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl group, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl linking group, in particular - CH₂-CH₂-CH₂-;
- **Y2**: represents independently from each other a C1-C10 hydrocarbon linking group, preferably a C1-C6 hydrocarbon linking group, more preferably a C1-C4 alkyl linking group, more preferably -CH₂- or -CH₂-(CH₂)₂-CH₂-, in particular -CH₂-;
- **Y3**: represents
-**Y1**-Si(**R1**)₂**R2** with the definitions given above, or
-(C=O)-**R5**, wherein **R5** represents an organic residue comprising at least 5 C atoms, preferably at least 8 C atoms, in particular 8 to 21 C atoms
   wherein the organic residue **R5** can be branched or linear and/or
   wherein the organic residue **R5** can be saturated or unsaturated, preferably saturated and/or
   wherein the organic residue **R5** can contain further heteroatoms selected from O, N or S, preferably O and S;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **R3** and **R4**: represent independently from each other a C1-C6 hydrocarbon residue, preferably a C1-C4 alkyl residue, in particular a methyl, ethyl or butyl residue, wherein **R3** and/or **R4** may contain heteroatoms selected from N and O,
or **R3** and **R4** can form a ring together, including the N atom they are bound to, wherein this ring comprises 4-8 carbon atoms, preferably 4-6 carbon atoms, and optionally further heteroatoms selected from O and N.
3. Compound according to item 1, which is a compound according to chemical formula (Ib) or its isomeric form 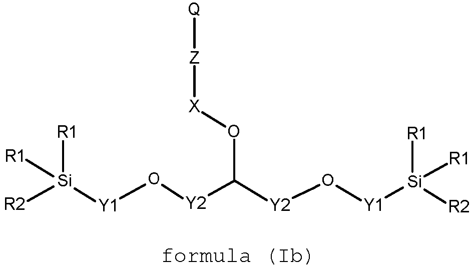 wherein
- **R1**: represents independently from each other H, OH, or a C1-C16 hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl residue, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl linking group, in particular - CH₂-CH₂-CH₂-;
- **Y2**: represents independently from each other a C1-C10 hydrocarbon linking group, preferably a C1-C6 hydrocarbon linking group, more preferably a C1-C4 alkyl linking group, more preferably -CH₂- or -CH₂-(CH₂)₂-CH₂-, in particular -CH₂-;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **Q**: represent an organic residue comprising 2 to 20 C atoms, preferably 2 to 12 C atoms, and at least one N atom, wherein the organic residue can be saturated or unsaturated, preferably saturated.
4. Compound according to any of the preceding items, which is a compound according to chemical formula (Ic) or its isomeric form 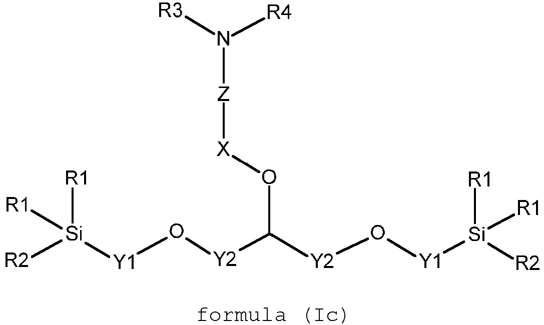 wherein
- **R1**: represents independently from each other H, OH, or a C1-C16 hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl goup, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl linking group, in particular -CH₂-CH₂-CH₂-;
- **Y2**: represents independently from each other a C1-C10 hydrocarbon linking group, preferably a C1-C6 hydrocarbon linking group, more preferably a C1-C4 alkyl linking group, more preferably -CH₂- or -CH₂-(CH₂)₂-CH₂-, in particular -CH₂-;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **R3** and **R4**: represent independently from each other a C1-C6 hydrocarbon residue, preferably a C1-C4 alkyl residue, in particular a methyl, ethyl or butyl residue, wherein **R3** and/or **R4** may contain heteroatoms selected from N and O,
or **R3** and **R4** can form a ring together, including the N atom they are bound to, wherein this ring comprises 4-8 carbon atoms, preferably 4-6 carbon atoms, and optionally further heteroatoms selected from O and N.
5. Compound according any of the preceding items, wherein all **R1** represent the same residue, selected from OH or CH₃, preferably CH₃.
6. Compound according to any of the preceding items, wherein said heteroatom is selected from Si, O, P and N, preferably O and N.
7. Compound according to any of the preceding items, wherein **R2** is independently from each other selected from a linear or branched C6-C20 alkyl or alkenyl residue, in particular alkyl residue, preferably from a linear or branched C6-C12 alkyl or alkenyl residue, in particular alkyl residue, most preferably selected from the group consisting of -C₆H₁₃, -C₈H₁₇, -C₁₂H₂₅ and -CH₂-CH(C₆H₁₃)(C₄H₉).
8. Compound according to any of the preceding items, wherein all **R2** represent the same residue, selected from a linear or branched C6-C20 alkyl residue, preferably a linear or branched C6-C12 alkyl residue, in particular selected from the group consisting of -C₆H₁₃, -C₈H₁₇, -C₁₂H₂₅ and -CH₂-CH(C₆H₁₃)(C₄H₉).
9. Compound according to any of the preceding items, wherein all **Y1** represent the same linking group, selected from a C1-C10 hydrocarbon linking group, preferably a C1-C6 hydrocarbon linking group, more preferably a C1-C4 alkyl group, in particular -CH₂-CH₂-CH₂-.
10. Compound according to any of the preceding items, wherein all **Y2** represent the same linking group, selected from a C1-C10 hydrocarbon linking group, preferably a C1-C6 hydrocarbon linking group, more preferably a C1-C4 alkyl linking group, more preferably -CH₂- or -CH₂-(CH₂)₂-CH₂-, in particular -CH₂-.
11. Compound according to any of the preceding items, wherein **X** is selected from

**b¹**-(C=O)-**b²**, **b¹**-(C=O)-O-**b²**, **b¹**-(C=O)-S-**b²** or **b¹**-(C=O)(N-**R'**)**-b²**,

wherein **b¹** represents the bond to the O atom and **b²** represents the bond to **Z** or the N atom, in case **Z** represents a chemical bond, and **R'** is selected from the group consisting of H and C1-C4 hydrocarbon, preferably H and C1-C4 alkyl, more preferably H or -CH₃, in particular H.
12. Compound according to any of the preceding items, wherein **Z** represents a chemical bond or a C1-C5 alkyl linker, more preferably a chemical bond, -CH₂-CH₂- or -CH₂-CH₂-CH₂-, in particular -CH₂-CH₂- or -CH₂-CH₂-CH₂-.
13. Compound according to any of the preceding items, wherein the ring, which **R3** and **R4** form together with the N atom they are bound to represents a pyrrolidine, piperidine, alkylpiperidine, such as methylpiperidine, azepane, alkylpiperazine, such as N-methylpiperazine, and morpholine.
14. Compound according to any of the preceding items, which is a compound according to chemical formula (II) 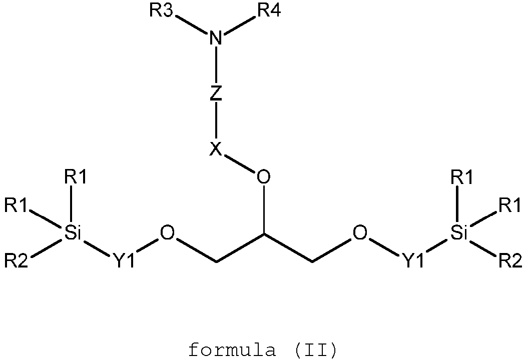 wherein
- **R1**: represents independently from each other H, OH, or a C1-C16
hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl group, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **Y1**: represents independently from each other a C1-C10 hydrocarbon linking group, preferably a C1-C6 hydrocarbon linking group, more preferably a C1-C4 alkyl group, in particular -CH₂-CH₂-CH₂-;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **R3** and **R4**: represent independently from each other a C1-C6 hydrocarbon residue, preferably a C1-C4 alkyl residues, in particular a methyl, ethyl or butyl residue,
or **R3** and **R4** can form a ring together, including the N atom they are bound to, wherein this ring comprises 4-8 carbon atoms, preferably 4-6 carbon atoms, and optionally heteroatoms selected from O and N.
15. Compound according to item 14, wherein all **R1** represent the same residue, selected from OH or CH₃, preferably CH₃.
16. Compound according to item 14 or 15, wherein said heteroatom is selected from Si, O, P and N, preferably O and N.
17. Compound according to any of items 14-16, wherein **R2** is independently from each other selected from a linear or branched C6-C20 alkyl residue, preferably from a linear or branched C6-C12 alkyl residue, in particular selected from the group consisting of -C₆H₁₃, -C₈H₁₇, -C₁₂H₂₅ and -CH₂-CH(C₆H₁₃)(C₄H₉).
18. Compound according to any of items 14-17, wherein all **R2** represent the same residue, selected from a linear or branched C6-C20 alkyl residue, preferably a linear or branched C6-C12 alkyl residue, in particular selected from the group consisting of -C₆H₁₃, -C₈H₁₇, -C₁₂H₂₅ and -CH₂-CH(C₆H₁₃)(C₄H₉).
19. Compound according to any of items 14-18, wherein all **Y1** represent the same linking group, selected from a C1-C10 hydrocarbon linking group, preferably a C1-C6 hydrocarbon linking group, more preferably a C1-C4 alkyl group, in particular -CH₂-CH₂-CH₂-.
20. Compound according to any of items 14-19, wherein **X** is selected from

**b¹**-(C=O)-**b²**, **b¹-**(C=O)-O-**b²**, **b¹-**(C=O)-S-**b²** or **b¹**-(C=O)(N-**R'**)-**b²**,

wherein **b¹** represents the bond to the O atom and **b²** represents the bond to **Z** or the N atom, in case **Z** represents a chemical bond, and **R'** is selected from the group consisting of H and C1-C4 hydrocarbon, preferably H and C1-C4 alkyl, more preferably H or -CH₃, in particular H.
21. Compound according to any of items 14-20, wherein **Z** represents a chemical bond or a C1-C5 alkyl linker, more preferably a chemical bond, -CH₂-CH₂- or -CH₂-CH₂-CH₂-, in particular -CH₂-CH₂- or -CH₂-CH₂-CH₂-.
22. Compound according to any of items 14-21, wherein the ring, which **R3** and **R4** form together with the N atom they are bound to represents a pyrrolidine, piperidine, alkylpiperidine, such as methylpiperidine, azepane, alkylpiperazine, such as N-methylpiperazine, and morpholine.
23. Compound according to any of the preceding items, which is a compound according to chemical formula (III) 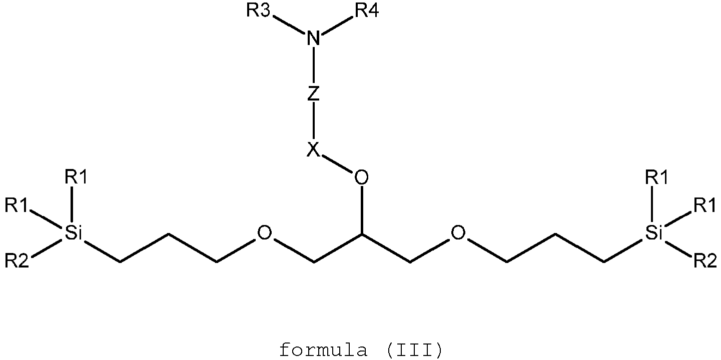 wherein
- **R1**: represents independently from each other H, OH, or a C1-C16
hydrocarbon residue, which can be linked to the Si atom by a O atom,
preferably H, OH, or a C1-C6 hydrocarbon residue, which can be linked to the Si atom by a O atom,
more preferably H, OH, or a C1-C6 alkyl group, which can be linked to the Si atom by a O atom,
more preferably H, OH, or a C1-C6 alkyl group,
more preferably H, OH, CH₃, or C₆H₁₃
more preferably H, OH or CH₃,
more preferably OH or CH₃,
in particular CH₃;
- **R2**: is independently from each other identical to **R1** or represents independently from each other a C6-C20 hydrocarbon residue, which can be substituted and/or can comprise at least one heteroatom,
preferably represents independently from each other a C6-C20 hydrocarbon residue, which can be substituted and/or can comprise at least one heteroatom,
preferably represents independently from each other a C6-C12 hydrocarbon residue, which can be substituted and/or can comprise at least one heteroatom;
- **X**: represents together with the O-atom, to which **X** is bound, a carbonate, thio-carbonate, carbamate or ester based linker;
- **Z**: represents a chemical bond or an organic linker comprising 1 to 5 C atoms, which may further contain at least one heteroatom selected from O and N; and
- **R3** and **R4**: represent independently from each other a C1-C6 hydrocarbon residue, preferably a C1-C4 alkyl residues, in particular a methyl, ethyl or butyl residue,
or **R3** and **R4** can form a ring together, including the N atom they are bound to, wherein this ring comprises 4-8 carbon atoms, preferably 4-6 carbon atoms, and optionally heteroatoms selected from O and N.
24. Compound according item 23, wherein all **R1** represent the same residue, selected from OH or CH₃, preferably CH₃.
25. Compound according to item 23 or 24, wherein said heteroatom is selected from O, P and N, preferably O and N.
26. Compound according to any of items 23-25, wherein **R2** is independently from each other selected from a linear or branched C6-C20 alkyl residue, preferably from a linear or branched C6-C12 alkyl residue, in particular selected from the group consisting of -C₆H₁₃, -C₈H₁₇, -C₁₂H₂₅ and -CH₂-CH(C₆H₁₃)(C₄H₉).
27. Compound according to any of items 23-26, wherein all **R2** represent the same residue, selected from a linear or branched C6-C20 alkyl residue, preferably a linear or branched C6-C12 alkyl residue, in particular selected from the group consisting of -C₆H₁₃, -C₈H₁₇, -C₁₂H₂₅ and -CH₂-CH(C₆H₁₃)(C₄H₉).
28. Compound according to any of items 23-27, wherein **X** is selected from

**b¹**-(C=O)-**b²**, **b¹**-(C=O)-O-**b²**, **b¹**-(C=O)-S-**b²** or **b**¹-(C=O)(N-**R'**)-**b²**,

wherein **b¹** represents the bond to the O atom and **b²** represents the bond to **Z** or the N atom, in case **Z** represents a chemical bond, and **R'** is selected from the group consisting of H and C1-C4 hydrocarbon, preferably H and C1-C4 alkyl, more preferably H or -CH₃, in particular H.
29. Compound according to any of items 23-28, wherein **Z** represents a chemical bond or a C1-C5 alkyl linker, more preferably a chemical bond, -CH₂-CH₂- or -CH₂-CH₂-CH₂-, in particular -CH₂-CH₂- or -CH₂-CH₂-CH₂-.
30. Compound according to any of items 23-29, wherein the ring, which **R3** and **R4** form together with the N atom they are bound to represents a pyrrolidine, piperidine, alkylpiperidine, such as methylpiperidine, azepane, alkylpiperazine, such as *N-*methylpiperazine, and morpholine.
31. Compound according to any of the preceding items, which is selected from the group consisting of the following compounds according to chemical formula (IV) to (LV):

| | |
|---|---|
| 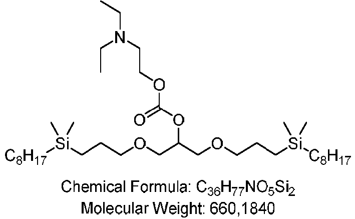 | 2-(Diethylamino)ethyl (9,9,21,21-tetramethyl-13,17-dioxa-9,21-disilanonacosan-15-yl) carbonate |
| | Formula (IV) |
| 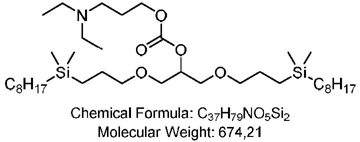 | 3-(Diethylamino)propyl (9,9,21,21-tetramethyl-13,17-dioxa-9,21-disilanonacosan-15-yl) carbonate |
| | Formula (V) |
| 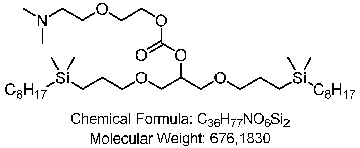 | 2-(2-(Dimethylamino)ethoxy)ethyl (9,9,21,21-tetramethyl-13,17-dioxa-9,21-disilanonacosan-15-yl) carbonate |
| | Formula (VI) |
| 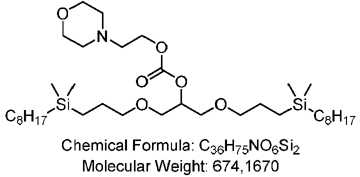 | 2-Morpholinoethyl (9,9,21,21-tetramethyl-13,17-dioxa-9,21-disilanonacosan-15-yl) carbonate |
| | Formula (VII) |
| 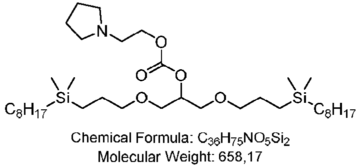 | 2-(Pyrrolidin-1-yl)ethyl (9,9,21,21-tetramethyl-13,17-dioxa-9,21-disilanonacosan-15-yl) carbonate |
| | Formula (VIII) |
| 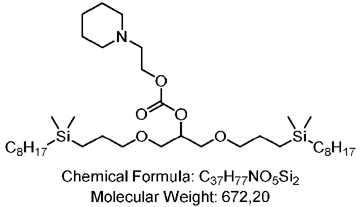 | 2-(Piperidin-1-yl)ethyl (9,9,21,21-tetramethyl-13,17-dioxa-9,21-disilanonacosan-15-yl) carbonate |
| | Formula (IX) |
| 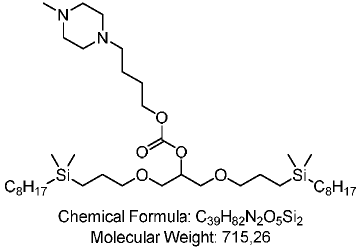 | **Example 7** |
| | 2-(4-Methylpiperazin-1-yl)butyl (9,9,21,21-tetramethyl-13,17-dioxa-9,21-disilanonacosan-15-yl) carbonate |
| | Formula (X) |
| 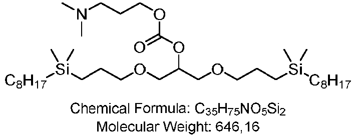 | 3-(Dimethylamino)propyl (9,9,21,21-tetramethyl-13,17-dioxa-9,21-disilanonacosan-15-yl) carbonate |
| | Formula (XI) |
| 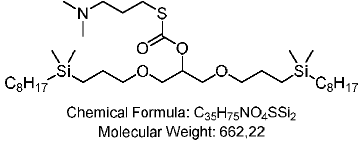 | S-(3-(Dimethylamino)propyl) O-(9,9,21,21-tetramethyl-13,17-dioxa-9,21-disilanonacosan-15-yl) carbonothioate |
| | Formula (XII) |
| 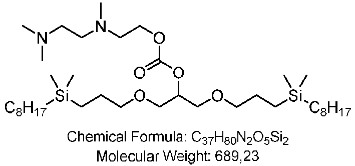 | 2-((2-(Dimethylamino)ethyl)(methyl)amino)eth yl (9,9,21,21-tetramethyl-13,17-dioxa-9,21-disilanonacosan-15-yl) carbonate |
| | Formula (XIII) |
| 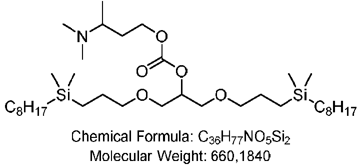 | 3-(Dimethylamino)butyl (9,9,21,21-tetramethyl-13,17-dioxa-9,21-disilanonacosan-15-yl) carbonate |
| | Formula (XIV) |
| 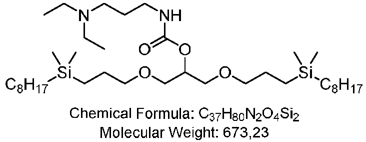 | 9,9,21,21-Tetramethyl-13,17-dioxa-9,21-disilanonacosan-15-yl (3-(diethylamino)propyl)-carbamate |
| | Formula (XV) |
| 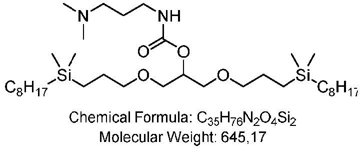 | 9,9,21,21-Tetramethyl-13,17-dioxa-9,21-disilanonacosan-15-yl (3-(dimethylamino)propyl)-carbamate |
| | Formula (XVI) |
| 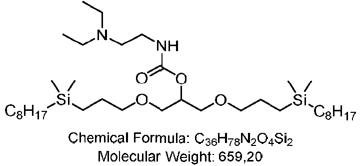 | 9,9,21,21-Tetramethyl-13,17-dioxa-9,21-disilanonacosan-15-yl (2-(diethylamino)ethyl)-carbamate |
| | Formula (XVII) |
| 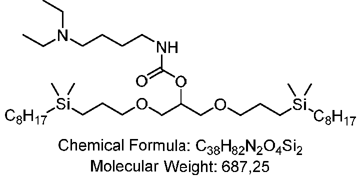 | 9,9,21,21-Tetramethyl-13,17-dioxa-9,21-disilanonacosan-15-yl (4-(diethylamino)butyl)-carbamate |
| | Formula (XVIII) |
| 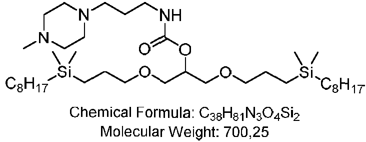 | 9,9,21,21-Tetramethyl-13,17-dioxa-9,21-disilanonacosan-15-yl (3-(4-methylpiperazin-1-yl)propyl) carbamate |
| | Formula (XIX) |
| 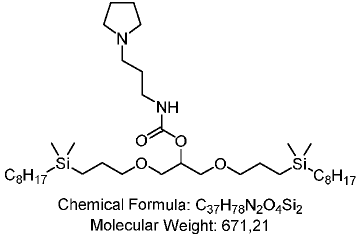 | 9,9,21,21-Tetramethyl-13,17-dioxa-9,21-disilanonacosan-15-yl (3-(pyrrolidin-1-yl)propyl)-carbamate |
| | Formula (XX) |
| 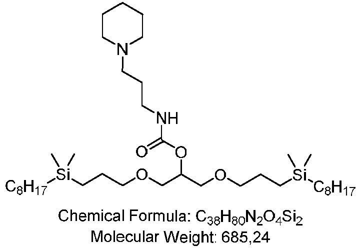 | 9,9,21,21-Tetramethyl-13,17-dioxa-9,21-disilanonacosan-15-yl (3-(piperidin-1-yl)propyl)-carbamate |
| | Formula (XXI) |
| 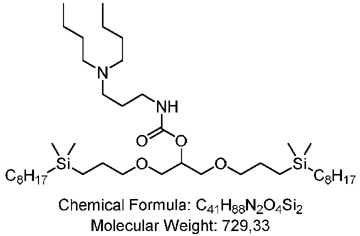 | 9,9,21,21-Tetramethyl-13,17-dioxa-9,21-disilanonacosan-15-yl (3-(dibutylamino)propyl)-carbamate |
| | Formula (XXII) |
| 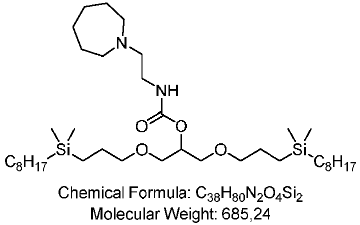 | 9,9,21,21-Tetramethyl-13,17-dioxa-9,21-disilanonacosan-15-yl (2-(azepan-1-yl)ethyl)-carbamate |
| | Formula (XXIII) |
| 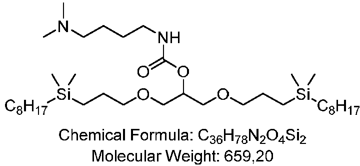 | 9,9,21,21-Tetramethyl-13,17-dioxa-9,21-disilanonacosan-15-yl (4-(dimethylamino)butyl)-carbamate |
| | Formula (XXIV) |
| 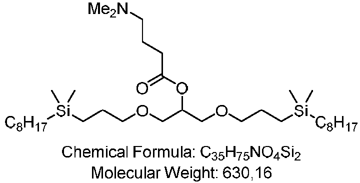 | 9,9,21,21-Tetramethyl-13,17-dioxa-9,21-disilanonacosan-15-yl 4-(dimethylamino)butanoate |
| | Formula (XXV) |
| 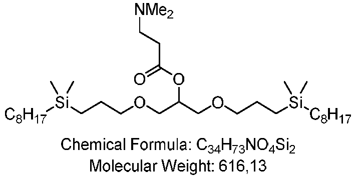 | 9,9,21,21-tetramethyl-13,17-dioxa-9,21-disilanonacosan-15-yl 3-(dimethylamino)propanoate |
| | Formula (XXVI) |
| 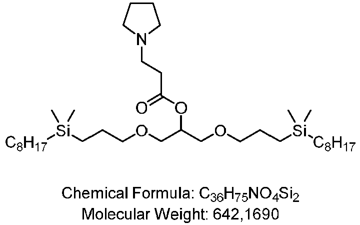 | 9,9,21,21-Tetramethyl-13,17-dioxa-9,21-disilanonacosan-15-yl 3-(pyrrolidin-1-yl)propanoate |
| | Formula (XXVII) |
| 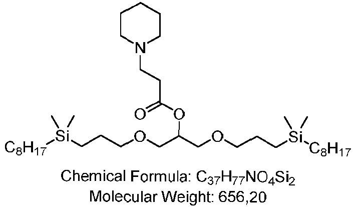 | 9,9,21,21-Tetramethyl-13,17-dioxa-9,21-disilanonacosan-15-yl 3-(piperidin-1-yl)propanoate |
| | Formula (XXVIII) |
| 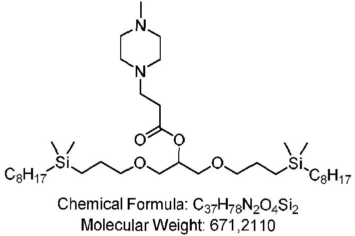 | 9,9,21,21-Tetramethyl-13,17-dioxa-9,21-disilanonacosan-15-yl 3-(4-methylpiperazin-1-yl)-propanoate |
| | Formula (XXIX) |
| 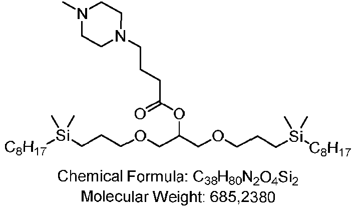 | 9,9,21,21-Tetramethyl-13,17-dioxa-9,21-disilanonacosan-15-yl 4-(4-methylpiperazin-1-yl)-butanoate |
| | Formula (XXX) |
| 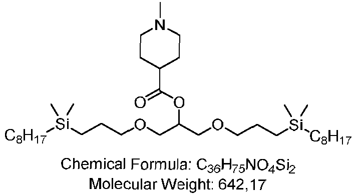 | 9,9,21,21-Tetramethyl-13,17-dioxa-9,21-disilanonacosan-15-yl 1-methylpiperidine-4-carboxylate |
| | Formula (XXXI) |
| 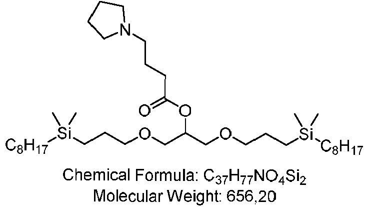 | 9,9,21,21-Tetramethyl-13,17-dioxa-9,21-disilanonacosan-15-yl 4-(pyrrolidin-1-yl)butanoate |
| | Formula (XXXII) |
| 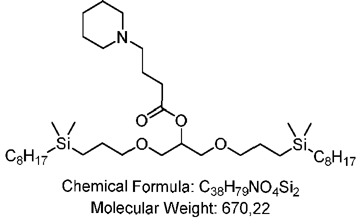 | 9,9,21,21-Tetramethyl-13,17-dioxa-9,21-disilanonacosan-15-yl 4-(piperidin-1-yl)butanoate |
| | Formula (XXXIII) |
| 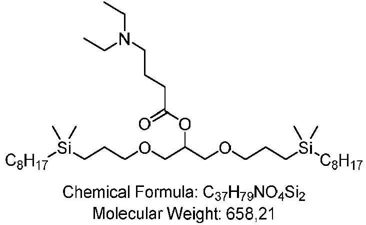 | 9,9,21,21-Tetramethyl-13,17-dioxa-9,21-disilanonacosan-15-yl 4-(diethylamino)butanoate |
| | Formula (XXXIV) |
| 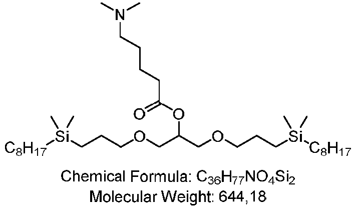 | 9,9,21,21-Tetramethyl-13,17-dioxa-9,21-disilanonacosan-15-yl 5-(dimethylamino)pentanoate |
| | Formula (XXXV) |
| 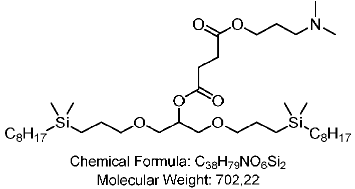 | 3-(Dimethylamino)propyl (9,9,21,21-tetramethyl-13,17-dioxa-9,21-disilanonacosan-15-yl) succinate Formula (XXXVI) |
| 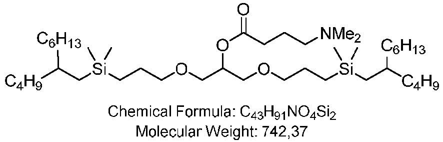 | |
| 7,23-Dibutyl-9,9,21,21-tetramethyl-13,17-dioxa-9,21-disilanonacosan-15-yl 4-(dimethyl-amino)butanoate | |
| Formula (XXXVII) | |
| 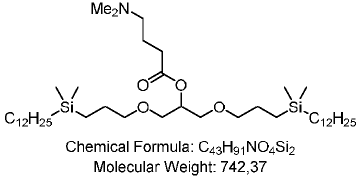 | 13,13,25,25-Tetramethyl-17,21-dioxa-13,25-disilaheptatriacontan-19-yl 4-(dimethylamino)-butanoate |
| | Formula (XXXVIII) |
| 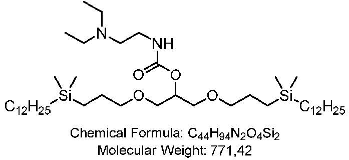 | 13,13,25,25-Tetramethyl-17,21-dioxa-13,25-disilaheptatriacontan-19-yl (2-(diethylamino)-ethyl)carbamate |
| | Formula (XXXIX) |
| 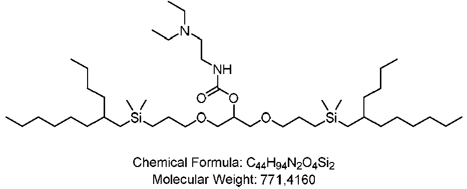 | |
| 7,23-Dibutyl-9,9,21,21-tetramethyl-13,17-dioxa-9,21-disilanonacosan-15-yl (2-(diethylamino)-ethyl)carbamate | |
| Formula (XL) | |
| 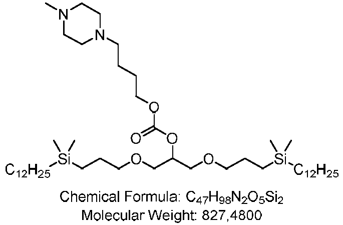 | 4-(4-Methylpiperazin-1-yl)butyl (13,13,25,25-tetramethyl-17,21-dioxa-13,25-disilaheptatria-contan-19-yl) carbonate |
| | Formula (XLI) |
| 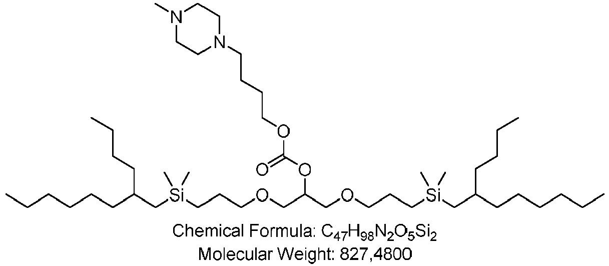 | |
| 7,23-Dibutyl-9,9,21,21-tetramethyl-13,17-dioxa-9,21-disilanonacosan-15-yl (4-(4-methyl-piperazin-1-yl)butyl) carbonate | |
| Formula (XLII) | |
| 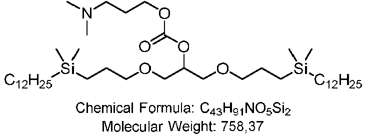 | 3-(Dimethylamino)propyl (13,13,25,25-tetramethyl-17,21-dioxa-13,25-disilaheptatriacontan-19-yl) carbonate |
| | Formula (XLIII) |
| 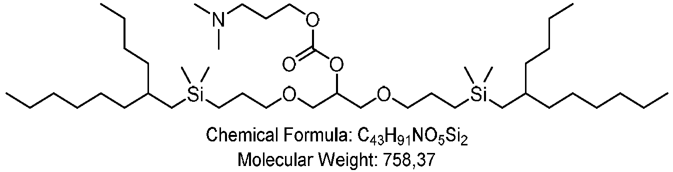 | |
| 7,23-Dibutyl-9,9,21,21-tetramethyl-13,17-dioxa-9,21-disilanonacosan-15-yl (3-(dimethyl-amino)propyl) carbonate | |
| Formula (XLIV) | |
| 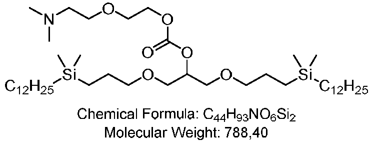 | |
| 2-(2-(Dimethylamino)ethoxy)ethyl (13,13,25,25-tetramethyl-17,21-dioxa-13,25-disilaheptatria-contan-19-yl) carbonate | |
| Formula (XLV) | |
| 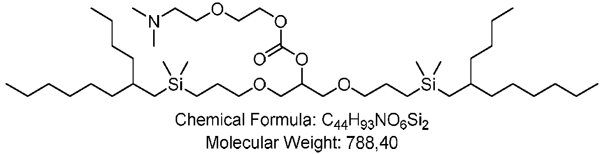 | |
| 7,23-Dibutyl-9,9,21,21-tetramethyl-13,17-dioxa-9,21-disilanonacosan-15-yl (2-(2-(dimethyl-amino)ethoxy)ethyl) carbonate | |
| Formula (XLVI) | |
| 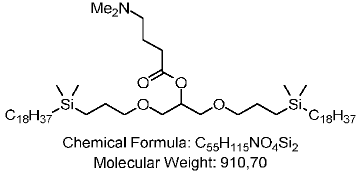 | 19,19,31,31-Tetramethyl-23,27-dioxa-19,31-disilanonatetracontan-25-yl 4-(dimethylamino)-butanoate) |
| | Formula (XLVII) |
| 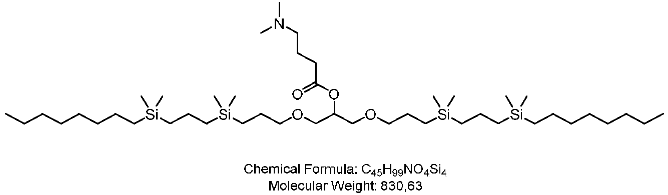 | |
| 9,9,13,13,25,25,29,29-Octamethyl-17,21-dioxa-9,13,25,29-tetrasilaheptatriacontan-19-yl 4-(dimethylamino)butanoate | |
| Formula (XLVIII) | |
| 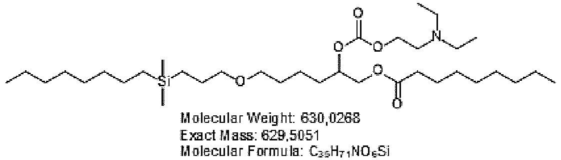 | |
| [2-[2-(Dimethylamino)ethoxycarbonyloxy]-6-[3-[diethyl(octyl)silyl]propoxy]hexyl] nonanoate | |
| Formula (XLIX) | |
| 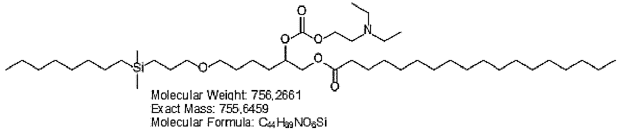 | |
| [2-[2-(Dimethylamino(ethoxycarbonyloxy]-6-[3-[diethyl(octyl)silyl]propoxy]hexyl] octadecanoate | |
| Formula (L) | |
| 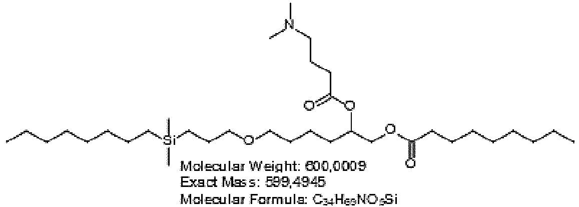 | |
| [2-[4-(Dimethylamino)butanoyloxy]-6-[3-[dimethyl(octyl)silyl]propoxy]hexyl] nonanoate | |
| Formula (LI) | |
| 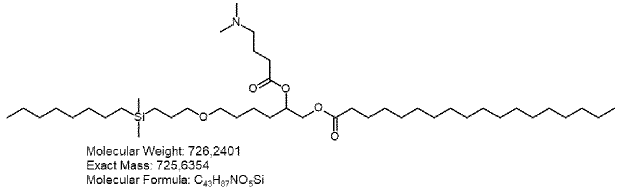 | |
| [2-[4-(dimethylamino)butanoyloxy]-6-[3-[dimethyl(octyl)silyl]propoxy]hexyl] octadecanoate | |
| Formula (LII) | |
| 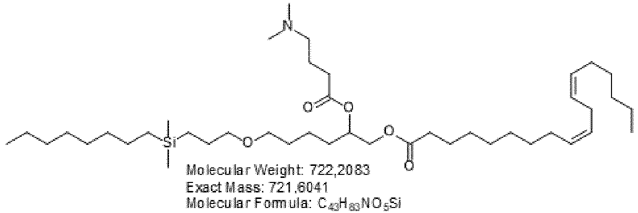 | |
| [2-[4-(Dimethylamino)butanoyloxy]-6-[3-[dimethyl(octyl)silyl]propoxy]hexyl] (9Z,12Z)-octa-deca-9,12-dienoate | |
| Formula (LIII) | |
| 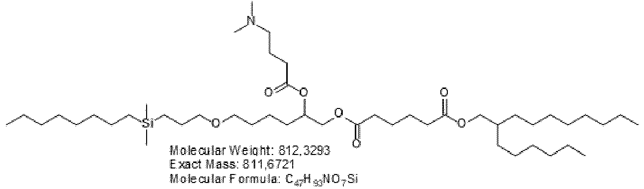 | |
| [2-[4-(Dimethylamino)butanoyloxy]-6-[3-[dimethyl(octyl)silyl]propoxy]hexyl] (2-hexyldecyl) hexanedioate | |
| Formula (LIV) | |
| 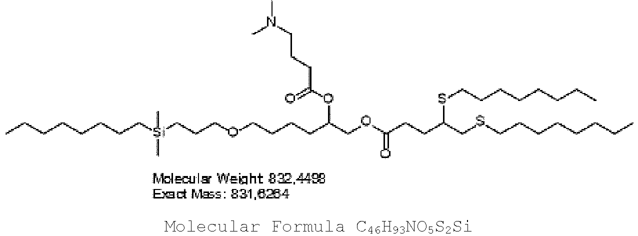 | |
| [2-[4-(dimethylamino)butanoyloxy]-6-[3-[dimethyl(octyl)silyl]propoxy]hexyl] 4,5-bis(octyl sulfanyl)pentanoate | |
| Formula (LV) | |

32. Lipid nanoparticle (LNP) comprising at least one compound according to any of the preceding items.
33. Lipid nanoparticle according to item 32, further comprising at least one colloidal stabilizing lipid.
34. Lipid nanoparticle according to item 33, wherein the colloidal stabilizing lipid is a polyethylenglycol(PEG)-lipid conjugate.
35. Lipid nanoparticle according to any of items 32-34, further comprising a non-cationic lipid, preferably selected from a sterol, a phospholipid or mixtures thereof.
36. Lipid nanoparticle according to item 35, wherein the sterol is cholesterol
37. Lipid nanoparticle according to item 35, wherein the phospholipid is DSPC.
38. Lipid nanoparticle according to any of the items 32-37, further comprising at least one active agent.
39. Lipid nanoparticle according to item 38, whereas the active agent is a therapeutically active biomolecule, preferably selected from nucleic acid molecules, in particular from mRNA.
40. Pharmaceutical composition comprising a lipid nanoparticle according to any of items 38-39 and a pharmaceutically acceptable carrier.
41. Method for the in vivo delivery of an active agent, the method comprising administering to a mammalian subject a lipid nanoparticle of any of items 38-39 or a pharmaceutical composition of item 40.
42. Method for treating a disease or disorder in a mammalian subject in need thereof, the method comprising administering to the mammalian subject a therapeutically effective amount of a lipid nanoparticle of any of items 38-39 or a pharmaceutical composition of item 40.
43. Lipid nanoparticle of any of items 38-39 or pharmaceutical composition of item 40 for use in the in vivo delivery of an active agent to a mammal.
44. Lipid nanoparticle of any of items 38-39 or pharmaceutical composition of item 40 for use in the treatment of a disease or a disorder in a mammalian subject.
45. The use of a lipid nanoparticle of any of items 38-39 or a pharmaceutical composition of item 40 to prepare a medicament for the in vivo delivery of an active agent to a mammal.
46. The use of a lipid nanoparticle of any of items 38-39 or a pharmaceutical composition of item 40 to prepare a medicament for the treatment of a disease or a disorder in a mammalian subject.

## Claims

1. Compound according to chemical formula (I) or its isomeric form 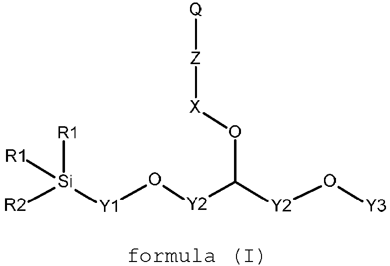 wherein
**R1** represents independently from each other H, OH, or a C1-C16 hydrocarbon residue, which can be linked to the Si atom by an O atom;
**R2** is independently from each other identical to **R1** or represents independently from each other a C6-C20 hydrocarbon residue, which can be substituted and/or can comprise at least one heteroatom;
**Y1** represents independently from each other a C1-C10 hydrocarbon linking group;
**Y2** represents independently from each other a C1-C10 hydrocarbon linking group;
**Y3** represents
-**Y1**-Si(**R1**)₂**R2** with the definitions given above, or
-(C=O)-**R5**, wherein **R5** represents an organic residue comprising at least 5 C atoms,
wherein the organic residue **R5** can be branched or linear and/or
wherein the organic residue **R5** can be saturated or unsaturated, and/or
wherein the organic residue **R5** can contain further heteroatoms selected from O, N or S;
**X** represents together with the O-atom, to which **X** is bound, a carbonate, thio-carbonate, carbamate or ester based linker;
**Z** represents a chemical bond or an organic linker comprising 1 to 5 C atoms, which may further contain at least one heteroatom selected from O and N; and
**Q** represent an organic residue comprising 2 to 20 C-atoms, and at least one N atom, wherein the organic residue can be saturated or unsaturated.

2. Compound according to claim 1, which is a compound according to chemical formula (Ic) or its isomeric form 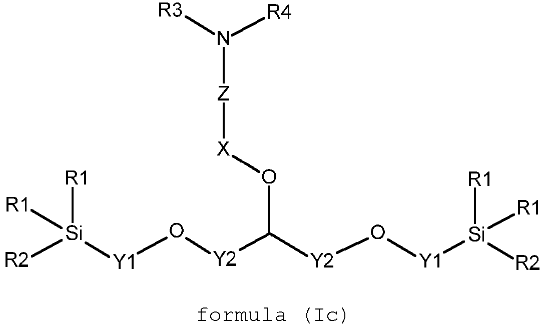 wherein
**R1** represents independently from each other H, OH, or a C1-C16 hydrocarbon residue, which can be linked to the Si atom by a O atom;
**R2** is independently from each other identical to **R1** or represents independently from each other a C6-C20 hydrocarbon residue, which can be substituted and/or can comprise at least one heteroatom;
**Y1** represents independently from each other a C1-C10 hydrocarbon linking group;
**Y2** represents independently from each other a C1-C10 hydrocarbon linking group;
**X** represents together with the O-atom, to which **X** is bound, a carbonate, thio-carbonate, carbamate or ester based linker;
**Z** represents a chemical bond or an organic linker comprising 1 to 5 C atoms, which may further contain at least one heteroatom selected from O and N; and
**R3** and **R4** represent independently from each other a C1-C6 hydrocarbon residue, wherein **R3** and/or **R4** may contain heteroatoms selected from N and O,
or **R3** and **R4** can form a ring together, including the N atom they are bound to, wherein this ring comprises 4-8 carbon atoms, and optionally further heteroatoms selected from O and N.

3. Compound according to any of the preceding claims, wherein all **Y1** represent the same linking group, selected from a C1-C10 hydrocarbon linking group.

4. Compound according to any of the preceding claims, wherein all **Y2** represent the same linking group, selected from a C1-C10 hydrocarbon linking group.

5. Compound according to any of the preceding claims, wherein **Z** represents a chemical bond or a C1-C5 alkyl linker.

6. Compound according to any of the preceding claims, wherein the cyclic organic residue **Q** is selected from the group, consisting of pyrrolidine, piperidine, alkylpiperidine, such as methylpiperidine, azepane, alkylpiperazine, such as *N-*methylpiperazine, and morpholine.

7. Compound according to any of the preceding claims, which is a compound according to chemical formula (II) 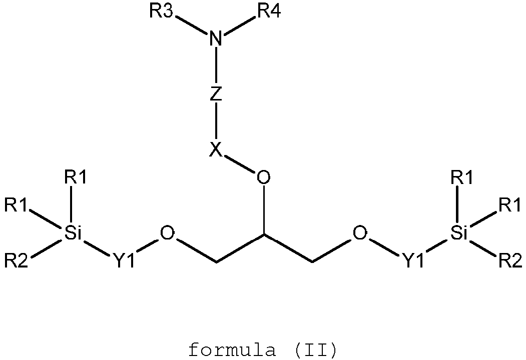 wherein
**R1** represents independently from each other H, OH, or a C1-C16 hydrocarbon residue, which can be linked to the Si atom by a O atom;
**R2** is independently from each other identical to **R1** or represents independently from each other a C6-C20 hydrocarbon residue, which can be substituted and/or can comprise at least one heteroatom;
**Y1** represents independently from each other a C1-C10 hydrocarbon linking group;
**X** represents together with the O-atom, to which **X** is bound, a carbonate, thio-carbonate, carbamate or ester based linker;
**Z** represents a chemical bond or an organic linker comprising 1 to 5 C atoms, which may further contain at least one heteroatom selected from O and N; and
**R3** and **R4** represent independently from each other a C1-C6 hydrocarbon residue,
or **R3** and **R4** can form a ring together, including the N atom they are bound to, wherein this ring comprises 4-8 carbon atoms.

8. Compound according to any of the preceding claims, which is a compound according to chemical formula (III) 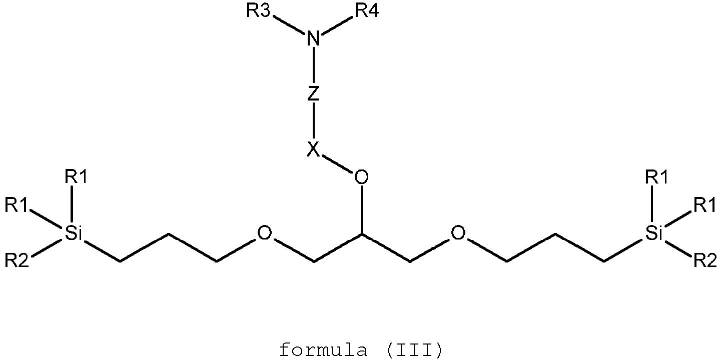 wherein
**R1** represents independently from each other H, OH, or a C1-C16 hydrocarbon residue, which can be linked to the Si atom by a O atom;
**R2** is independently from each other identical to **R1** or represents independently from each other a C6-C20 hydrocarbon residue, which can be substituted and/or can comprise at least one heteroatom;
**X** represents together with the O-atom, to which **X** is bound, a carbonate, thio-carbonate, carbamate or ester based linker;
**Z** represents a chemical bond or an organic linker comprising 1 to 5 C atoms, which may further contain at least one heteroatom selected from O and N; and
**R3** and **R4** represent independently from each other a C1-C6 hydrocarbon residue,
or **R3** and **R4** can form a ring together, including the N atom they are bound to, wherein this ring comprises 4-8 carbon atoms.

9. Compound according to any of the preceding claims, which is selected from the group consisting of the following compounds according to chemical formula (IV) to (LV):
| | |
|---|---|
| 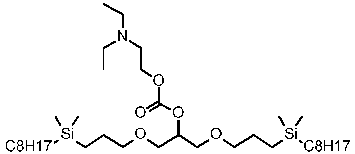 | 2-(Diethylamino)ethyl (9,9,21,21-tetramethyl-13,17-dioxa-9,21-disilanonacosan-15-yl) carbonate |
| | Formula (IV) |
| 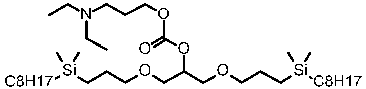 | 3-(Diethylamino)propyl (9,9,21,21-tetramethyl-13,17-dioxa-9,21-disilanonacosan-15-yl) carbonate |
| | Formula (V) |
| 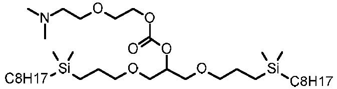 | 2-(2-(Dimethylamino)ethoxy)ethyl (9,9,21,21-tetramethyl-13,17-dioxa-9,21-disilanonacosan-15-yl) carbonate |
| | Formula (VI) |
| 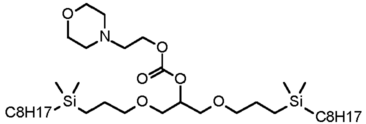 | 2-Morpholinoethyl (9,9,21,21-tetramethyl-13,17-dioxa-9,21-disilanonacosan-15-yl) carbonate |
| | Formula (VII) |
| 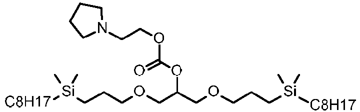 | 2-(Pyrrolidin-1-yl)ethyl (9,9,21,21-tetramethyl-13,17-dioxa-9,21-disilanonacosan-15-yl) carbonate |
| | Formula (VIII) |
| 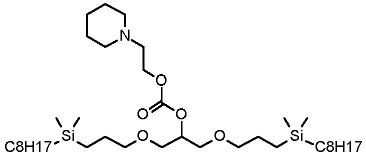 | 2-(Piperidin-1-yl)ethyl (9,9,21,21-tetramethyl-13,17-dioxa-9,21-disilanonacosan-15-yl) carbonate |
| | Formula (IX) |
| 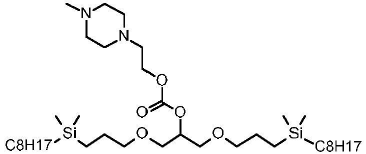 | 2-(4-Methylpiperazin-1-yl)ethyl (9,9,21,21-tetramethyl-13,17-dioxa-9,21-disilanonacosan-15-yl) carbonate |
| | Formula (X) |
| 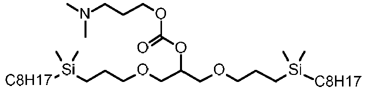 | 3-(Dimethylamino)propyl (9,9,21,21-tetramethyl-13,17-dioxa-9,21-disilanonacosan-15-yl) carbonate |
| | Formula (XI) |
| 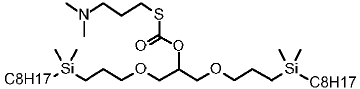 | S-(3-(Dimethylamino)propyl) O-(9,9,21,21-tetramethyl-13,17-dioxa-9,21-disilanonacosan-15-yl) carbonothioate |
| | Formula (XII) |
| 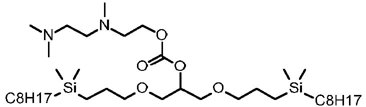 | 2-((2-(Dimethylamino) ethyl) (methyl) amino) et hyl (9,9,21,21-tetramethyl-13,17-dioxa-9,21-disilanonacosan-15-yl) carbonate |
| | Formula (XIII) |
| 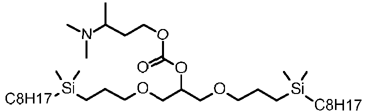 | 3-(Dimethylamino)butyl (9,9,21,21-tetramethyl-13,17-dioxa-9,21-disilanonacosan-15-yl) carbonate |
| | Formula (XIV) |
| 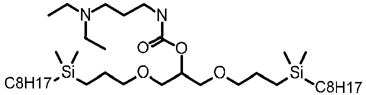 | 9,9,21,21-Tetramethyl-13,17-dioxa-9,21-disilanonacosan-15-yl (3-(diethylamino)propyl)-carbamate |
| | Formula (XV) |
| 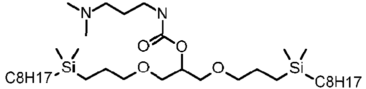 | 9,9,21,21-Tetramethyl-13,17-dioxa-9,21-disilanonacosan-15-yl (3-(dimethylamino)propyl)-carbamate |
| | Formula (XVI) |
| 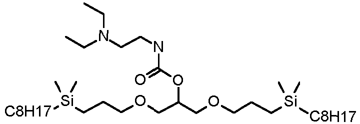 | 9,9,21,21-Tetramethyl-13,17-dioxa-9,21-disilanonacosan-15-yl (2-(diethylamino)ethyl)-carbamate |
| | Formula (XVII) |
| 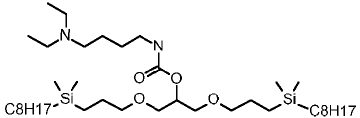 | 9,9,21,21-Tetramethyl-13,17-dioxa-9,21-disilanonacosan-15-yl (4-(diethylamino)butyl)-carbamate |
| | Formula (XVIII) |
| 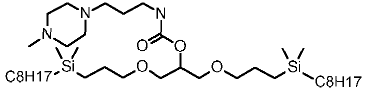 | 9,9,21,21-Tetramethyl-13,17-dioxa-9,21-disilanonacosan-15-yl (3-(4-methylpiperazin-1-yl)propyl) carbamate |
| | Formula (XIX) |
| 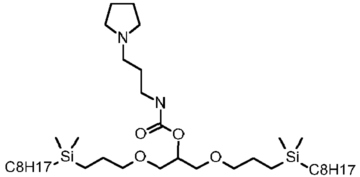 | 9,9,21,21-Tetramethyl-13,17-dioxa-9,21-disilanonacosan-15-yl (3-(pyrrolidin-1-yl)propyl)-carbamate |
| | Formula (XX) |
| 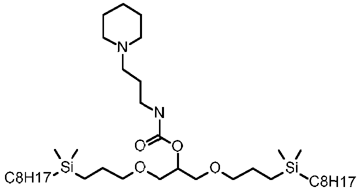 | 9,9,21,21-Tetramethyl-13,17-dioxa-9,21-disilanonacosan-15-yl (3-(piperidin-1-yl)propyl)-carbamate |
| | Formula (XXI) |
| 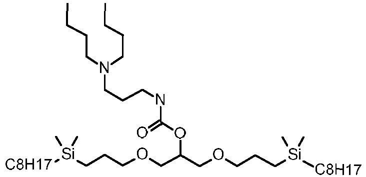 | 9,9,21,21-Tetramethyl-13,17-dioxa-9,21-disilanonacosan-15-yl (3-(dibutylamino)propyl)-carbamate |
| | Formula (XXII) |
| 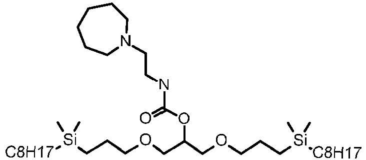 | 9,9,21,21-Tetramethyl-13,17-dioxa-9,21-disilanonacosan-15-yl (2-(azepan-1-yl)ethyl)-carbamate |
| | Formula (XXIII) |
| 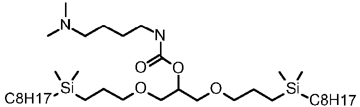 | 9,9,21,21-Tetramethyl-13,17-dioxa-9,21-disilanonacosan-15-yl (4-(dimethylamino)butyl)-carbamate |
| | Formula (XXIV) |
| 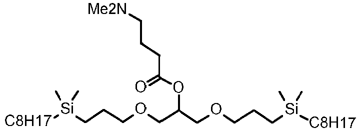 | 9,9,21,21-Tetramethyl-13,17-dioxa-9,21-disilanonacosan-15-yl 4-(dimethylamino)butanoate |
| | Formula (XXV) |
| 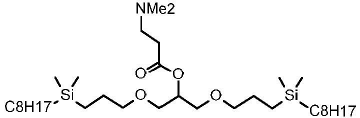 | 9,9,21,21-tetramethyl-13,17-dioxa-9,21-disilanonacosan-15-yl 3-(dimethylamino)propanoate |
| | Formula (XXVI) |
| 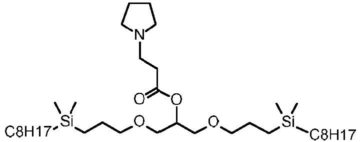 | 9,9,21,21-Tetramethyl-13,17-dioxa-9,21-disilanonacosan-15-yl 3-(pyrrolidin-1-yl)propanoate |
| | Formula (XXVII) |
| 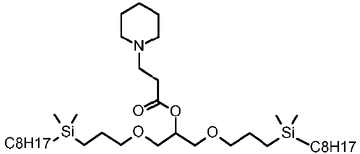 | 9,9,21,21-Tetramethyl-13,17-dioxa-9,21-disilanonacosan-15-yl 3-(piperidin-1-yl)propanoate |
| | Formula (XXVIII) |
| 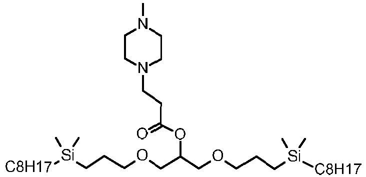 | 9,9,21,21-Tetramethyl-13,17-dioxa-9,21-disilanonacosan-15-yl 3-(4-methylpiperazin-1-yl)-propanoate |
| | Formula (XXIX) |
| 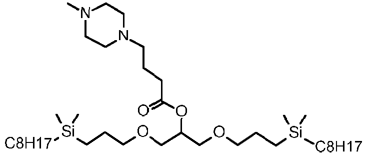 | 9,9,21,21-Tetramethyl-13,17-dioxa-9,21-disilanonacosan-15-yl 4-(4-methylpiperazin-1-yl)-butanoate |
| | Formula (XXX) |
| 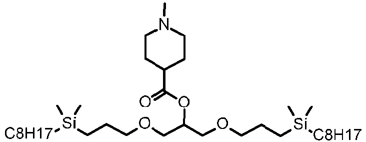 | 9,9,21,21-Tetramethyl-13,17-dioxa-9,21-disilanonacosan-15-yl 1-methylpiperidine-4-carboxylate |
| | Formula (XXXI) |
| 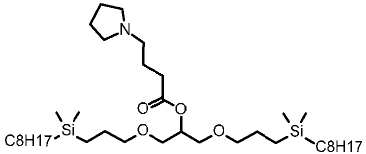 | 9,9,21,21-Tetramethyl-13,17-dioxa-9,21-disilanonacosan-15-yl 4-(pyrrolidin-1-yl)butanoate |
| | Formula (XXXII) |
| 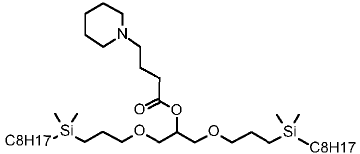 | 9,9,21,21-Tetramethyl-13,17-dioxa-9,21-disilanonacosan-15-yl 4-(piperidin-1-yl)butanoate |
| | Formula (XXXIII) |
| 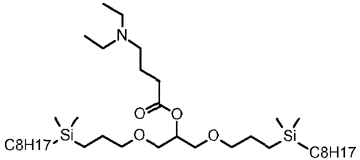 | 9,9,21,21-Tetramethyl-13,17-dioxa-9,21-disilanonacosan-15-yl 4-(diethylamino)butanoate |
| | Formula (XXXIV) |
| 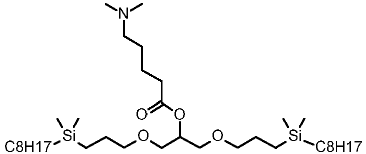 | 9,9,21,21-Tetramethyl-13,17-dioxa-9,21-disilanonacosan-15-yl 5-(dimethylamino)pentanoate |
| | Formula (XXXV) |
| 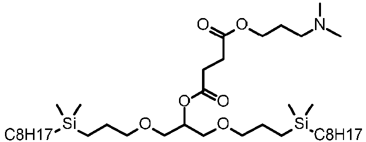 | 3-(Dimethylamino)propyl (9,9,21,21-tetramethyl-13,17-dioxa-9,21-disilanonacosan-15-yl) succinate |
| | Formula (XXXVI) |
| 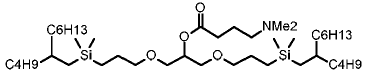 | 7,23-Dibutyl-9,9,21,21-tetramethyl-13,17-dioxa-9,21-disilanonacosan-15-vl 4-(dimethyl-amino)butanoate |
| | Formula (XXXVII) |
| 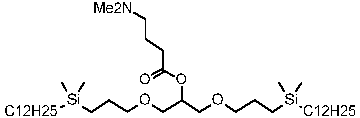 | 13,13,25,25-Tetramethyl-17,21-dioxa-13,25-disilaheptatriacontan-19-yl 4-(dimethylamino)-butanoate |
| | Formula (XXXVIII) |
| 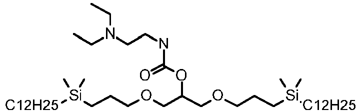 | 13,13,25,25-Tetramethyl-17,21-dioxa-13,25-disilaheptatriacontan-19-yl (2-(diethylamino)-ethyl)carbamate |
| | Formula (XXXIX) |
| 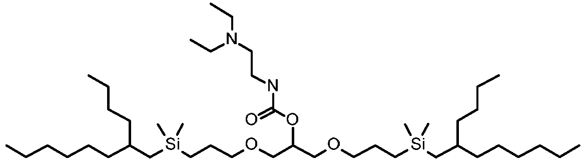 | |
| 7,23-Dibutyl-9,9,21,21-tetramethyl-13,17-dioxa-9,21-disilanonacosan-15-yl (2-(diethylamino)-ethyl)carbamate | |
| Formula (XL) | |
| 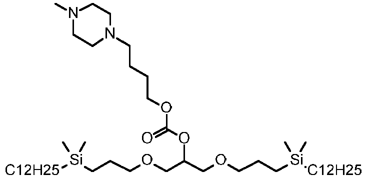 | 4-(4-Methylpiperazin-1-yl)butyl (13,13,25,25-tetramethyl-17,21-dioxa-13,25-disilaheptatria-contan-19-yl) carbonate |
| | Formula (XLI) |
| 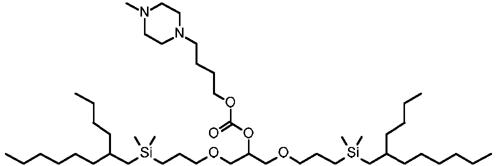 | |
| 7,23-Dibutyl-9,9,21,21-tetramethyl-13,17-dioxa-9,21-disilanonacosan-15-yl (4-(4-methyl-piperazin-1-yl)butyl) carbonate | |
| Formula (XLII) | |
| 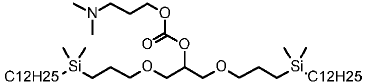 | 3-(Dimethylamino)propyl (13,13,25,25-tetramethyl-17,21-dioxa-13,25-disilaheptatriacontan-19-yl) carbonate |
| | Formula (XLIII) |
| 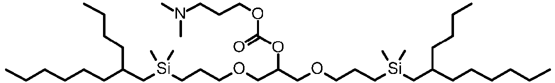 | |
| 7,23-Dibutyl-9,9,21,21-tetramethyl-13,17-dioxa-9,21-disilanonacosan-15-yl (3-(dimethyl-amino)propyl) carbonate | |
| Formula (XLIV) | |
| 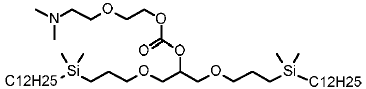 | 2-(2-(Dimethylamino)ethoxy)ethyl (13,13,25,25-tetramethyl-17,21-dioxa-13,25-disilaheptatria-contan-19-yl) carbonate |
| | Formula (XLV) |
| 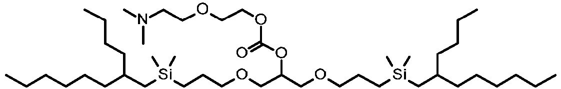 | |
| 7,23-Dibutyl-9,9,21,21-tetramethyl-13,17-dioxa-9,21-disilanonacosan-15-yl (2-(2-(dimethyl-amino)ethoxy)ethyl) carbonate | |
| Formula (XLVI) | |
| 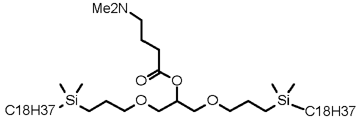 | 19,19,31,31-Tetramethyl-23,27-dioxa-19,31-disilanonatetracontan-25-yl 4-(dimethylamino)-butanoate) |
| | Formula (XLVII) |
| 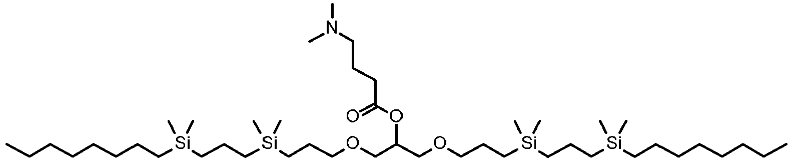 | |
| 9,9,13,13,25,25,29,29-Octamethyl-17,21-dioxa-9,13,25,29-tetrasilaheptatriacontan-19-yl 4-(dimethylamino)butanoate | |
| Formula (XLVIII) | |
| 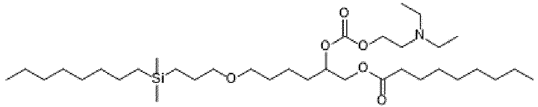 | |
| [2-[2-(Dimethylamino)ethoxycarbonyloxy]-6-[3-[diethyl(octyl)silyl]propoxy]hexyl] nonanoate | |
| Formula (XLIX) | |
| 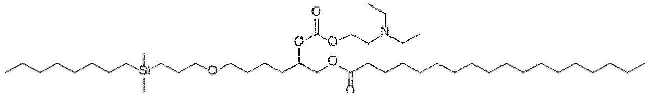 | |
| [2-[2-(Dimethylamino(ethoxycarbonyloxy]-6-[3-[diethyl(octyl)silyl]propoxy]hexyl] octadecanoate | |
| Formula (L) | |
| 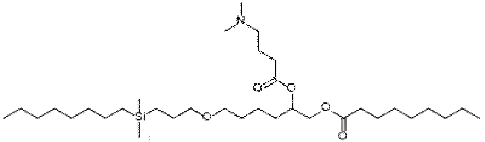 | |
| [2-[4-(Dimethylamino)butanoyloxy]-6-[3-[dimethyl(octyl)silyl]propoxy]hexyl] nonanoate | |
| Formula (LI) | |
| 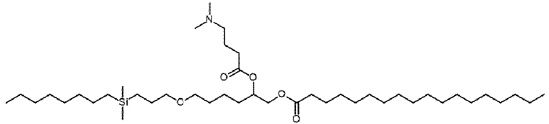 | |
| [2-[4-(dimethylamino)butanoyloxy]-6-[3-[dimethyl(octyl)silyl]propoxy]hexyl] octadecanoate | |
| Formula (LII) | |
| 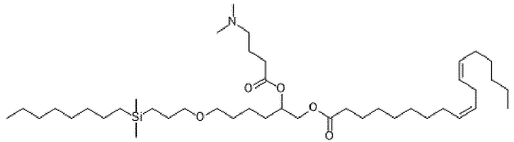 | |
| [2-[4-(Dimethylamino)butanoyloxy]-6-[3-[dimethyl(octyl)silyl]propoxy]hexyl] (9Z,12Z)-octa-deca-9,12-dienoate | |
| Formula (LIII) | |
| 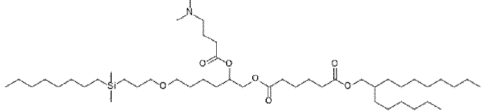 | |
| [2-[4-(Dimethylamino)butanoyloxy]-6-[3-[dimethyl(octyl)silyl]propoxy]hexyl] (2-hexyldecyl) hexanedioate | |
| Formula (LIV) | |
| 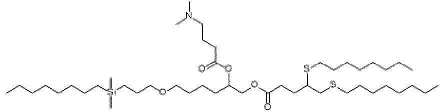 | |
| [2-[4-(dimethylamino)butanoyloxy]-6-[3-[dimethyl(octyl)silyl]propoxy]hexyl] 4,5-bis(octyl sulfanyl)pentanoate | |
| Formula (LV) | |

10. Lipid nanoparticle (LNP) comprising at least one compound according to any of the preceding claims.

11. Lipid nanoparticle according to claim 10, further comprising at least one active agent.

12. Lipid nanoparticle according to claim 11, whereas the active agent is a therapeutically active biomolecule.

13. Pharmaceutical composition comprising a lipid nanoparticle according to any of claims 11-12 and a pharmaceutically acceptable carrier.

14. Lipid nanoparticle of any of claims 11-12 or pharmaceutical composition of claim 13 for use in the in vivo delivery of an active agent to a mammal.

15. Lipid nanoparticle of any of claims 11-12 or pharmaceutical composition of claim 13 for use in the treatment of a disease or a disorder in a mammalian subject.
